# EUROPEAN PATENT SPECIFICATION

(11) **EP 3 551 757 B1**
(45) Date of publication and mention of the grant of the patent: **18.11.2026**
(21) Application number: 17826016.2
(22) Date of filing: 08.12.2017
(51) Int. Cl.: C12N 15/113

(54) **MODIFIED GUIDE RNAS**
MODIFIZIERTE GUIDE-RNAS
ARN GUIDES MODIFIÉS

(30) Priority: 08.12.2016 US 201662431756 P
(43) Date of publication of application: 16.10.2019
(73) Proprietor: Intellia Therapeutics, Inc., Cambridge, MA 02139 (US)
(72) Inventor: SMITH, Amy Madison Rhoden, Cambridge, Massachusetts 02139 (US); MORRISSEY, David V., Cambridge, Massachusetts 02139 (US); STRAPPS, Walter, Cambridge, Massachusetts 02139 (US)
(74) Representative: Schlich
(86) International application number: PCT/US2017/065306
(87) International publication number: WO 2018/107028

(56) References cited:
- WO-A1-2016/089433
- WO-A1-2016/164356
- WO-A1-2017/068377
- WO-A1-2017/136794
- WO-A1-2017/173054
- WO-A2-2017/004279
- AYAL HENDEL ET AL: "Chemically modified guide RNAs enhance CRISPR-Cas genome editing in human primary cells", NATURE BIOTECHNOLOGY (ADVANCE ONLINE PUBLICATION), vol. 33, no. 9, 29 June 2015 (2015-06-29), pages 985 - 989, XP055233915, ISSN: 1087-0156, DOI: 10.1038/nbt.3290
- MING WANG ET AL: "Efficient delivery of genome-editing proteins using bioreducible lipid nanoparticles", PROCEEDINGS NATIONAL ACADEMY OF SCIENCES PNAS, vol. 113, no. 11, 29 February 2016 (2016-02-29), US, pages 2868 - 2873, XP055452122, ISSN: 0027-8424, DOI: 10.1073/pnas.1520244113
- HAO YIN ET AL: "Therapeutic genome editing by combined viral and non-viral delivery of CRISPR system components in vivo", NATURE BIOTECHNOLOGY (ADVANCE ONLINE PUBLICATION), vol. 34, no. 3, 1 February 2016 (2016-02-01), pages 328 - 333, XP055382113, ISSN: 1087-0156, DOI: 10.1038/nbt.3471
- YU XIN ET AL: "Improved delivery of Cas9 protein/gRNA complexes using lipofectamine CRISPRMAX", BIOTECHNOLOGY LETTERS, SPRINGER NETHERLANDS, NL, vol. 38, no. 6, 18 February 2016 (2016-02-18), pages 919 - 929, XP035901439, ISSN: 0141-5492, [retrieved on 20160218], DOI: 10.1007/S10529-016-2064-9

## Description

### SEQUENCE LISTING

The instant application contains a Sequence Listing which has been submitted electronically in ASCII format. Said ASCII copy, created on December 7, 2017, is named 01155-0004-00PCT_SeqList.txt and is 118,877 bytes in size.

This application claims the benefit of priority to United States Provisional Application No. 62/431,756, which was filed on December 8, 2016

This disclosure relates to the field of gene editing using CRISPR/Cas systems, a part of the prokaryotic immune system that recognizes and cuts exogenous genetic elements. The CRISPR/Cas system relies on a single nuclease, termed CRISPR-associated protein 9 (Cas9), which induces site-specific breaks in DNA. Cas9 is guided to specific DNA sequences by small RNA molecules termed guide RNA (gRNA). Guide RNA comprises trRNA (also known as tracrRNA) and crisprRNA (crRNA). The trRNA and crRNA may be contained within a single guide RNA (sgRNA) or in two separate RNA molecules of a dual guide RNA (dgRNA). Cas9 in combination with trRNA and crRNA or an sgRNA is termed the Cas9 ribonucleoprotein complex (RNP).

Oligonucleotides, and in particular RNA, are sometimes degraded in cells and in serum by endonuclease or exonuclease cleavage. Improved methods and compositions for preventing such degradation, improving stability of gRNAs and enhancing gene editing efficiency is desired, especially for therapeutic applications.
WO2017004279 and WO2017136794 describe compositions comprising and methods for chemical modification of single guide RNA (sgRNA), tracrRNA and/or crRNA used individually or in combination with one another or Cas system components. WO2017068377 describes modified guide RNAs which can be used with the CRISPR Cas9 system for modifying gene expression, including in mammalian cells for medical or research uses.

### SUMMARY

The invention provides a single guide RNA (sgRNA) comprising mN*mN*mN*NNNNNNNNNNNNNNNNNGUUUUAGAmGmCmUmAmGmAmAmAmU mAmGmCAAGUUAAAAUAAGGCUAGUCCGUUAUCAmAmCmUmUmGmAmAmAm AmAmGmUmGmGmCmAmCmCmGmAmGmUmCmGmGmUmGmCmU*mU*mU*mU (SEQ ID NO: 358), wherein N denotes any one of A, C, G, and U; * denotes a phosphorothioate (PS) linkage; and mN, mA, mC, mG, and mU respectively denote N, A, C, G, and U modified with 2'-O-methyl (2'-O-Me). The modified guide RNAs described herein may improve the stability of the guide RNA and the guide RNA/Cas9 complex and improve the activity of Cas9 (e.g., SpyCas9 and equivalents) to cleave target DNA..

The guide RNAs described herein comprise at least one modified nucleotide. Modifications may include 2'-O-methyl (2'-O-Me), 2'-O-(2-methoxyethyl) (2'-O-moe), 2'-fluoro (2'-F), phosphorothioate (PS) bond between nucleotides, G-C substitutions, and inverted abasic linkages between nucleotides and equivalents thereof.

In some reference examples described herein, a single guide RNA (sgRNA) is described as comprising a 5' end modification and one or more modification in one or more of: the upper stem region; the hairpin 1 region; and the hairpin 2 region, wherein the 5' end modification comprises at least two phosphorothioate linkages within the first seven nucleotides at the 5' end of the 5' terminus. In some instances, the modification is a 2'-O-methyl (2'-O-Me) modified nucleotide. In some reference examples, the modification is a 2'-fluoro (2'-F) modified nucleotide.

In some reference examples, the sgRNA comprises modifications at US1 to US12 and/or a modification at H1-1 and/or a modification in H2-1. In some reference examples, the sgRNA comprises modifications at H1-1 to H1-12 and/or H2-1 to H2-15. In some reference examples, the sgRNA comprises one or more modifications in each of the upper stem region, the hairpin 1 region, and the hairpin 2 region. In some reference examples, the sgRNA comprises a modified nucleotide between hairpin 1 and hairpin 2 regions. In some reference examples, the sgRNA comprises a modification in the lower stem region.

In some reference examples, the sgRNA comprises a modification at the 5' terminus and/or the 3' terminus. In some reference examples, the sgRNA comprises a 3' end modification in the 3' terminus. In some reference examples, the sgRNA comprises modifications on at least two of the last four nucleotides at the 3' end of the 3' terminus. In some reference examples, the sgRNA comprises a 5' end modification in the 5' terminus. In some reference examples, the sgRNA comprises modifications on at least two of the first four nucleotides at the 5' end of the 5' terminus. In some reference examples, the sgRNA comprises a 3' end modification in the 3' terminus and a 5' end modification in the 5' terminus. In some reference examples, the sgRNA comprises modifications on at least two of the last four nucleotides at the 3' end of the 3' terminus and on at least two of the first four nucleotides at the 5' end of the 5' terminus. In some instances, these modifications are 2'-O-Me, 2'-F, 2'-O-moe, or phosphorothioate (PS) bonds linking the nucleotides. In some reference examples, the sgRNA comprises PS bonds between at least two of the last four nucleotides at the 3' end of the 3' terminus and/or at least two of the first four nucleotides at the 5' end of the 5' terminus. In some instances, the sgRNA comprises 5' terminus and 3' terminus with more than one modification as described herein, such as, with PS bonds and 2'-O-Me modifications.

In some reference examples, the sgRNA comprises a modification in the bulge region. In some reference examples, 50% of the nucleotides in the bulge region are modified, wherein the modification is 2'-O-Me or 2'-F.

In some reference examples, the sgRNA comprises a modification in the nexus region. In some reference examples, the sgRNA comprises modifications at N15, N16, N17, and/or N18 in the nexus region, wherein the modification is 2'-O-Me or 2'-F. In some instances, N16, N17, and N18 are linked with PS bonds.

In some reference examples, the sgRNA comprises at least the first three nucleotides at the 5' end of the 5' terminus, and the last three nucleotides at the 3' end of the 3' terminus are modified.

In some reference examples, the sgRNA comprises modifications at the 3' terminus and/or 5' terminus. In some instances, the first four nucleotides at the 5' end of the 5' terminus, and the last four nucleotides at the 3' end of the 3' terminus are linked with phosphorothioate (PS) bonds. In some reference examples, the 5' and 3' modification comprises 2'-O-Me or 2'-O-moe. In some reference examples, the 5' and 3' modification comprises 2'-F. In some reference examples, the 5' and/or 3' modification comprises PS bonds linking nucleotides. In some reference examples, the 5' and/or 3' modification comprises one or more of 2'-O-Me, 2'-O-moe, 2' -F, and PS bonds linking nucleotides.

In some reference examples, the sgRNA comprises modifications at the first four nucleotides at the 5' end of the 5' terminus and the last four nucleotides at the 3' end of the 3'terminus. In some instances, these modifications are linking PS bond (i.e., PS bonds that link the first four and last four nucleotides). In some reference examples, the sgRNA further comprises 2'-O-Me modifications at the first three nucleotides at the 5' end of the 5' terminus and the last three nucleotides at the 3' end of the 3' terminus.

In some reference examples, the sgRNA comprises modifications at the first four nucleotides at the 5' end of the 5' terminus and the last four nucleotides at the 3' end of the 3' terminus, wherein the modifications are at least PS bonds linking the four nucleotides, and further wherein the first three nucleotides at the 5' end of the 5' terminus and the last three nucleotides at the 3' end of the 3' terminus comprise 2'-O-Me, 2'-O-moe, or 2'-F modifications.

In some reference examples, the sgRNA comprises modifications LS1, LS6, LS7, LS8, LS11, and LS12, wherein the modification is 2'-O-Me or 2'-F.

In some reference examples, the sgRNA comprises modifications at each of the nucleotides in the bulge region, wherein the modification is 2'-O-Me or 2'-F.

In some reference examples, the sgRNA comprises modifications at each of the nucleotides in the upper stem region, wherein the modification is 2'-O-Me or 2'-F.

In some reference examples, the sgRNA comprises modifications at each of the nucleotides in the hairpin 1 region, wherein the modification is 2'-O-Me or 2'-F.

In some reference examples, the sgRNA comprises modifications at each of the nucleotides in the hairpin 2 region, wherein the modification is 2'-O-Me or 2'-F.

In some reference examples, an sgRNA is described as comprising 2'-O-Me modified nucleotides at the following positions:
a. the first three nucleotides at the 5' end of the 5' terminus;
b. LS1, LS6, LS7, LS8, LS11, and/or LS12 in the lower stem region;
c. B1 and/or B2 in the bulge region;
d. each nucleotide in the upper stem region;
e. N16, N17, and/or N18 in the nexus region;
f. each nucleotide in the hairpin 1 region;
g. each nucleotide in the hairpin 2 region; and
h. the last four nucleotides at the 3' end of the 3' terminus.
In some reference examples, B3-B6 are modified with 2'-O-Me. In some instances, the sgRNA further comprising three phosphorothioate (PS) bonds linking the first four nucleotides at the 5' end of the 5' terminus and three PS bonds linking the last four nucleotides at the 3' end of the 3' terminus. In some reference examples, the sgRNA comprises 2'-F modifications at LS9 and LS10. In some reference examples, the sgRNA comprises 2'F modifications at N15, N16, N17, and N18. In some reference examples, the sgRNA comprises 2'F modifications at H2-9, H2-10, H2-11, H2-12, H2-13, H2-14, and H2-15. In some reference examples, the sgRNA comprises 2'F modifications at the second to last, third to last, and fourth to last nucleotides at the 3' end of the 3' terminus.

In some reference examples, an sgRNA is described as comprising 2'-F modified nucleotides at the following positions:
a. LS9 and LS10 in the lower stem region;
b. N15, N16, N17, and N18 in the nexus region; and
c. H2-9, H2-10, H2-11, H2-12, H2-13, H2-14, and H2-15 in the hairpin 2 region.
In some reference examples, the sgRNA comprises 2'-F modified nucleotides at the second to last, third to last, and fourth to last nucleotides at the 3' terminus. In some reference examples, the sgRNA comprises three phosphorothioate (PS) bonds linking the first four nucleotides at the 5' end of the 5' terminus and three PS bonds linking the last four nucleotides at the 3' end of the 3' terminus. In some reference examples, the sgRNA comprises 2'-O-Me or 2'-F modified nucleotides at the first three nucleotides at the 5' end of the 5' terminus, and 2'-O-Me or 2'-F modified nucleotides at three of the last four nucleotides at the 3' end of the 3' terminus.

In some reference examples, an sgRNA is described as comprising
a. 2'-O-Me modified nucleotides at the first three nucleotides at the 5' end of the 5' terminus;
b. Optional 2'-O-Me modified nucleotides at LS1 and/or LS6;
c. 2'-O-Me modified nucleotides at US1-US12;
d. 2'-O-Me modified nucleotides at H1-1 - H1-12;
e. Optional 2'-O-Me modified nucleotide between Hairpin 1 and Hairpin 2;
f. 2'-O-Me modified nucleotides at H2-1 - H2-15; and
g. 2'-O-Me modified nucleotides at the last four nucleotides at the 3' end of the 3' terminus; and optionally
further comprising three phosphorothioate (PS) bonds linking the first four nucleotides at the 5' end of the 5' terminus and three PS bonds linking the last four nucleotides at the 3' end of the 3' terminus.

In some reference examples, an sgRNA is described as comprising:
a. 2'-O-Me modified nucleotides at the first three nucleotides at the 5' end of the 5' terminus;
b. 2'-F modified nucleotides at LS1-LS6;
c. 2'-O-Me modified nucleotides at US1-US12;
d. 2'-O-Me modified nucleotides at H1-1 - H1-12;
e. a 2'-O-Me modified nucleotide between Hairpin 1 and Hairpin 2;
f. 2'-O-Me modified nucleotides at H2-1 - H2-15; and
g. 2'-O-Me modified nucleotides at the last four nucleotides at the 3' end of the 3' terminus; and optionally
further comprising three phosphorothioate (PS) bonds linking the first four nucleotides at the 5' end of the 5' terminus and three PS bonds linking the last four nucleotides at the 3' end of the 3' terminus.

In some reference examples, an sgRNA is described as comprising:
a. 2'-O-Me modified nucleotides at the first three nucleotides at the 5' terminus;
b. 2'-F modified nucleotides at LS2-LS5;
c. 2'-O-Me modified nucleotides at LS1 and LS6;
d. 2'-O-Me modified nucleotides at US1-US12;
e. 2'-O-Me modified nucleotides at H1-1 - H1-12;
f. a 2'-O-Me modified nucleotide between Hairpin 1 and Hairpin 2;
g. 2'-O-Me modified nucleotides at H2-1 - H2-15; and
h. 2'-O-Me modified nucleotides at the last four nucleotides at the 3' terminus and optionally
further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides at the first three nucleotides at the 5' terminus;
b. 2'-O-Me modified nucleotides at US1-US12;
c. 2'-O-Me modified nucleotides at LS7, LS8, LS11, and LS12;
d. 2'-O-Me modified nucleotides at H1-1 - H1-12;
e. a 2'-O-Me modified nucleotide between Hairpin 1 and Hairpin 2;
f. 2'-O-Me modified nucleotides at H2-1 - H2-15; and
g. 2'-O-Me modified nucleotides at the last four nucleotides at the 3' terminus,
and optionally further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides at the first three nucleotides at the 5' terminus;
b. 2'-O-Me modified nucleotides at US1-US12;
c. 2'-O-Me modified nucleotides at LS7, LS8, LS11, and LS12;
d. 2'-F modified nucleotides at LS9 and LS10;
e. 2'-O-Me modified nucleotides at H1-1 - H1-12;
f. a 2'-O-Me modified nucleotide between Hairpin 1 and Hairpin 2;
g. 2'-O-Me modified nucleotides at H2-1 - H2-15; and
h. 2'-O-Me modified nucleotides at the last four nucleotides at the 3' terminus,
and optionally further comprising three phosphorothioate (PS) bonds linking the first four nucleotides at the 5' end of the 5' terminus and three PS bonds linking the last four nucleotides at the 3' end of the 3' terminus.

In some reference examples, an sgRNA is described as comprising:
a. 2'-O-Me modified nucleotides at the first three nucleotides at the 5' terminus;
b. 2'-O-Me modified nucleotides at US1-US12;
c. 2'-O-Me modified nucleotides at LS8, LS10, and LS12;
d. 2'-O-F modified nucleotides at LS7, LS9, and LS11;
e. 2'-O-Me modified nucleotides at H1-1 - H1-12;
f. a 2'-O-Me modified nucleotide between Hairpin 1 and Hairpin 2;
g. 2'-O-Me modified nucleotides at H2-1 - H2-15; and
h. 2'-O-Me modified nucleotides at the last four nucleotides at the 3' terminus, and optionally
further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides at the first three nucleotides at the 5' terminus;
b. 2'-O-Me modified nucleotides at LS1, LS6, LS7, LS8, LS11, and LS12
c. 2'-O-Me modified nucleotides at US1-US12;
d. 2'-O-Me modified nucleotides at H1-1 - H1-12;
e. a 2'-O-Me modified nucleotide between Hairpin 1 and Hairpin 2;
f. 2'-O-Me modified nucleotides at H2-1 - H2-15; and
g. 2'-O-Me modified nucleotides at the last four nucleotides at the 3' terminus, and optionally
further comprising three phosphorothioate (PS) bonds linking the first four nucleotides at the 5' end of the 5' terminus and three PS bonds linking the last four nucleotides at the 3' end of the 3' terminus

In some reference examples, a sgRNA is described as comprising:
a. 2'-O-Me modified nucleotides at the first three nucleotides at the 5' terminus;
b. 2'-O-Me modified nucleotides at LS1, LS6, LS7, LS8, LS11, and LS12;
c. 2'-F modified nucleotides at LS9 and LS10;
d. 2'-O-Me modified nucleotides at US1-US12;
e. 2'-O-Me modified nucleotides at H1-1 - H1-12;
f. a 2'-O-Me modified nucleotide between Hairpin 1 and Hairpin 2;
g. 2'-O-Me modified nucleotides at H2-1 - H2-15; and
h. 2'-O-Me modified nucleotides at the last four nucleotides at the 3' terminus, and optionally
further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides at the first three nucleotides at the 5' end of the 5' terminus;
b. 2'-O-Me modified nucleotides at US1-US12;
c. 2'-O-Me modified nucleotides at H1-1 - H1-12;
d. a 2'-O-Me modified nucleotide between Hairpin 1 and Hairpin 2;
e. 2'-O-Me modified nucleotides at H2-1 - H2-8;
f. 2'-F modified nucleotides at H2-9 - H2-15;
g. 2'-F modified nucleotides at the second from last, third from last, and fourth from last nucleotide at the 3' terminus; and
h. a 2'-O-Me modified nucleotide at the last nucleotide at the 3' terminus, and optionally
further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides at the first three nucleotides at the 5' end of the 5' terminus;
b. 2'-O-Me modified nucleotides at US1-US12;
c. 2'-O-Me modified nucleotides at H1-2, H1-4, H1-6, H1-8, H1-10, and H1-12;
d. 2'-F modified nucleotides at H1-1, H1-3, H1-5, H1-7, H1-9, and H1-11;
e. a 2'-F modified nucleotide between Hairpin 1 and Hairpin 2;
f. 2'-F modified nucleotides at H2-2, H2-4, H2-6, H2-8, H2-10, H2-12; and H2-14;
g. 2'-O-Me modified nucleotides at H2-1, H2-3, H2-5, H2-7, H2-9, H2-11; H2-13, and H2-15;
h. 2'-F modified nucleotides at the second from last, and fourth from last nucleotide at the 3' terminus; and
i. 2'-O-Me modified nucleotide at the third from last and last nucleotide at the 3' end of the 3' terminus,
and optionally further comprising three phosphorothioate (PS) bonds linking the first four nucleotides at the 5' end of the 5' terminus and three PS bonds linking the last four nucleotides at the 3' end of the 3' terminus.

In some reference examples, a sgRNA is described as comprising:
a. 2'-O-Me modified nucleotides LS8, LS10, LS12, H1-2, H1-4, H1-6, H1-8, H1-10, H1-12, H2-1, H2-3, H2-5, H2-7, H2-9, H2-11, H2-13, and H2-15; and
b. 2'-F modified nucleotides at LS7, LS9, LS11; H1-1, H1-3, H1-5, H1-7, H1-9, H1-11, H1-13, H2-2, H2-4, H2-6, H2-8, H2-10, H2-12, and H2-14, and optionally
further comprising three phosphorothioate (PS) bonds linking the first four nucleotides at the 5' end of the 5' terminus and three PS bonds linking the last four nucleotides at the 3' end of the 3' terminus; and optionally further comprising:
c. 2'-O-Me modified nucleotides at the last and third to last nucleotide at the 3' end of the 3' terminus; and/or
d. 2'-F modified nucleotides at the second to last, fourth to last, and/or last nucleotide at the 3' end of the 3' terminus.

In some reference examples, a sgRNA is described as comprising the nucleic acids of any of SEQ ID Nos: 228-353, including the modifications of Table 4. In some reference examples, a sgRNA is described as comprising any of SEQ ID Nos: 228-332, including the modifications of Table 4. In some reference examples, an sgRNA is described as comprising any of SEQ ID Nos: 235-240, 265-285, and 309-329, including the modifications of Table 4. In some reference examples, an sgRNA is described as comprising SEQ ID No: 240. In some reference examples, a sgRNA is described as comprising SEQ ID No. 240, including the modifications of Table 4. In some reference examples, a sgRNA is described as comprising SEQ ID No: 242. In some reference examples, a sgRNA is described as comprising SEQ ID No: 358. In additional reference examples, a sgRNA comprising nucleic acids having at least 99, 98, 97, 96, 95, 94, 93, 92, 91, 90, 85, 80, 75, or 70% identity to the nucleic acids of any one of SEQ ID Nos: 235-240, 265-285, and 309-329, wherein the modification at each nucleotide of the sgRNA that corresponds to a nucleotide of the reference sequence identifier in Table 4, is identical to or equivalent to the modification shown in the reference sequence identifier in Table 4, optionally further comprising three phosphorothioate (PS) bonds linking the first four nucleotides at the 5' end of the 5' terminus and three PS bonds linking the last four nucleotides at the 3' end of the 3' terminus. In some reference examples, the sgRNA further comprises at least three PS bonds linking the nucleotides in the hairpin 1 region. In some reference examples, the sgRNA further comprises at least three PS bonds linking the nucleotides in the hairpin 2 region. In some reference examples, the sgRNA further comprises at least three PS bonds linking the nucleotides in the upper stem region. In some embodiments, the sgRNA forms a ribonucleoprotein complex with *S. pyogenes* Cas9.

### FIGURE LEGENDS

FIG 1 shows percent editing as measured by next-generation sequence (NGS) of mouse transthyretin (TTR) gene following transfection of Neuro2A cells with modified crRNAs together with Cas9 mRNA and unmodified trRNA (TR000002).
FIG 2 shows percent editing as measured by NGS of mouse TTR gene following transfection of Neuro2A cells with modified trRNAs together with unmodified crRNA (CR000686) and Cas9 mRNA.
FIG 3 shows percent editing as measured by NGS of mouse TTR gene following transfection of Neuro2A cells with Cas9 mRNA and crRNAs and trRNAs having G-C pairings not found in parental sequences.
FIG 4 shows percent editing as measured by NGS of mouse TTR gene following transfection of Neuro2A cells with modified crRNAs and trRNAs together with Cas9 mRNA. Standard deviations follow the value.
FIG 5 shows percent editing as measured by NGS of mouse TTR gene following transfection of Neuro2A cells with modified sgRNAs together with Cas9 mRNA.
FIG 6 shows percent editing as measured by NGS of mouse TTR gene following transfection of Neuro2A cells with modified crRNAs and unmodified trRNA (TR000002) together with Cas9 mRNA. The asterisk denotes a dual guide that for technical reasons did not show activity in this experiment. This dual guide was tested again in the experiment represented in Figure 9, in which it showed editing activity.
FIG 7 shows percent editing as measured by NGS of mouse TTR gene following transfection of Neuro2A cells with unmodified crRNA (CR000686) and modified trRNAs together with Cas9 mRNA.
FIG 8 shows percent editing as measured by NGS of mouse TTR gene following transfection of Neuro2A cells with Cas9 mRNA and crRNA and trRNA pairings with G-C pairings or G-U mismatches not found in the parental sequences.
FIG 9 shows percent editing as measured by NGS of mouse TTR gene following transfection of Neuro2A cells with modified crRNAs and modified trRNAs together with Cas9 mRNA. Standard deviations follow the value.
FIG 10 shows percent editing as measured by NGS of mouse TTR gene following transfection of Neuro2A cells with modified sgRNAs together with Cas9 mRNA.
FIG 11 shows percent editing as measured by NGS of mouse Factor VII (FVII) gene following transfection of Neuro2A cells with modified sgRNAs together with Cas9 mRNA.
FIGs 12A and 12B show percent editing as measured by NGS of mouse TTR (FIG 12A) or FVII (FIG 12B) following transfection of Neuro2A cells with modified crRNAs and unmodified trRNA together with Cas9 mRNA.
FIGs 13A and 13B shows percent editing as measured by NGS of mouse TTR (FIG 13A) or FVII (FIG 13B) following transfection of Neuro2A cells with modified trRNAs and unmodified crRNA together with Cas9 mRNA.
FIGs 14A, 14B, 14C, and 14D show interferon alpha (IFN-alpha, 14A), interleukin 6 (IL-6, 14B), monocyte chemotactic protein 1 (MCP-1, 14C), and tumor necrosis factor alpha (TNF-alpha, 14D) levels in serum after in vivo administration of LNPs comprising Cas9 mRNA and sgRNAs.
FIGs 15A, 15B, and 15C show in vivo results following administration of LNPs comprising Cas9 mRNA and sgRNAs. FIG 15A shows percentage of total editing in liver. FIG 15B shows serum TTR levels. FIG 15C shows the mean and standard deviation for the results of FIG 15A. FIG 15D summarizes modifications to the G000209 sgRNA (SEQ ID NO: 228). FIG 15E summarizes modifications to the G000267 sgRNA (SEQ ID NO: 234). In FIG 15D and 15E, the nucleotides in bold are 2'-O-Me modified.
FIGs 16A, 16B, 16C, and 16D show interferon alpha (IFN-alpha, 16A), tumor necrosis factor alpha (TNF-alpha, 16B), interleukin 6 (IL-6, 16C), and monocyte chemotactic protein 1 (MCP-1, 16D) levels in serum after in vivo administration of LNPs comprising Cas9 mRNA and sgRNAs.
FIGs 17A, 17B, 17C, and 17D show in vivo results following administration of LNPs comprising Cas9 mRNA and sgRNAs. FIG 17A shows percentage of total editing in liver. FIG 17B shows the mean and standard deviation for the results of FIG 17A. FIG 17C shows serum TTR levels. FIG 17D shows the mean and standard deviation for the results of FIG 17B.
FIGs 18A, 18B, and 18C show in vivo results following administration of LNPs comprising Cas9 mRNA and sgRNAs. FIG 18A shows percentage of total editing in liver. FIG 18B summarizes liver editing data. FIG 18C shows serum TTR levels. MPK = milligrams per kilogram; BLOD = below level of detection.
FIGs 19A, 19B, 19C, and 19D show interferon alpha (IFN-alpha, 19A), monocyte chemotactic protein 1 (MCP-1, 19B), interleukin 6 (IL-6, 19C), and tumor necrosis factor alpha (TNF-alpha, 19D) levels in serum after in vivo administration of LNPs comprising Cas9 mRNA and sgRNAs.
FIGs 20A and 20B show editing in liver of FVII locus (FIG 20A) and TTR locus (FIG 20B) following in vivo administration of LNPs comprising Cas9 mRNA and sgRNAs.
FIGs 21A, 21B, and 21C show schematics of an annotated sgRNA (SEQ ID NO: 341) (FIG 21A), non-annotated dgRNA CR000686 (SEQ ID NO: 1) and TR000002 (SEQ ID NO: 188) (FIG 21B), and annotated dgRNA CR000686 (SEQ ID NO: 1) and TR000002 (SEQ ID NO: 188) (FIG 21C).
FIG 22A, 22B, and 22C show in vivo results following administration of LNPs comprising Cas9 mRNA and sgRNAs. FIG 22A shows percentage of total editing of TTR locus in liver. FIG 22B summarizes liver editing data. FIG 22C shows serum TTR levels.
FIG 23A, 23B, and 23C show in vivo results following administration of LNPs comprising Cas9 mRNA and sgRNAs. FIG 23A shows percentage of total editing of TTR locus in liver. FIG 23B summarizes liver editing data. FIG 23C shows serum TTR levels.
FIGs 24A, 24B, and 24C show editing in primary mouse hepatocytes following administration of LNPs comprising Cas9 mRNA and sgRNAs. FIG24A shows editing percentage of total editing of TTR locus. FIG24B shows normalized transforms of editing percentage as a function of mRNA dose used to calculate EC50. FIG 24C shows EC50 values for the LNPs tested.

### DETAILED DESCRIPTION

Described herein are modified guide RNAs, including dual and single guide RNAs for use in gene editing methods. The modified guides are more stable and show improved in vitro and in vivo efficacy as compared to their non-modified counterparts. Sequences of engineered and tested guide RNAs are shown in Table 4.

**Table 4:**

| **SEQ ID NO** | **Name** | **Alias** | **Description** | **Sequence** |
|---|---|---|---|---|
| | **crRNA** | | | |
| 1 | CR000686 | | unmodified | CCAGUCCAGCGAGGCAAAGGGUUUUAGAGCUAUGCUGUUUUG |
| 2 | CR003393 | CR686-1 | upper | CCAGUCCAGCGAGGCAAAGGUUUUAGAmGmCmUmAmUmGmCmUm GmUmUmUmUmG |
| 3 | CR003394 | CR686-2 | partial upper | CCAGUCCAGCGAGGCAAAGGUUUUAGAGCUAmUmGmCmUmGmUmU mUmUmG |
| 4 | CR003395 | CR686-3 | partial upper | CCAGUCCAGCGAGGCAAAGGGUUUUAGAGCUAUGCmUmGmUmUmUm UmG |
| 5 | CR003396 | CR686-4 | partial upper | CCAGUCCAGCGAGGCAAAGGGUUUUAGAGCUAUGCUGUmUmUmUmG |
| 6 | CR003397 | CR686-5 | lower | CCAGUCCAGCGAGGCAAAGGmGmUmUmUmUmAGAGCUAUGCUGUUU UG |
| 7 | CR003398 | CR686-6 | lower walk | CCAGUCCAGCGAGGCAAAGGmGUUUUAGAGCUAUGCUGUUUUG |
| 8 | CR003399 | CR686-7 | lower walk | CCAGUCCAGCGAGGCAAAGGGmUUUUAGAGCUAUGCUGUUUUG |
| 9 | CR003400 | CR686-8 | lower walk | CCAGUCCAGCGAGGCAAAGGGUmUUUAGAGCUAUGCUGUUUUG |
| 10 | CR003401 | CR686-9 | lower walk | CCAGUCCAGCGAGGCAAAGGGUUmUUAGAGCUAUGCUGUUUUG |
| 11 | CR003402 | CR686-10 | lower walk | CCAGUCCAGCGAGGCAAAGGGUUUmUAGAGCUAUGCUGUUUUG |
| 12 | CR003403 | CR686-11 | lower walk | CCAGUCCAGCGAGGCAAAGGGUUUUmAGAGCUAUGCUGUUUUG |
| 13 | CR003404 | CR686-12 | partial lower | CCAGUCCAGCGAGGCAAAGGGmUmUmUmUAGAGCUAUGCUGUUUUG |
| 14 | CR003405 | CR686-13 | partial lower | CCAGUCCAGCGAGGCAAAGGGUmUmUUAGAGCUAUGCUGUUUUG |
| 15 | CR003406 | CR686-GC1 | Lower GC | CCAGUCCAGCGAGGCAAAGGGGCGCAGAGCUAUGCUGUUUUG |
| 16 | CR003407 | CR686-GC3 | Upper GC | CCAGUCCAGCGAGGCAAAGGGUUUUAGAGCUAUGCUGGCGCG |
| 17 | CR003408 | CR686-GC5 | Lower Upper GC | CCAGUCCAGCGAGGCAAAGGGGCGCAGAGCUAUGCUGGCGCG |
| 18 | CR003409 | CR686 all OMe | | mCmCmAmGmUmCmCmAmGmCmGmAmGmGmCmAmAmAmGmGm GmUmUmUmUmAmGmAmGmCmUmAmUmGmCmUmGmUmUmUmU mG |
| 19 | CR003393 - mod only | | upper | GUUUUAGAmGmCmUmAmUmGmCmUmGmUmUmUmUmG |
| 20 | CR003394 - mod only | | partial upper | GUUUUAGAGCUAmUmGmCmUmGmUmUmUmUmG |
| 21 | CR003395 - mod only | | partial upper | GUUUUAGAGCUAUGCmUmGmUmUmUmMUmMG |
| 22 | CR003396 - mod only | | partial upper | GUUUUAGAGCUAUGCUGUmUmUmUmG |
| 23 | CR003397 - mod only | | lower | mGmUmUmUmUmAGAGCUAUGCUGUUUUG |
| 24 | CR003398 - mod only | | lower walk | mGUUUUAGAGCUAUGCUGUUUUG |
| 25 | CR003399 - mod only | | lower walk | GmUUUUAGAGCUAUGCUGUUUUG |
| 26 | CR003400 - mod only | | lower walk | GUmUUUAGAGCUAUGCUGUUUUG |
| 27 | CR003401 - mod only | | lower walk | GUUmUUAGAGCUAUGCUGUUUUG |
| 28 | CR003402 - mod only | | lower walk | GUUUmUAGAGCUAUGCUGUUUUG |
| 29 | CR003403 - mod only | | lower walk | GUUUUmAGAGCUAUGCUGUUUUG |
| 30 | CR003404 - mod only | | partial lower | GmUmUmUmUAGAGCUAUGCUGUUUUG |
| 31 | CR003405 - mod only | | partial lower | GUmUmUUAGAGCUAUGCUGUUUUG |
| 32 | CR003721 | CR686-14 | upper and lower | CCAGUCCAGCGAGGCAAAGGmGUUUmUmAGAmGmCmUmAmUmGmC mUmGmUmUmUmUmG |
| 33 | CR003722 | CR686-15 | lower combo | CCAGUCCAGCGAGGCAAAGGmGUUUmUmAGAGCUAUGCUGUUUUG |
| 34 | CR003723 | CR686-16 | upper, lower combo | CCAGUCCAGCGAGGCAAAGGmGUUUUmAGAmGmCmUmAmUmGmCm UmGmUmUmUmUmG |
| 35 | CR003724 | CR686-17 | lower combo | CCAGUCCAGCGAGGCAAAGGmGUUUUmAGAGCUAUGCUGUUUUG |
| 36 | CR003725 | CR686-18 | upper, lower combo | CCAGUCCAGCGAGGCAAAGGmGUUUUAGAmGmCmUmAmUmGmCmU mGmUmUmUmUmG |
| 37 | CR003726 | CR686-19 | nexus walk | CCAGUCCAGCGAGGCAAAGGUUUUAmGAmGmCmUmAmUmGmCmU mGmUmUmUmUmG |
| 38 | CR003727 | CR686-20 | nexus walk | CCAGUCCAGCGAGGCAAAGGGUUUUAGmAmGmCmUmAmUmGmCmU mGmUmUmUmUmG |
| 39 | CR003728 | CR686-21 | nexus walk | CCAGUCCAGCGAGGCAAAGGUUUUAfGAmGmCmUmAmUmGmCmUm GmUmUmUmUmG |
| 40 | CR003729 | CR686-22 | nexus walk | CCAGUCCAGCGAGGCAAAGGGUUUUAGfAmGmCmUmAmUmGmCmUm GmUmUmUmUmG |
| 41 | CR003730 | CR686-23 | 2'F lower walk | CCAGUCCAGCGAGGCAAAGGGfUUUUAGAmGmCmUmAmUmGmCmUm GmUmUmUmUmG |
| 42 | CR003731 | CR686-24 | 2'F lower walk | CCAGUCCAGCGAGGCAAAGGGUfUUUAGAmGmCmUmAmUmGmCmUm GmUmUmUmUmG |
| 43 | CR003732 | CR686-25 | 2'F lower walk | CCAGUCCAGCGAGGCAAAGGGUUfUUAGAmGmCmUmAmUmGmCmUm GmUmUmUmUmG |
| 44 | CR003733 | CR686-26 | 2'F lower walk | CCAGUCCAGCGAGGCAAAGGGUUUfUAGAmGmCmUmAmUmGmCmUm GmUmUmUmUmG |
| 45 | CR003734 | CR686-27 | 2'F lower combo | CCAGUCCAGCGAGGCAAAGGfGfUfUfUfUfAGAmGmCmUmAmUmGmCm UmGmUmUmUmUmG |
| 46 | CR003735 | CR686-28 | lower alt | CCAGUCCAGCGAGGCAAAGGfGmUfUmUfUmAGAmGmCmUmAmUmG mCmUmGmUmUmUmUmG |
| 47 | CR003736 | CR686-29 | lower alt | CCAGUCCAGCGAGGCAAAGGmGfUmUfUmUfAGAmGmCmUmAmUmG mCmUmGmUmUmUmUmG |
| 48 | CR003737 | CR686-GC6 | Lower GC | CCAGUCCAGCGAGGCAAAGGGUCUCAGAmGmCmUmAmUmGmCmUm GmUmUmUmUmG |
| 49 | CR003738 | CR686-GC7 | Lower C walk | CCAGUCCAGCGAGGCAAAGGCUUUAGAmGmCmUmAmUmGmCmUm GmUmUmUmUmG |
| 50 | CR003739 | CR686-GC8 | Lower C walk | CCAGUCCAGCGAGGCAAAGGGUCUUAGAmGmCmUmAmUmGmCmUm GmUmUmUmUmG |
| 51 | CR003740 | CR686-GC9 | Lower C walk | CCAGUCCAGCGAGGCAAAGGGUUCUAGAmGmCmUmAmUmGmCmUm GmUmUmUmUmG |
| 52 | CR003741 | CR686-GC10 | Lower C walk | CCAGUCCAGCGAGGCAAAGGGUUUCAGAmGmCmUmAmUmGmCmUm GmUmUmUmUmG |
| 53 | CR003721-mod only | CR686-14-mod only | upper and lower | mGUUUmUmAGAmGmCmUmAmUmGmCmUmGmUmUmUmUmG |
| 54 | CR003722-mod only | CR686-15-mod only | lower combo | mGUUUmUmAGAGCUAUGCUGUUUUG |
| 55 | CR003723-mod only | CR686-16-mod only | upper, lower combo | mGUUUUmAGAmGmCmUmAmUmGmCmUmGmUmUmUmUmG |
| 56 | CR003724-mod only | CR686-17-mod only | lower combo | mGUUUUmAGAGCUAUGCUGUUUUG |
| 57 | CR003725-mod only | CR686-18-mod only | upper, lower combo | mGUUUUAGAmGmCmUmAmUmGmCmUmGmUmUmUmUmG |
| 58 | CR003726-mod only | CR686-19-mod only | nexus walk | GUUUUAmGAmGCmMmUmAmUmGmCMmUmGmUmUmUmUmG |
| 59 | CR003727-mod only | CR686-20-mod only | nexus walk | GUUUUAGmAmGmCmUmAmUmGmCmUmGmUmUmUmUmG |
| 60 | CR003728-mod only | CR686-21-mod only | nexus walk | GUUUUAfGAmGmCmUmAmUmGmCmUmMGmMUmUmUmMUmMG |
| 61 | CR003729-mod only | CR686-22-mod only | nexus walk | GUUUUAGfAmGmCmUmAmUmGmCmUmGmUmUmUmUmG |
| 62 | CR003730-mod only | CR686-23-mod only | 2'F lower walk | GfUUUUAGAmGmCmUmAmUmGmCmUmMGmMUmUmUmMUmMG |
| 63 | CR003731-mod only | CR686-24-mod only | 2'F lower walk | GUfUUUAGAmGmCmUmAmUmGmCmUmGmUmUmUmUmG |
| 64 | CR003732-mod only | CR686-25-mod only | 2'F lower walk | GUUfUUAGAmGmCmUmAmUmGmCmUmMGmMUmUmUmMUmG |
| 65 | CR003733-mod only | CR686-26-mod only | 2'F lower walk | GUUUfUAGAmGmCmUmAmUmGmCmUmMGmMUmUmUmUmG |
| 66 | CR003734-mod only | CR686-27-mod only | 2'F lower combo | fGfUfUfUfUfAGAmGmCmUmAmUmGmCmUmGmUmUmUmUmG |
| 67 | CR003735-mod only | CR686-28-mod only | lower alt | fGmUfUmUfUmAGAmGmCmUmAmUmGmCmUmGmUmUmUmUmG |
| 68 | CR003736-mod only | CR686-29-mod only | lower alt | mGfUmUfUmUfAGAmGmCmUmAmUmGmCmMUmMGmUmMUmMUmMUmMG |
| 69 | CR003737-mod only | CR686-GC6-mod only | Lower GC | GUCUCAGAmGmCmUmAmUmGmCmUmGmUmUmUmUmG |
| 70 | CR003738-mod only | CR686-GC7-mod only | Lower C walk | GCUUUAGAmGmCmUmAmUmGmCmUmGmUmUmUmUmG |
| 71 | CR003739-mod only | CR686-GC8-mod only | Lower C walk | GUCUUAGAmGmCmUmAmUmGmCmUmGmUmUmUmUmG |
| 72 | CR003740-mod only | CR686-GC9-mod only | Lower C walk | GUUCUAGAmGmCmUmAmUmGmCmUmGmUmUmUmUmG |
| 73 | CR003741-mod only | CR686-GC10-mod only | Lower C walk | GUUUCAGAmGmCmUmAmUmGmCmUmGmUmUmUmUmG |
| 74 | CR000705 | | unmodified | UUACAGCCACGUCUACAGCAGUUUUAGAGCUAUGCUGUUUUG |
| 75 | CR004188 | CR705-1 | upper | UUACAGCCACGUCUACAGCAGUUUUAGAmGmCmUmAmUmGmCmUm GmUmUmUmUmG |
| 76 | CR004189 | CR705-2 | partial upper | UUACAGCCACGUCUACAGCAGUUUUAGAGCUAmUmGmCmUmGmUmU mUmUmG |
| 77 | CR004190 | CR705-3 | partial upper | UUACAGCCACGUCUACAGCAGUUUUAGAGCUAUGCmUmGmUmUmUm UmG |
| 78 | CR004191 | CR705-4 | partial upper | UUACAGCCACGUCUACAGCAGUUUUAGAGCUAUGCUGUmUmUmUmG |
| 79 | CR004192 | CR705-5 | lower | UUACAGCCACGUCUACAGCAmGmUmUmUmUmAGAGCUAUGCUGUUU UG |
| 80 | CR004193 | CR705-6 | lower walk | UUACAGCCACGUCUACAGCAmGUUUUAGAGCUAUGCUGUUUUG |
| 81 | CR004194 | CR705-7 | lower walk | UUACAGCCACGUCUACAGCAGmUUUUAGAGCUAUGCUGUUUUG |
| 82 | CR004195 | CR705-8 | lower walk | UUACAGCCACGUCUACAGCAGUmUUUAGAGCUAUGCUGUUUUG |
| 83 | CR004196 | CR705-9 | lower walk | UUACAGCCACGUCUACAGCAGUUmUUAGAGCUAUGCUGUUUUG |
| 84 | CR004197 | CR705-10 | lower walk | UUACAGCCACGUCUACAGCAGUUUmUAGAGCUAUGCUGUUUUG |
| 85 | CR004198 | CR705-11 | lower walk | UUACAGCCACGUCUACAGCAGUUUUmAGAGCUAUGCUGUUUUG |
| 86 | CR004199 | CR705-14 | upper and lower | UUACAGCCACGUCUACAGCAmGUUUmUmAGAmGmCmUmAmUmGmC mUmGmUmUmUmUmG |
| 87 | CR004200 | CR705-15 | lower combo | UUACAGCCACGUCUACAGCAmGUUUmUmAGAGCUAUGCUGUUUUG |
| 88 | CR004201 | CR705-16 | upper, lower combo | UUACAGCCACGUCUACAGCAmGUUUUmAGAmGmCmUmAmUmGmCm UmGmUmUmUmUmG |
| 89 | CR004202 | CR705-17 | lower combo | UUACAGCCACGUCUACAGCAmGUUUUmAGAGCUAUGCUGUUUUG |
| 90 | CR004203 | CR705-18 | upper, lower combo | UUACAGCCACGUCUACAGCAmGUUUUAGAmGmCmUmAmUmGmCmU mGmUmUmUmUmG |
| 91 | CR004204 | CR705-19 | nexus walk | UUACAGCCACGUCUACAGCAGUUUUAmGAmGmCmUmAmUmGmCmU mGmUmUmUmUmG |
| 92 | CR004205 | CR705-20 | nexus walk | UUACAGCCACGUCUACAGCAGUUUUAGmAmGmCmUmAmUmGmCmU mGmUmUmUmUmG |
| 93 | CR004206 | CR705-21 | nexus walk | UUACAGCCACGUCUACAGCAGUUUUAfGAmGmCmUmAmUmGmCmUm GmUmUmUmUmG |
| 94 | CR004207 | CR705-22 | nexus walk | UUACAGCCACGUCUACAGCAGUUUUAGfAmGmCmUmAmUmGmCmUm GmUmUmUmUmG |
| 95 | CR004208 | CR705-23 | 2'F lower walk | UUACAGCCACGUCUACAGCAGfUUUUAGAmGmCmUmAmUmGmCmUm GmUmUmUmUmG |
| 96 | CR004209 | CR705-24 | 2'F lower walk | UUACAGCCACGUCUACAGCAGUfUUUAGAmGmCmUmAmUmGmCmUm GmUmUmUmUmG |
| 97 | CR004210 | CR705-25 | 2'F lower walk | GmUmUmUmUmG UUACAGCCACGUCUACAGCAGUUfUUAGAmGmCmUmAmUmGmCmUm GmUmUmUmUmG |
| 98 | CR004211 | CR705-26 | 2'F lower walk | UUACAGCCACGUCUACAGCAGUUUfUAGAmGmCmUmAmUmGmCmUm GmUmUmUmUmG |
| 99 | CR004212 | CR705-27 | 2'F lower combo | UUACAGCCACGUCUACAGCAfGfUfUfUfUfAGAmGmCmUmAmUmGmCm UmGmUmUmUmUmG |
| 100 | CR004213 | CR705-28 | lower alt | UUACAGCCACGUCUACAGCAfGmUfUmUfUmAGAmGmCmUmAmUmG mCmUmGmUmUmUmUmG |
| 101 | CR004214 | CR705-29 | lower alt | UUACAGCCACGUCUACAGCAmGfUmUfUmUfAGAmGmCmUmAmUmG mCmUmGmUmUmUmUmG |
| 102 | CR004215 | CR705-GC1 | Lower GC | UUACAGCCACGUCUACAGCAGGCGCAGAGCUAUGCUGUUUUG |
| 103 | CR004216 | CR705-GC3 | Upper GC | UUACAGCCACGUCUACAGCAGUUUUAGAGCUAUGCUGGCGCG |
| 104 | CR004188-mod only | CR705-1-mod only | upper | GUUUUAGAmGmCmUmAmUmGmCmUmGmUmUmUmUmG |
| 105 | CR004189-mod only | CR705-2-mod only | partial upper | GUUUUAGAGCUAmUmGmCmUmGmUmUmUmUmG |
| 106 | CR004190-mod only | CR705-3-mod only | partial upper | GUUUUAGAGCUAUGCmUmGmUmUmUmMUmMG |
| 107 | CR004191-mod only | CR705-4-mod only | partial upper | GUUUUAGAGCUAUGCUGUmUmUmUmG |
| 108 | CR004192-mod only | CR705-5-mod only | lower | mGmUmUmUmUmAGAGCUAUGCUGUUUUG |
| 109 | CR004193-mod only | CR705-6-mod only | lower walk | mGUUUUAGAGCUAUGCUGUUUUG |
| 110 | CR004194-mod only | CR705-7-mod only | lower walk | GmUUUUAGAGCUAUGCUGUUUUG |
| 111 | CR004195-mod only-mod only | CR705-8-mod only | lower walk | GUmUUUAGAGCUAUGCUGUUUUG |
| 112 | CR004196-mod only | CR705-9-mod only | lower walk | GUUmUUAGAGCUAUGCUGUUUUG |
| 113 | CR004197-mod only | CR705-10-mod only | lower walk | GUUUmUAGAGCUAUGCUGUUUUG |
| 114 | CR004198-mod only | CR705-11-mod only | lower walk | GUUUUmAGAGCUAUGCUGUUUUG |
| 115 | CR004199-mod only | CR705-14-mod only | upper and lower | mGUUUmUmAGAmGmCmUmAmUmGmCmUmGmUmUmUmUmG |
| 116 | CR004200-mod only | CR705-15-mod only | lower combo | mGUUUmUmAGAGCUAUGCUGUUUUG |
| 117 | CR004201-mod only | CR705-16-mod only | upper, lower combo | mGUUUUmAGAmGmCmUmAmUmGmCmUmGmUmUmUmUmG |
| 118 | CR004202-mod only | CR705-17-mod only | lower combo | mGUUUUmAGAGCUAUGCUGUUUUG |
| 119 | CR004203-mod only | CR705-18-mod only | upper, lower combo | mGUUUUAGAmGmCmUmAmUmGmCmUmGmUmUmUmUmG |
| 120 | CR004204-mod only | CR705-19-mod only | nexus walk | GUUUUAmGAmGCmMmUmAmUmGmCMmUmGmUmUmUmUmG |
| 121 | CR004205-mod only | CR705-20-mod only | nexus walk | GUUUUAGmAmGmCmUmAmUmGmCmUmGmUmUmUmUmG |
| 122 | CR004206-mod only | CR705-21-mod only | nexus walk | GAmGmCmUmAmUmGmCmUmGmUmUmUmUmG |
| 123 | CR004207-mod only | CR705-22-mod only | nexus walk | GfAmGmCmUmAmUmGmCMmUmGmUmUmUmUmG |
| 124 | CR004208-mod only | CR705-23-mod only | 2'F lower walk | GfUUUUAGAmGmCmUmAmUmGmCmUmGmUmUmUmUmG |
| 125 | CR004209-mod only | CR705-24-mod only | 2'F lower walk | GUfUUUAGAmGmCmUmAmUmGmCmUmGmUmUmUmUmG |
| 126 | CR004210-mod only | CR705-25-mod only | 2'F lower walk | GUUfUUAGAmGmCmUmAmUmGmCmUmMGmMUmUmUmMUmG |
| 127 | CR004211-mod only | CR705-26-mod only | 2'F lower walk | GUUUfUAGAmGmCmUmAmUmGmCmUmMGmMUmUmUmUmG |
| 128 | CR004212-mod only | CR705-27-mod only | 2'F lower combo | fGfUfUfUfUfAGAmGmCmUmAmUmGmCmUmGmUmUmUmUmG |
| 129 | CR004213-mod only | CR705-28-mod only | lower alt | fGmUfUmUfUmAGAmGmCmUmAmUmGmCmUmGmUmUmUmUmG |
| 130 | CR004214-mod only | CR705-29-mod only | lower alt | mGfUmUfUmUfAGAmGmCmUmAmUmGmCmMUmMGmUmMUmMUmMUmMG |
| 131 | CR000657 | | unmodified | CAGGGCUCUUGAAGAUCUCCGUUUUAGAGCUAUGCUGUUUUG |
| 132 | CR004218 | CR657-1 | upper | CAGGGCUCUUGAAGAUCUCCGUUUUAGAmGmCmUmAmUmGmCmUm GmUmUmUmUmG |
| 133 | CR004219 | CR657-2 | partial upper | CAGGGCUCUUGAAGAUCUCCGUUUUAGAGCUAmUmGmCmUmGmUm UmUmUmG |
| 134 | CR004220 | CR657-3 | partial upper | CAGGGCUCUUGAAGAUCUCCGUUUUAGAGCUAUGCmUmGmUmUmUm UmG |
| 135 | CR004221 | CR657-4 | partial upper | CAGGGCUCUUGAAGAUCUCCGUUUUAGAGCUAUGCUGUmUmUmUmG |
| 136 | CR004222 | CR657-5 | lower | CAGGGCUCUUGAAGAUCUCCmGmUmUmUmUmAGAGCUAUGCUGUUU UG |
| 137 | CR004223 | CR657-6 | lower walk | CAGGGCUCUUGAAGAUCUCCmGUUUUAGAGCUAUGCUGUUUUG |
| 138 | CR004224 | CR657-7 | lower walk | CAGGGCUCUUGAAGAUCUCCGmUUUUAGAGCUAUGCUGUUUUG |
| 139 | CR004225 | CR657-8 | lower walk | CAGGGCUCUUGAAGAUCUCCGUmUUUAGAGCUAUGCUGUUUUG |
| 140 | CR004226 | CR657-9 | lower walk | CAGGGCUCUUGAAGAUCUCCGUUmUUAGAGCUAUGCUGUUUUG |
| 141 | CR004227 | CR657-10 | lower walk | CAGGGCUCUUGAAGAUCUCCGUUUmUAGAGCUAUGCUGUUUUG |
| 142 | CR004228 | CR657-11 | lower walk | CAGGGCUCUUGAAGAUCUCCGUUUUmAGAGCUAUGCUGUUUUG |
| 143 | CR004229 | CR657-14 | upper and lower | CAGGGCUCUUGAAGAUCUCCmGUUUmUmAGAmGmCmUmAmUmGmC mUmGmUmUmUmUmG |
| 144 | CR004230 | CR657-15 | lower combo | CAGGGCUCUUGAAGAUCUCCmGUUUmUmAGAGCUAUGCUGUUUUG |
| 145 | CR004231 | CR657-16 | upper, lower combo | CAGGGCUCUUGAAGAUCUCCmGUUUUmAGAmGmCmUmAmUmGmCm UmGmUmUmUmUmG |
| 146 | CR004232 | CR657-17 | lower combo | CAGGGCUCUUGAAGAUCUCCmGUUUUmAGAGCUAUGCUGUUUUG |
| 147 | CR004233 | CR657-18 | upper, lower combo | CAGGGCUCUUGAAGAUCUCCmGUUUUAGAmGmCmUmAmUmGmCmU mGmUmUmUmUmG |
| 148 | CR004234 | CR657-19 | nexus walk | CAGGGCUCUUGAAGAUCUCCGUUUUAmGAmGmCmUmAmUmGmCmU mGmUmUmUmUmG |
| 149 | CR004235 | CR657-20 | nexus walk | CAGGGCUCUUGAAGAUCUCCGUUUUAGmAmGmCmUmAmUmGmCmU mGmUmUmUmUmG |
| 150 | CR004236 | CR657-21 | nexus walk | CAGGGCUCUUGAAGAUCUCCGUUUUAfGAmGmCmUmAmUmGmCmU mGmUmUmUmUmG |
| 151 | CR004237 | CR657-22 | nexus walk | CAGGGCUCUUGAAGAUCUCCGUUUUAGfAmGmCmUmAmUmGmCmU mGmUmUmUmUmG |
| 152 | CR004238 | CR657-23 | 2'F lower walk | CAGGGCUCUUGAAGAUCUCCGfUUUUAGAmGmCmUmAmUmGmCmU mGmUmUmUmUmG |
| 153 | CR004239 | CR657-24 | 2'F lower walk | CAGGGCUCUUGAAGAUCUCCGUfUUUAGAmGmCmUmAmUmGmCmU mGmUmUmUmUmG |
| 154 | CR004240 | CR657-25 | 2'F lower walk | CAGGGCUCUUGAAGAUCUCCGUUfUUAGAmGmCmUmAmUmGmCmU mGmUmUmUmUmG |
| 155 | CR004241 | CR657-26 | 2'F lower walk | CAGGGCUCUUGAAGAUCUCCGUUUfUAGAmGmCmUmAmUmGmCmU mGmUmUmUmUmG |
| 156 | CR004242 | CR657-27 | 2'F lower combo | CAGGGCUCUUGAAGAUCUCCfGfUfUfUfUfAGAmGmCmUmAmUmGmC mUmGmUmUmUmUmG |
| 157 | CR004243 | CR657-28 | lower alt | CAGGGCUCUUGAAGAUCUCCfGmUfUmUfUmAGAmGmCmUmAmUmG mCmUmGmUmUmUmUmG |
| 158 | CR004244 | CR657-29 | lower alt | CAGGGCUCUUGAAGAUCUCCmGfUmUfUmUfAGAmGmCmUmAmUmG mCmUmGmUmUmUmUmG |
| 159 | CR004245 | CR657-GC1 | Lower GC | CAGGGCUCUUGAAGAUCUCCGGCGCAGAGCUAUGCUGUUUUG |
| 160 | CR004246 | CR657-GC3 | Upper GC | CAGGGCUCUUGAAGAUCUCCGUUUUAGAGCUAUGCUGGCGCG |
| 161 | CR004218-mod only | CR657-1-mod only | upper | GUUUUAGAmGmCmUmAmUmGmCmUmGmUmUmUmUmG |
| 162 | CR004219-mod only | CR657-2-mod only | partial upper | GUUUUAGAGCUAmUmGmCmUmGmUmUmUmUmG |
| 163 | CR004220-mod only | CR657-3-mod only | partial upper | GUUUUAGAGCUAUGCmUmGmUmUmUmMUmMG |
| 164 | CR004221-mod only | CR657-4-mod only | partial upper | GUUUUAGAGCUAUGCUGUmUmUmUmG |
| 165 | CR004222-mod only | CR657-5-mod only | lower | mGmUmUmUmUmAGAGCUAUGCUGUUUUG |
| 166 | CR004223-mod only | CR657-6-mod only | lower walk | mGUUUUAGAGCUAUGCUGUUUUG |
| 167 | CR004224-mod only | CR657-7-mod only | lower walk | GmUUUUAGAGCUAUGCUGUUUUG |
| 168 | CR004225-mod only | CR657-8-mod only | lower walk | GUmUUUAGAGCUAUGCUGUUUUG |
| 169 | CR004226-mod only | CR657-9-mod only | lower walk | GUUmUUAGAGCUAUGCUGUUUUG |
| 170 | CR004227-mod only | CR657-10-mod only | lower walk | GUUUmUAGAGCUAUGCUGUUUUG |
| 171 | CR004228-mod only | CR657-11-mod only | lower walk | GUUUUmAGAGCUAUGCUGUUUUG |
| 172 | CR004229-mod only | CR657-14-mod only | upper and lower | mGUUUmUmAGAmGmCmUmAmUmGmCmUmGmUmUmUmUmG |
| 173 | CR004230-mod only | CR657-15-mod only | lower combo | mGUUUmUmAGAGCUAUGCUGUUUUG |
| 174 | CR004231-mod only | CR657-16-mod only | upper, lower combo | mGUUUUmAGAmGmCmUmAmUmGmCmUmGmUmUmUmUmG |
| 175 | CR004232-mod only | CR657-17-mod only | lower combo | mGUUUUmAGAGCUAUGCUGUUUUG |
| 176 | CR004233-mod only | CR657-18-mod only | upper, lower combo | mGUUUUAGAmGmCmUmAmUmGmCmUmGmUmUmUmUmG |
| 177 | CR004234-mod only | CR657-19-mod only | nexus walk | GUUUUAmGAmGCmMmUmAmUmGmCMmUmGmUmUmUmUmG |
| 178 | CR004235-mod only | CR657-20-mod only | nexus walk | GUUUUAGmAmGmCmUmAmUmGmCmUmGmUmUmUmUmG |
| 179 | CR004236-mod only | CR657-21-mod only | nexus walk | GUUUUAfGAmGmCmUmAmUmGmCmUmGmUmUmUmUmG |
| 180 | CR004237-mod only | CR657-22-mod only | nexus walk | GUUUUAGfAmGmCmUmAmUmGmCmUmGmUmUmUmUmG |
| 181 | CR004238-mod only | CR657-23-mod only | 2'F lower walk | GfUUUUAGAmGmCmUmAmUmGmCmUmGmUmUmUmUmG |
| 182 | CR004239-mod only | CR657-24-mod only | 2'F lower walk | GUfUUUAGAmGmCmUmAmUmGmCmUmGmUmUmUmUmG |
| 183 | CR004240-mod only | CR657-25-mod only | 2'F lower walk | GUUfUUAGAmGmCmUmAmUmGmCmUmMGmMUmUmUmMUmG |
| 184 | CR004241-mod only | CR657-26-mod only | 2'F lower walk | GUUUfUAGAmGmCmUmAmUmGmCmUmMGmMUmUmUmUmG |
| 185 | CR004242-mod only | CR657-27-mod only | 2'F lower combo | fGfUfUfUfUfAGAmGmCmUmAmUmGmCmUmGmUmUmUmUmG |
| 186 | CR004243-mod only | CR657-28-mod only | lower alt | fGmUfUmUfUmAGAmGmCmUmAmUmGmCmUmGmUmUmUmUmG |
| 187 | CR004244-mod only | CR657-29-mod only | lower alt | mGfUmUfUmUfAGAmGmCmUmAmUmGmCmMUmMGmUmMUmMUmMUmMG |

| | **trRNA** | | | |
|---|---|---|---|---|
| 188 | TR000002 | | unmodified | AACAGCAUAGCAAGUUAAAAUAAGGCUAGUCCGUUAUCAACUUGAAAAAG UGGCACCGAGUCGGUGCUUUUUUU |
| 189 | TR000110 | TR2-v2-1 | shortened tail | AACAGCAUAGCAAGUUAAAAUAAGGCUAGUCCGUUAUCAACUUGAAAAAG UGGCACCGAGUCGGUGCUUUU |
| 190 | TR000111 | TR2-v2-2 | Upper, hairpins | mAmAmCmAmGmCmAmUmAmGmCAAGUUAAAAUAAGGCUAGUCCGUU AUCAmAmCmUmUmGmAmAmAmAmAmGmUmGmGmCmAmCmCmGm AmGmUmCmGmGmUmGmCmUmUmUmU |
| 191 | TR000112 | TR2-v2-3 | upper only | mAmAmCmAmGmCmAmUmAmGmCAAGUUAAAAUAAGGCUAGUCCGUU AUCAACUUGAAAAAGUGGCACCGAGUCGGUGCUUUU |
| 192 | TR000113 | TR2-v2-4 | hairpin 1 | AACAGCAUAGCAAGUUAAAAUAAGGCUAGUCCGUUAUCAmAmCmUmUm GmAmAmAmAmAmGmUmGGCACCGAGUCGGUGCUUUU |
| 193 | TR000114 | TR2-v2-5 | hairpin 2 | AACAGCAUAGCAAGUUAAAAUAAGGCUAGUCCGUUAUCAACUUGAAAAAG UGmGmCmAmCmCmGmAmGmUmCmGmGmUmGmCmUmUmUmU |
| 194 | TR000115 | TR2-v2-6 | upper, hairpin 2 | mAmAmCmAmGmCmAmUmAmGmCAAGUUAAAAUAAGGCUAGUCCGUU AUCAACUUGAAAAAGUGmGmCmAmCmCmGmAmGmUmCmGmGmUmG mCmUmUmUmU |
| 195 | TR000116 | TR2-v2-7 | both hairpins | AACAGCAUAGCAAGUUAAAAUAAGGCUAGUCCGUUAUCAmAmCmUmUm GmAmAmAmAmAmGmUmGmGmCmAmCmCmGmAmGmUmCmGmGm UmGmCmUmUmUmU |
| 196 | TR000117 | TR2-v2-8 | lower walk | AACAGCAUAGCAAGUmUmAAAAUAAGGCUAGUCCGUUAUCAACUUGAAA AAGUGGCACCGAGUCGGUGCUUUU |
| 197 | TR000118 | TR2-v2-9 | lower walk | AACAGCAUAGCAAGUUAmAmAAUAAGGCUAGUCCGUUAUCAACUUGAAA AAGUGGCACCGAGUCGGUGCUUUU |
| 198 | TR000119 | TR2-v2-10 | lower walk | AACAGCAUAGCAAGUUAAAmAmUAAGGCUAGUCCGUUAUCAACUUGAAA AAGUGGCACCGAGUCGGUGCUUUU |
| 199 | TR000120 | TR2-v2-11 | partial nexus | AACAGCAUAGCAAGUUAAAAUAAGGCUAGUCCGmUmUmAUCAACUUGA AAAAGUGGCACCGAGUCGGUGCUUUU |
| 200 | TR000121 | TR2-v2-12 | partial nexus | AACAGCAUAGCAAGUUAAAAUAAGGCUAGUCCGUUAmUmCmAACUUGA AAAAGUGGCACCGAGUCGGUGCUUUU |
| 201 | TR000122 | TR2-GC1 | Lower GC | AACAGCAUAGCAAGUUGCGCUAAGGCUAGUCCGUUAUCAACUUGAAAAAG UGGCACCGAGUCGGUGCUUUU |
| 202 | TR000123 | TR2-GC3 | upper GC | GCCAGCAUAGCAAGUUAAAAUAAGGCUAGUCCGUUAUCAACUUGAAAAAG UGGCACCGAGUCGGUGCUUUU |
| 203 | TR000124 | TR2-GC5 | Lower Upper GC | GCCAGCAUAGCAAGUUGCGCUAAGGCUAGUCCGUUAUCAACUUGAAAAA GUGGCACCGAGUCGGUGCUUUU |
| 204 | TR000125 | TR2 all OMe | | mAmAmCmAmGmCmAmUmAmGmCmAmAmGmUmUmAmAmAmAmU mAmAmGmGmCmUmAmGmUmCmCmGmUmUmAmUmCmAmAmCmU mUmGmAmAmAmAmAmGmUmGmGmCmAmCmCmGmAmGmUmCmG mGmUmGmCmUmUmUmUmUmUmU |
| 205 | TR000126 | TR2-v2-13 | lower | mAmAmCmAmGmCmAmUmAmGmCAAGUmUmAmAmAmAmUAAGGCU AGUCCGUUAUCAmAmCmUmUmGmAmAmAmAmAmGmUmGmGmCmA mCmCmGmAmGmUmCmGmGmUmGmCmUmUmUmU |
| 206 | TR000127 | TR2-v2-14 | lower | mAmAmCmAmGmCmAmUmAmGmCAAGUmUmAAmAmUAAGGCUAG UCCGUUAUCAmAmCmUmUmGmAmAmAmAmGmUmGmGmCmAmC mCmGmAmGmUmCmGmGmUmGmCmUmUmUmU |
| 207 | TR000128 | TR2-v2-15 | lower | mAmAmCmAmGmCmAmUmAmGmCAAGUmUmAfAfAmAmUAAGGCUA GUCCGUUAUCAmAmCmUmUmGmAmAmAmAmAmGmUmGmGmCmAm CmCmGmAmGmUmCmGmGmUmGmCmUmUmUmU |
| 208 | TR000129 | TR2-v2-16 | lower alt | mAmAmCmAmGmCmAmUmAmGmCAAGUmUfAmAfAmAfUAAGGCUAG UCCGUUAUCAmAmCmUmUmGmAmAmAmAmGmUmGmGmCmAmC mCmGmAmGmUmCmGmGmUmGmCmUmUmUmU |
| 209 | TR000130 | TR2-v2-17 | lower alt | mAmAmCmAmGmCmAmUmAmGmCAAGUfUmAfAmAfAmUAAGGCUAG UCCGUUAUCAmAmCmUmUmGmAmAmAmAmAmGmUmGmGmCmAmC mCmGmAmGmUmCmGmGmUmGmCmUmUmUmU |
| 210 | TR000131 | TR2-v2-18 | nexus walk | mAmAmCmAmGmCmAmUmAmGmCAAGUUAAAAUAAGGCUAGUCCGUU AUCmAmAmCmUmUmGmAmAmAmAmAmGmUmGmGmCmAmCmCmG mAmGmUmCmGmGmUmGmCmUmUmUmU |
| 211 | TR000132 | TR2-v2-19 | nexus walk | mAmAmCmAmGmCmAmUmAmGmCAAGUUAAAAUAAGGCUAGUCCGUU AUmCAmAmCmUmUmGmAmAmAmAmAmGmUmGmGmCmAmCmCmG mAmGmUmCmGmGmUmGmCmUmUmUmU |
| 212 | TR000133 | TR2-v2-20 | nexus walk | mAmAmCmAmGmCmAmUmAmGmCAAGUUAAAAUAAGGCUAGUCCGUU AmUCAmAmCmUmUmGmAmAmAmAmAmGmUmGmGmCmAmCmCmG mAmGmUmCmGmGmUmGmCmUmUmUmU |
| 213 | TR000134 | TR2-v2-21 | nexus walk | mAmAmCmAmGmCmAmUmAmGmCAAGUUAAAAUAAGGCUAGUCCGUU mAUCAmAmCmUmUmGmAmAmAmAmAmGmUmGmGmCmAmCmCmG mAmGmUmCmGmGmUmGmCmUmUmUmU |
| 214 | TR000135 | TR2-v2-22 | nexus walk | mAmAmCmAmGmCmAmUmAmGmCAAGUUAAAAUAAGGCUAGUCCGU mUAUCAmAmCmUmUmGmAmAmAmAmAmGmUmGmGmCmAmCmCm GmAmGmUmCmGmGmUmGmCmUmUmUmU |
| 215 | TR000136 | TR2-v2-23 | nexus walk | mAmAmCmAmGmCmAmUmAmGmCAAGUUAAAAUAAGGCUAGUCCGm UUAUCAmAmCmUmUmGmAmAmAmAmAmGmUmGmGmCmAmCmCm GmAmGmUmCmGmGmUmGmCmUmUmUmU |
| 216 | TR000137 | TR2-v2-24 | nexus walk | mAmAmCmAmGmCmAmUmAmGmCAAGUUAAAAUAAGGCUAGUCCGUU AUfCfAmAmCmUmUmGmAmAmAmAmAmGmUmGmGmCmAmCmCmG mAmGmUmCmGmGmUmGmCmUmUmUmU |
| 217 | TR000138 | TR2-v2-25 | nexus walk | mAmAmCmAmGmCmAmUmAmGmCAAGUUAAAAUAAGGCUAGUCCGUU fAfUCAmAmCmUmUmGmAmAmAmAmAmGmUmGmGmCmAmCmCmG mAmGmUmCmGmGmUmGmCmUmUmUmU |
| 218 | TR000139 | TR2-v2-26 | nexus walk | mAmAmCmAmGmCmAmUmAmGmCAAGUUAAAAUAAGGCUAGUCCGfUf UAUCAmAmCmUmUmGmAmAmAmAmAmGmUmGmGmCmAmCmCmG mAmGmUmCmGmGmUmGmCmUmUmUmU |
| 219 | TR000140 | TR2-v2-27 | nexus walk | mAmAmCmAmGmCmAmUmAmGmCAAGUUAAAAUAmGmGmCUAGUC CGUUAUCAmAmCmUmUmGmAmAmAmAmAmGmUmGmGmCmAmCm CmGmAmGmUmCmGmGmUmGmCmUmUmUmU |
| 220 | TR000141 | TR2-v2-28 | nexus walk | mAmAmCmAmGmCmAmUmAmGmCAAGUUAAAAUAAGGCUAGUmCmC mGUUAUCAmAmCmUmUmGmAmAmAmAmAmGmUmGmGmCmAmCm CmGmAmGmUmCmGmGmUmGmCmUmUmUmU |
| 221 | TR000142 | TR2-v2-29 | bulge walk | mAmAmCmAmGmCmAmUmAmGmCmAmAGUUAAAAUAAGGCUAGUCC GUUAUCAmAmCmUmUmGmAmAmAmAmAmGmUmGmGmCmAmCmC mGmAmGmUmCmGmGmUmGmCmUmUmUmU |
| 222 | TR000143 | TR2-v2-30 | bulge walk | mAmAmCmAmGmCmAmUmAmGmCAAmGmUUAAAAUAAGGCUAGUCC GUUAUCAmAmCmUmUmGmAmAmAmAmAmGmUmGmGmCmAmCmC mGmAmGmUmCmGmGmUmGmCmUmUmUmU |
| 223 | TR000144 | TR2-GC6 | Lower GC | AACAGCAUAGCAAGUUGAGAUAAGGCUAGUCCGUUAUCAACUUGAAAAA GUGGCACCGAGUCGGUGCUUUU |
| 224 | TR000145 | TR2-GC7 | Lower GC walk | AACAGCAUAGCAAGUUAAAGUAAGGCUAGUCCGUUAUCAACUUGAAAAA GUGGCACCGAGUCGGUGCUUUU |
| 225 | TR000146 | TR2-GC8 | Lower GC walk | AACAGCAUAGCAAGUUAAGAUAAGGCUAGUCCGUUAUCAACUUGAAAAA GUGGCACCGAGUCGGUGCUUUU |
| 226 | TR000147 | TR2-GC9 | Lower GC walk | AACAGCAUAGCAAGUUAGAAUAAGGCUAGUCCGUUAUCAACUUGAAAAA GUGGCACCGAGUCGGUGCUUUU |
| 227 | TR000148 | TR2-GC10 | Lower GC walk | AACAGCAUAGCAAGUUGAAAUAAGGCUAGUCCGUUAUCAACUUGAAAAA GUGGCACCGAGUCGGUGCUUUU |

| | **sgRNA** | | | |
|---|---|---|---|---|
| 228 | G000209 | | | mC*mC*mA*GUCCAGCGAGGCAAAGGGUUUUAGAGCUAGAAAUAGCAAG UUAAAAUAAGGCUAGUCCGUUAUCAACUUGAAAAAGUGGCACCGAGUCG GUGCmU*mU*mU*U |
| 229 | G000262 | G209-1 | hairpin 2 | mC*mC*mA*GUCCAGCGAGGCAAAGGGUUUUAGAGCUAGAAAUAGCAAG UUAAAAUAAGGCUAGUCCGUUAUCAACUUGAAAAAGUmGmGmCmAmC mCmGmAmGmUmCmGmGmUmGmCmU*mU*mU*mU |
| 230 | G000263 | G209-2 | hairpins | mC*mC*mA*GUCCAGCGAGGCAAAGGUUUUAGAGCUAGAAAUAGCAAG UUAAAAUAAGGCUAGUCCGUUAUCAmAmCmUmUmGmAmAmAmAmAm GmUmGmGmCmAmCmCmGmAmGmUmCmGmGmUmGmCmU*mU*mU *mU |
| 231 | G000264 | G209-3 | tetraloop | mC*mC*mA*GUCCAGCGAGGCAAAGGGUUUUAGAGCUAmGmAmAmAU AGCAAGUUAAAAUAAGGCUAGUCCGUUAUCAACUUGAAAAAGUGGCACC GAGUCGGUGCmU*mU*mU*U |
| 232 | G000265 | G209-4 | upper | mC*mC*mA*GUCCAGCGAGGCAAAGGGUUUUAGAmGmCmUmAGAAAm UmAmGmCAAGUUAAAAUAAGGCUAGUCCGUUAUCAACUUGAAAAAGUG GCACCGAGUCGGUGCmU*mU*mU*U |
| 233 | G000266 | G209-5 | upper and loop | mC*mC*mA*GUCCAGCGAGGCAAAGGGUUUUAGAmGmCmUmAmGmA mAmAmUmAmGmCAAGUUAAAAUAAGGCUAGUCCGUUAUCAACUUGAA AAAGUGGCACCGAGUCGGUGCmU*mU*mU*U |
| 234 | G000267 | G209-6 | upper, loop, hairpins | mC*mC*mA*GUCCAGCGAGGCAAAGGGUUUUAGAmGmCmUmAmGmA mAmAmUmAmGmCAAGUUAAAAUAAGGCUAGUCCGUUAUCAmAmCmU mUmGmAmAmAmAmAmGmUmGmGmCmAmCmCmGmAmGmUmCmG mGmUmGmCmU*mU*mU*mU |
| 235 | G000262-mod only | G209-1-mod only | hairpin 2 | GUUUUAGAGCUAGAAAUAGCAAGUUAAAAUAAGGCUAGUCCGUUAUCAA CUUGAAAAAGUmGmGmCmAmCmCmGmAmGmUmCmGmGmUmGmCm U*mU*mU*mU |
| 236 | G000263-mod only | G209-2-mod only | hairpins | GUUUUAGAGCUAGAAAUAGCAAGUUAAAAUAAGGCUAGUCCGUUAUCAm AmCmUmUmGmAmAmAmAmAmGmUmGmGmCmAmCmCmGmAmGm UmCmGmGmUmGmCmU*mU*mU*mU |
| 237 | G000264-mod only | G209-3-mod only | tetraloop | GUUUUAGAGCUAmGmAmAmAUAGCAAGUUAAAAUAAGGCUAGUCCGU UAUCAACUUGAAAAAGUGGCACCGAGUCGGUGCmU*mU*mU*U |
| 238 | G000265-mod only | G209-4-mod only | upper | GUUUUAGAmGmCmUmAGAAAmUmAmGmCAAGUUAAAAUAAGGCUAG UCCGUUAUCAACUUGAAAAAGUGGCACCGAGUCGGUGCmU*mU*mU*U |
| 239 | G000266-mod only | G209-5-mod only | upper and loop | GUUUUAGAmGmCmUmAmGmAmAmAmUmAmGmCAAGUUAAAAUAAG GCUAGUCCGUUAUCAACUUGAAAAAGUGGCACCGAGUCGGUGCmU*mU* mU*U |
| 240 | G000267-mod only | G209-6-mod only | upper, loop, hairpins | GUUUUAGAmGmCmUmAmGmAmAmAmUmAmGmCAAGUUAAAAUAAG GCUAGUCCGUUAUCAmAmCmUmUmGmAmAmAmAmAmGmUmGmGm CmAmCmCmGmAmGmUmCmGmGmUmGmCmU*mU*mU*mU |
| 241 | G000211 | | end mod | mU*mU*mA*CAGCCACGUCUACAGCAGUUUUAGAGCUAGAAAUAGCAAG UUAAAAUAAGGCUAGUCCGUUAUCAACUUGAAAAAGUGGCACCGAGUCG GUGCmU*mU*mU*U |
| 242 | G000282 | | mod6 | mU*mU*mA*CAGCCACGUCUACAGCAGUUUUAGAmGmCmUmAmGmAm AmAmUmAmGmCAAGUUAAAAUAAGGCUAGUCCGUUAUCAmAmCmUm UmGmAmAmAmAmAmGmUmGmGmCmAmCmCmGmAmGmUmCmGm GmUmGmCmU*mU*mU*mU |
| 243 | G000201 | | unmod | UUACAGCCACGUCUACAGCAGUUUUAGAGCUAGAAAUAGCAAGUUAAAA UAAGGCUAGUCCGUUAUCAACUUGAAAAAGUGGCACCGAGUCGGUGCUU UU |
| 244 | G000331 | G211-7 | lower cr | mU*mU*mA*CAGCCACGUCUACAGCAmGUUUUmAGAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUmC mGmGmUmGmCmU*mU*mU*mU |
| 245 | G000332 | G211-8 | lower cr | mU*mU*mA*CAGCCACGUCUACAGCAfGfUfUfUfUfAGAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUmC mGmGmUmGmCmU*mU*mU*mU |
| 246 | G000333 | G211-9 | lower cr | mU*mU*mA*CAGCCACGUCUACAGCAmGfUfUfUmAGAmGmCmUmAm GmAmAmAmUmAmGmCAAGUUAAAAUAAGGCUAGUCCGUUAUCAmAm CmUmUmGmAmAmAmAmAmGmUmGmGmCmAmCmCmGmAmGmUm CmGmGmUmGmCmU*mU*mU*mU |
| 247 | G000334 | G211-10 | lower tr | mU*mU*mA*CAGCCACGUCUACAGCAGUUUUAGAmGmCmUmAmGmAm AmAmUmAmGmCAAGUmUmAAAmAmUAAGGCUAGUCCGUUAUCAmAm CmUmUmGmAmAmAmAmAmGmUmGmGmCmAmCmCmGmAmGmUm CmGmGmUmGmCmU*mU*mU*mU |
| 248 | G000335 | G211-11 | lower tr | mU*mU*mA*CAGCCACGUCUACAGCAGUUUUAGAmGmCmUmAmGmAm AmAmUmAmGmCAAGUmUmAfAfAmAmUAAGGCUAGUCCGUUAUCAmA mCmUmUmGmAmAmAmAmGmUmGmGmCmAmCmCmGmAmGmU mCmGmGmUmGmCmU*mU*mU*mU |
| 249 | G000336 | G211-12 | lower tr | mU*mU*mA*CAGCCACGUCUACAGCAGUUUUAGAmGmCmUmAmGmAm AmAmUmAmGmCAAGUfUmAfAmAfAmUAAGGCUAGUCCGUUAUCAmA mCmUmUmGmAmAmAmAmAmGmUmGmGmCmAmCmCmGmAmGmU mCmGmGmUmGmCmU*mU*mU*mU |
| 250 | G000337 | G211-13 | lower all | mU*mU*mA*CAGCCACGUCUACAGCAmGUUUUmAGAmGmCmUmAmG mAmAmAmUmAmGmCAAGUmUmAAAmAmUAAGGCUAGUCCGUUAUCA mAmCmUmUmGmAmAmAmAmAmGmUmGmGmCmAmCmCmGmAmG mUmCmGmGmUmGmCmU*mU*mU*mU |
| 251 | G000338 | G211-14 | lower all | mU*mU*mA*CAGCCACGUCUACAGCAmGUUUUmAGAmGmCmUmAmG mAmAmAmUmAmGmCAAGUmUmAfAfAmAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 252 | G000339 | G211-15 | lower all | mU*mU*mA*CAGCCACGUCUACAGCAmGUUUmAGAmGmCmUmAmG mAmAmAmUmAmGmCAAGUfUmAfAmAfAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 253 | G000340 | G211-16 | lower all | mU*mU*mA*CAGCCACGUCUACAGCAfGfUfUfUfUfAGAmGmCmUmAmG mAmAmAmUmAmGmCAAGUmUmAAAmAmUAAGGCUAGUCCGUUAUCA mAmCmUmUmGmAmAmAmAmAmGmUmGmGmCmAmCmCmGmAmG mUmCmGmGmUmGmCmU*mU*mU*mU |
| 254 | G000341 | G211-17 | lower all | mU*mU*mA*CAGCCACGUCUACAGCAfGfUfUfUfUfAGAmGmCmUmAmG mAmAmAmUmAmGmCAAGUmUmAfAfAmAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 255 | G000342 | G211-18 | lower all | mU*mU*mA*CAGCCACGUCUACAGCAfGfUfUfUfAGAmGmCmUmAmG mAmAmAmUmAmGmCAAGUfUmAfAmAfAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 256 | G000343 | G211-19 | Bulge cr | mU*mU*mA*CAGCCACGUCUACAGCAGUUUUAmGmAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUmC mGmGmUmGmCmU*mU*mU*mU |
| 257 | G000344 | G211-20 | Bulge tr | mU*mU*mA*CAGCCACGUCUACAGCAGUUUUAGAmGmCmUmAmGmAm AmAmUmAmGmCmAmAmGmUUAAAAUAAGGCUAGUCCGUUAUCAmAm CmUmUmGmAmAmAmAmAmGmUmGmGmCmAmCmCmGmAmGmUm CmGmGmUmGmCmU*mU*mU*mU |
| 258 | G000345 | G211-21 | nexus | mU*mU*mA*CAGCCACGUCUACAGCAGUUUUAGAmGmCmUmAmGmAm AmAmUmAmGmCAAGUUAAAAUAAGGCUAGUCCGUUfAfUfCfAmAmCmU mUmGmAmAmAmAmAmGmUmGmGmCmAmCmCmGmAmGmUmCmG mGmUmGmCmU*mU*mU*mU |
| 259 | G000346 | G211-22 | nexus | mU*mU*mA*CAGCCACGUCUACAGCAGUUUUAGAmGmCmUmAmGmAm AmAmUmAmGmCAAGUUAAAAUAAGGCUAGUCCGUUAmUmCmAmAmC mUmUmGmAmAmAmAmAmGmUmGmGmCmAmCmCmGmAmGmUmC mGmGmUmGmCmU*mU*mU*mU |
| 260 | G000347 | G211-23 | lower all | mU*mU*mA*CAGCCACGUCUACAGCAfGfUfUfUfUfAmGmAmGmCmUmA mGmAmAmAmUmAmGmCmAmAmGmUmUmAfAfAmAmUAAGGCUAGU CCGUUAmUmCmAmAmCmUmUmGmAmAmAmAmGmUmGmGmCm AmCmCmGmAmGmUmCmGmGmUmGmCmU*mU*mU*mU |
| 261 | G000348 | G211-24 | no PS | mUmUmACAGCCACGUCUACAGCAGUUUAGAmGmCmUmAmGmAmAm AmUmAmGmCAAGUUAAAAUAAGGCUAGUCCGUUAUCAmAmCmUmUm GmAmAmAmAmAmGmUmGmGmCmAmCmCmGmAmGmUmCmGmGm UmGmCmUmUmUmU |
| 262 | G000349 | G211-25 | 2 OMe PS | mU*mU*ACAGCCACGUCUACAGCAGUUUUAGAmGmCmUmAmGmAmA mAmUmAmGmCAAGUUAAAAUAAGGCUAGUCCGUUAUCAmAmCmUmU mGmAmAmAmAmAmGmUmGmGmCmAmCmCmGmAmGmUmCmGmG mUmGmCmUmU*mU*mU |
| 263 | G000350 | G211-26 | 2'F hairpin | mU*mU*mA*CAGCCACGUCUACAGCAGUUUUAGAmGmCmUmAmGmAm AmAmUmAmGmCAAGUUAAAAUAAGGCUAGUCCGUUAUCAmAmCmUm UmGmAmAmAmAmAmGmUmGmGmCmAmCmCmGmAmGfUfCfGfGfUf GfCfU*fU*fU*mU |
| 264 | G000351 | G211-27 | Alt hairpin | mU*mU*mA*CAGCCACGUCUACAGCAGUUUUAGAmGmCmUmAmGmAm AmAmUmAmGmCAAGUUAAAAUAAGGCUAGUCCGUUAUCAfAmCfUmUf GmAfAmAfAmAfGmUfGmGfCmAfCmCfGmAfGmUfCmGfGmUfGmCfU*m U*fU*mU |
| 265 | G000331-mod only | G211-7-mod only | lower cr | mGUUUUmAGAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 266 | G000332-mod only | G211-8-mod only | lower cr | fGfUfUfUfUfAGAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 267 | G000333-mod only | G211-9-mod only | lower cr | mGfUfUfUfUmAGAmGmCmUmAmGmAmAmAmUmAmGmCAAGUUAAA AUAAGGCUAGUCCGUUAUCAmAmCmUmUmGmAmAmAmAmAmGmUm GmGmCmAmCmCmGmAmGmUmCmGmGmUmGmCmU*mU*mU*mU |
| 268 | G000334-mod only | G211-10-mod only | lower tr | GUUUUAGAmGmCmUmAmGmAmAmAmUmAmGmCAAGUmUmAAAmA mUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 269 | G000335-mod only | G211-11-mod only | lower tr | GUUUUAGAmGmCmUmAmGmAmAmAmUmAmGmCAAGUmUmAfAfAm AmUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 270 | G000336-mod only | G211-12-mod only | lower tr | GUUUUAGAmGmCmUmAmGmAmAmAmUmAmGmCAAGUfUmAfAmAfA mUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 271 | G000337-mod only | G211-13-mod only | lower all | mGUUUUmAGAmGmCmUmAmGmAmAmAmUmAmGmCAAGUmUmAAA mAmUAAGGCUAGUCCGUUAUCAmAmCmUmUmGmAmAmAmAmAmGm UmGmGmCmAmCmCmGmAmGmUmCmGmGmUmGmCmU*mU*mU*m U |
| 272 | G000338-mod only | G211-14-mod only | lower all | mGUUUUmAGAmGmCmUmAmGmAmAmAmUmAmGmCAAGUmUmAfAf AmAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 273 | G000339-mod only | G211-15-mod only | lower all | mGUUUUmAGAmGmCmUmAmGmAmAmAmUmAmGmCAAGUfUmAfAm AfAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 274 | G000340-mod only | G211-16-mod only | lower all | fGfUfUfUfUfAGAmGmCmUmAmGmAmAmAmUmAmGmCAAGUmUmAA AmAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 275 | G000341-mod only | G211-17-mod only | lower all | fGfUfUfUfUfAGAmGmCmUmAmGmAmAmAmUmAmGmCAAGUmUmAfA fAmAmUAAGGCUAGUCCGUUAUCAmAmCmUmUmGmAmAmAmAmAm GmUmGmGmCmAmCmCmGmAmGmUmCmGmGmUmGmCmU*mU*mU *mU |
| 276 | G000342-mod only | G211-18-mod only | lower all | fGfUfUfUfUfAGAmGmCmUmAmGmAmAmAmUmAmGmCAAGUfUmAfA mAfAmUAAGGCUAGUCCGUUAUCAmAmCmUmUmGmAmAmAmAmAm GmUmGmGmCmAmCmCmGmAmGmUmCmGmGmUmGmCmU*mU*mU *mU |
| 277 | G000343-mod only | G211-19-mod only | Bulge cr | GUUUUAmGmAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 278 | G000344-mod only | G211-20-mod only | Bulge tr | GUUUUAGAmGmCmUmAmGmAmAmAmUmAmGmCmAmAmGmUUAAA AUAAGGCUAGUCCGUUAUCAmAmCmUmUmGmAmAmAmAmAmGmUm GmGmCmAmCmCmGmAmGmUmCmGmGmUmGmCmU*mU*mU*mU |
| 279 | G000345-mod only | G211-21-mod only | nexus | GUUUUAGAmGmCmUmAmGmAmAmAmUmAmGmCAAGUUAAAAUAAG GCUAGUCCGUUfAfUfCfAmAmCmUmUmGmAmAmAmAmAmGmUmGm GmCmAmCmCmGmAmGmUmCmGmGmUmGmCmU*mU*mU*mU |
| 280 | G000346-mod only | G211-22-mod only | nexus | GUUUUAGAmGmCmUmAmGmAmAmAmUmAmGmCAAGUUAAAAUAAG GCUAGUCCGUUAmUmCmAmAmCmUmUmGmAmAmAmAmAmGmUmG mGmCmAmCmCmGmAmGmUmCmGmGmUmGmCmU*mU*mU*mU |
| 281 | G000347-mod only | G211-23-mod only | lower all | fGfUfUfUfUfAmGmAmGmCmUmAmGmAmAmAmUmAmGmCmAmAmG mUmUmAfAfAmAmUAAGGCUAGUCCGUUAmUmCmAmAmCmUmUmG mAmAmAmAmAmGmUmGmGmCmAmCmCmGmAmGmUmCmGmGmU mGmCmU*mU*mU*mU |
| 282 | G000348-mod only | G211-24-mod only | no PS | GUUUUAGAmGmCmUmAmGmAmAmAmUmAmGmCAAGUUAAAAUAAG GCUAGUCCGUUAUCAmAmCmUmUmGmAmAmAmAmGmUmGmGm CmAmCmCmGmAmGmUmCmGmGmUmGmCmUmUmUmU |
| 283 | G000349-mod only | G211-25-mod only | 2 OMe PS | GUUUUAGAmGmCmUmAmGmAmAmAmUmAmGmCAAGUUAAAAUAAG GCUAGUCCGUUAUCAmAmCmUmUmGmAmAmAmAmAmGmUmGmGm CmAmCmCmGmAmGmUmCmGmGmUmGmCmUmU*mU*mU |
| 284 | G000350-mod only | G211-26-mod only | 2'F hairpin | GUUUUAGAmGmCmUmAmGmAmAmAmUmAmGmCAAGUUAAAAUAAG GCUAGUCCGUUAUCAmAmCmUmUmGmAmAmAmAmAmGmUmGmGm CmAmCmCmGmAmGfUfCfGfGfUfGfCfU*fU*fU*mU |
| 285 | G000351-mod only | G211-27-mod only | Alt hairpin | GUUUUAGAmGmCmUmAmGmAmAmAmUmAmGmCAAGUUAAAAUAAG GCUAGUCCGUUAUCAfAmCfUmUfGmAfAmAfAmAfGmUfGmGfCmAfCmC fGmAfGmUfCmGfGmUfGmCfU*mU*fU*mU |
| 286 | G000208 | | end mod | mC*mA*mG*GGCUCUUGAAGAUCUCCGUUUUAGAGCUAGAAAUAGCAAG UUAAAAUAAGGCUAGUCCGUUAUCAACUUGAAAAAGUGGCACCGAGUCG GUGCmU*mU*mU*U |
| 287 | G000373 | | mod6 | mC*mA*mG*GGCUCUUGAAGAUCUCCGUUUUAGAmGmCmUmAmGmA mAmAmUmAmGmCAAGUUAAAAUAAGGCUAGUCCGUUAUCAmAmCmU mUmGmAmAmAmAmAmGmUmGmGmCmAmCmCmGmAmGmUmCmG mGmUmGmCmU*mU*mU*mU |
| 288 | G000352 | G208-7 | lower cr | mC*mA*mG*GGCUCUUGAAGAUCUCCmGUUUUmAGAmGmCmUmAmG mAmAmAmUmAmGmCAAGUUAAAAUAAGGCUAGUCCGUUAUCAmAmC mUmUmGmAmAmAmAmGmUmGmGmCmAmCmCmGmAmGmUmC mGmGmUmGmCmU*mU*mU*mU |
| 289 | G000353 | G208-8 | lower cr | mC*mA*mG*GGCUCUUGAAGAUCUCCfGfUfUfUfUfAGAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUmC mGmGmUmGmCmU*mU*mU*mU |
| 290 | G000354 | G208-9 | lower cr | mC*mA*mG*GGCUCUUGAAGAUCUCCmGfUfUfUfUmAGAmGmCmUmA mGmAmAmAmUmAmGmCAAGUUAAAAUAAGGCUAGUCCGUUAUCAmA mCmUmUmGmAmAmAmAmAmGmUmGmGmCmAmCmCmGmAmGmU mCmGmGmUmGmCmU*mU*mU*mU |
| 291 | G000355 | G208-10 | lower tr | mC*mA*mG*GGCUCUUGAAGAUCUCCGUUUUAGAmGmCmUmAmGmA mAmAmUmAmGmCAAGUmUmAAAmAmUAAGGCUAGUCCGUUAUCAmA mCmUmUmGmAmAmAmAmAmGmUmGmGmCmAmCmCmGmAmGmU mCmGmGmUmGmCmU*mU*mU*mU |
| 292 | G000356 | G208-11 | lower tr | mC*mA*mG*GGCUCUUGAAGAUCUCCGUUUUAGAmGmCmUmAmGmA mAmAmUmAmGmCAAGUmUmAfAfAmAmUAAGGCUAGUCCGUUAUCAm AmCmUmUmGmAmAmAmAmAmGmUmGmGmCmAmCmCmGmAmGm UmCmGmGmUmGmCmU*mU*mU*mU |
| 293 | G000357 | G208-12 | lower tr | mC*mA*mG*GGCUCUUGAAGAUCUCCGUUUUAGAmGmCmUmAmGmA mAmAmUmAmGmCAAGUfUmAfAmAfAmUAAGGCUAGUCCGUUAUCAm AmCmUmUmGmAmAmAmAmAmGmUmGmGmCmAmCmCmGmAmGm UmCmGmGmUmGmCmU*mU*mU*mU |
| 294 | G000358 | G208-13 | lower all | mC*mA*mG*GGCUCUUGAAGAUCUCCmGUUUUmAGAmGmCmUmAmG mAmAmAmUmAmGmCAAGUmUmAAAmAmUAAGGCUAGUCCGUUAUCA mAmCmUmUmGmAmAmAmAmAmGmUmGmGmCmAmCmCmGmAmG mUmCmGmGmUmGmCmU*mU*mU*mU |
| 295 | G000359 | G208-14 | lower all | mC*mA*mG*GGCUCUUGAAGAUCUCCmGUUUUmAGAmGmCmUmAmG mAmAmAmUmAmGmCAAGUmUmAfAfAmAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 296 | G000360 | G208-15 | lower all | mC*mA*mG*GGCUCUUGAAGAUCUCCmGUUUUmAGAmGmCmUmAmG mAmAmAmUmAmGmCAAGUfUmAfAmAfAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 297 | G000361 | G208-16 | lower all | mC*mA*mG*GGCUCUUGAAGAUCUCCfGfUfUfUfAGAmGmCmUmAmG mAmAmAmUmAmGmCAAGUmUmAAAmAmUAAGGCUAGUCCGUUAUCA mAmCmUmUmGmAmAmAmAmAmGmUmGmGmCmAmCmCmGmAmG mUmCmGmGmUmGmCmU*mU*mU*mU |
| 298 | G000362 | G208-17 | lower all | mC*mA*mG*GGCUCUUGAAGAUCUCCfGfUfUfUfAGAmGmCmUmAmG mAmAmAmUmAmGmCAAGUmUmAfAfAmAmUAAGGCUAGUCCGUUAUC AmAmCmUmUmGmAmAmAmAmAmGmUmGmGmCmAmCmCmGmAm GmUmCmGmGmUmGmCmU*mU*mU*mU |
| 299 | G000363 | G208-18 | lower all | mC*mA*mG*GGCUCUUGAAGAUCUCCfGfUfUfUfAGAmGmCmUmAmG mAmAmAmUmAmGmCAAGUfUmAfAmAfAmUAAGGCUAGUCCGUUAUC AmAmCmUmUmGmAmAmAmAmGmUmGmGmCmAmCmCmGmAm GmUmCmGmGmUmGmCmU*mU*mU*mU |
| 300 | G000364 | G208-19 | Bulge cr | mC*mA*mG*GGCUCUUGAAGAUCUCCGUUUUAmGmAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUmC mGmGmUmGmCmU*mU*mU*mU |
| 301 | G000365 | G208-20 | Bulge tr | mC*mA*mG*GGCUCUUGAAGAUCUCCGUUUUAGAmGmCmUmAmGmA mAmAmUmAmGmCmAmAmGmUUAAAAUAAGGCUAGUCCGUUAUCAmA mCmUmUmGmAmAmAmAmAmGmUmGmGmCmAmCmCmGmAmGmU mCmGmGmUmGmCmU*mU*mU*mU |
| 302 | G000366 | G208-21 | nexus | mC*mA*mG*GGCUCUUGAAGAUCUCCGUUUUAGAmGmCmUmAmGmA mAmAmUmAmGmCAAGUUAAAAUAAGGCUAGUCCGUUfAfUfCfAmAmC mUmUmGmAmAmAmAmAmGmUmGmGmCmAmCmCmGmAmGmUmC mGmGmUmGmCmU*mU*mU*mU |
| 303 | G000367 | G208-22 | nexus | mC*mA*mG*GGCUCUUGAAGAUCUCCGUUUUAGAmGmCmUmAmGmA mAmAmUmAmGmCAAGUUAAAAUAAGGCUAGUCCGUUAmUmCmAmAm CmUmUmGmAmAmAmAmAmGmUmGmGmCmAmCmCmGmAmGmUm CmGmGmUmGmCmU*mU*mU*mU |
| 304 | G000368 | G208-23 | lower all | mC*mA*mG*GGCUCUUGAAGAUCUCCfGfUfUfUfAmGmAmGmCmUm AmGmAmAmAmUmAmGmCmAmAmGmUmUmAfAfAmAmUAAGGCUAG UCCGUUAmUmCmAmAmCmUmUmGmAmAmAmAmGmUmGmGmC mAmCmCmGmAmGmUmCmGmGmUmGmCmU*mU*mU*mU |
| 305 | G000369 | G208-24 | no PS | mCmAmGGGCUCUUGAAGAUCUCCGUUUUAGAmGmCmUmAmGmAmA mAmUmAmGmCAAGUUAAAAUAAGGCUAGUCCGUUAUCAmAmCmUmU mGmAmAmAmAmAmGmUmGmGmCmAmCmCmGmAmGmUmCmGmG mUmGmCmUmUmUmU |
| 306 | G000370 | G208-25 | 2 OMe PS | mC*mAG*GGCUCUUGAAGAUCUCCGUUUUAGAmGmCmUmAmGmAmA mAmUmAmGmCAAGUUAAAAUAAGGCUAGUCCGUUAUCAmAmCmUmU mGmAmAmAmAmAmGmUmGmGmCmAmCmCmGmAmGmUmCmGmG mUmGmCmUmU*mU*mU |
| 307 | G000371 | G208-26 | 2'F hairpin | mC*mA*mG*GGCUCUUGAAGAUCUCCGUUUUAGAmGmCmUmAmGmA mAmAmUmAmGmCAAGUUAAAAUAAGGCUAGUCCGUUAUCAmAmCmU mUmGmAmAmAmAmAmGmUmGmGmCmAmCmCmGmAmGfUfCfGfGfU fGfCfU*fU*fU*mU |
| 308 | G000372 | G208-27 | Alt hairpin | mC*mA*mG*GGCUCUUGAAGAUCUCCGUUUUAGAmGmCmUmAmGmA mAmAmUmAmGmCAAGUUAAAAUAAGGCUAGUCCGUUAUCAfAmCfUmU fGmAfAmAfAmAfGmUfGmGfCmAfCmCfGmAfGmUfCmGfGmUfGmCfU* mU*fU*mU |
| 309 | G000352-mod only | G208-7-mod only | lower cr | mGUUUUmAGAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 310 | G000353-mod only | G208-8-mod only | lower cr | fGfUfUfUfUfAGAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 311 | G000354-mod only | G208-9-mod only | lower cr | mGfUfUfUfUmAGAmGmCmUmAmGmAmAmAmUmAmGmCAAGUUAAA AUAAGGCUAGUCCGUUAUCAmAmCmUmUmGmAmAmAmAmAmGmUm GmGmCmAmCmCmGmAmGmUmCmGmGmUmGmCmU*mU*mU*mU |
| 312 | G000355-mod only | G208-10-mod only | lower tr | GUUUUAGAmGmCmUmAmGmAmAmAmUmAmGmCAAGUmUmAAAmA mUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 313 | G000356-mod only | G208-11-mod only | lower tr | GUUUUAGAmGmCmUmAmGmAmAmAmUmAmGmCAAGUmUmAfAfAm AmUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 314 | G000357-mod only | G208-12-mod only | lower tr | GUUUUAGAmGmCmUmAmGmAmAmAmUmAmGmCAAGUfUmAfAmAfA mUAAGGCUAGUCCGUUAUCAmAmCmUmUmGmAmAmAmAmAmGmU mGmGmCmAmCmCmGmAmGmUmCmGmGmUmGmCmU*mU*mU*mU |
| 315 | G000358-mod only | G208-13-mod only | lower all | mGUUUUmAGAmGmCmUmAmGmAmAmAmUmAmGmCAAGUmUmAAA mAmUAAGGCUAGUCCGUUAUCAmAmCmUmUmGmAmAmAmAmAmGm UmGmGmCmAmCmCmGmAmGmUmCmGmGmUmGmCmU*mU*mU*m U |
| 316 | G000359-mod only | G208-14-mod only | lower all | mGUUUUmAGAmGmCmUmAmGmAmAmAmUmAmGmCAAGUmUmAfAf AmAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 317 | G000360-mod only | G208-15-mod only | lower all | mGUUUUmAGAmGmCmUmAmGmAmAmAmUmAmGmCAAGUfUmAfAm AfAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 318 | G000361-mod only | G208-16-mod only | lower all | fGfUfUfUfUfAGAmGmCmUmAmGmAmAmAmUmAmGmCAAGUmUmAA AmAmUAAGGCUAGUCCGUUAUCAmAmCmUmUmGmAmAmAmAmAmG mUmGmGmCmAmCmCmGmAmGmUmCmGmGmUmGmCmU*mU*mU* mU |
| 319 | G000362-mod only | G208-17-mod only | lower all | fGfUfUfUfUfAGAmGmCmUmAmGmAmAmAmUmAmGmCAAGUmUmAfA fAmAmUAAGGCUAGUCCGUUAUCAmAmCmUmUmGmAmAmAmAmAm GmUmGmGmCmAmCmCmGmAmGmUmCmGmGmUmGmCmU*mU*mU *mU |
| 320 | G000363-mod only | G208-18-mod only | lower all | fGfUfUfUfUfAGAmGmCmUmAmGmAmAmAmUmAmGmCAAGUfUmAfA mAfAmUAAGGCUAGUCCGUUAUCAmAmCmUmUmGmAmAmAmAmAm GmUmGmGmCmAmCmCmGmAmGmUmCmGmGmUmGmCmU*mU*mU *mU |
| 321 | G000364-mod only | G208-19-mod only | Bulge cr | GUUUUAmGmAmGmCmUmAmGmAmAmAmUmAmGmCAAGUUAAAAU AAGGCUAGUCCGUUAUCAmAmCmUmUmGmAmAmAmAmAmGmUmG mGmCmAmCmCmGmAmGmUmCmGmGmUmGmCmU*mU*mU*mU |
| 322 | G000365-mod only | G208-20-mod only | Bulge tr | GUUUUAGAmGmCmUmAmGmAmAmAmUmAmGmCmAmAmGmUUAAA AUAAGGCUAGUCCGUUAUCAmAmCmUmUmGmAmAmAmAmAmGmUm GmGmCmAmCmCmGmAmGmUmCmGmGmUmGmCmU*mU*mU*mU |
| 323 | G000366-mod only | G208-21-mod only | nexus | GUUUUAGAmGmCmUmAmGmAmAmAmUmAmGmCAAGUUAAAAUAAG GCUAGUCCGUUfAfUfCfAmAmCmUmUmGmAmAmAmAmAmGmUmGm GmCmAmCmCmGmAmGmUmCmGmGmUmGmCmU*mU*mU*mU |
| 324 | G000367-mod only | G208-22-mod only | nexus | GUUUUAGAmGmCmUmAmGmAmAmAmUmAmGmCAAGUUAAAAUAAG GCUAGUCCGUUAmUmCmAmAmCmUmUmGmAmAmAmAmAmGmUmG mGmCmAmCmCmGmAmGmUmCmGmGmUmGmCmU*mU*mU*mU |
| 325 | G000368-mod only | G208-23-mod only | lower all | fGfUfUfUfUfAmGmAmGmCmUmAmGmAmAmAmUmAmGmCmAmAmG mUmUmAfAfAmAmUAAGGCUAGUCCGUUAmUmCmAmAmCmUmUmG mAmAmAmAmAmGmUmGmGmCmAmCmCmGmAmGmUmCmGmGmU mGmCmU*mU*mU*mU |
| 326 | G000369-mod only | G208-24-mod only | no PS | GUUUUAGAmGmCmUmAmGmAmAmAmUmAmGmCAAGUUAAAAUAAG GCUAGUCCGUUAUCAmAmCmUmUmGmAmAmAmAmGmUmGmGm CmAmCmCmGmAmGmUmCmGmGmUmGmCmUmUmUmU |
| 327 | G000370-mod only | G208-25-mod only | 2 OMe PS | GUUUUAGAmGmCmUmAmGmAmAmAmUmAmGmCAAGUUAAAAUAAG GCUAGUCCGUUAUCAmAmCmUmUmGmAmAmAmAmAmGmUmGmGm CmAmCmCmGmAmGmUmCmGmGmUmGmCmUmU*mU*mU |
| 328 | G000371-mod only | G208-26-mod only | 2'F hairpin | GUUUUAGAmGmCmUmAmGmAmAmAmUmAmGmCAAGUUAAAAUAAG GCUAGUCCGUUAUCAmAmCmUmUmGmAmAmAmAmAmGmUmGmGm CmAmCmCmGmAmGfUfCfGfGfUfGfCfU*fU*fU*mU |
| 329 | G000372-mod only | G208-27-mod only | Alt hairpin | GUUUUAGAmGmCmUmAmGmAmAmAmUmAmGmCAAGUUAAAAUAAG GCUAGUCCGUUAUCAfAmCfUmUfGmAfAmAfAmAfGmUfGmGfCmAfCmC fGmAfGmUfCmGfGmUfGmCfU*mU*fU*mU |
| 330 | G000269 | | end mod | mC*mC*mC*AUACUCCUACAGCACCAGUUUUAGAGCUAGAAAUAGCAAG UUAAAAUAAGGCUAGUCCGUUAUCAACUUGAAAAAGUGGCACCGAGUCG GUGCmU*mU*mU*U |
| 331 | G000283 | | mod6 | mC*mC*mC*AUACUCCUACAGCACCAGUUUUAGAmGmCmUmAmGmAm AmAmUmAmGmCAAGUUAAAAUAAGGCUAGUCCGUUAUCAmAmCmUm UmGmAmAmAmAmAmGmUmGmGmCmAmCmCmGmAmGmUmCmGm GmUmGmCmU*mU*mU*mU |
| 332 | G000285 | | unmod | CCCAUACUCCUACAGCACCAGUUUUAGAGCUAGAAAUAGCAAGUUAAAAU AAGGCUAGUCCGUUAUCAACUUGAAAAAGUGGCACCGAGUCGGUGCUUU U |
| 342 | G000537 | G211-33 | 5'end 3xOMePS | mU*mU*mA*CAGCCACGUCUACAGCAGUUUUAGAmGmCmUmAmGmA mAmAmUmAmGmCAAGUUAAAAUAAGGCUAGUCCGUUAUCAmAmCmU mUmGmAmAmAmAmAmGmUmGmGmCmAmCmCmGmAmGmUmCm GmGmUmGmCmUmUmUmU |
| 343 | G000538 | G211-34 | 3'end 3xOMePS | mUmUmACAGCCACGUCUACAGCAGUUUAGAmGmCmUmAmGmAmA mAmUmAmGmCAAGUUAAAAUAAGGCUAGUCCGUUAUCAmAmCmUm UmGmAmAmAmAmAmGmUmGmGmCmAmCmCmGmAmGmUmCmG mGmUmGmCmU*mU*mU*mU |
| 344 | G000539 | G211-35 | 5xOMePS | mU*mU*mA*mC*mA*GCCACGUCUACAGCAGUUUUAGAmGmCmUmA mGmAmAmAmUmAmGmCAAGUUAAAAUAAGGCUAGUCCGUUAUCAm AmCmUmUmGmAmAmAmAmAmGmUmGmGmCmAmCmCmGmAmGm UmCmGmGmUmG*mC*mU*mU*mU*mU |
| 345 | G000541 | G211-37 | 3xOMePS+2PS | mU*mU*mA*C*A*GCCACGUCUACAGCAGUUUUAGAmGmCmUmAmGm AmAmAmUmAmGmCAAGUUAAAAUAAGGCUAGUCCGUUAUCAmAmCm UmUmGmAmAmAmAmAmGmUmGmGmCmAmCmCmGmAmGmUmCm GmGmUmG*mC*mU*mU*mU*mU |
| 346 | G000542 | G211-38 | 3xOMePS+7PS | mU*mU*mA*C*A*G*C*C*A*C*GUCUACAGCAGUUUUAGAmGmCmUm AmGmAmAmAmUmAmGmCAAGUUAAAAUAAGGCUAGUCCGUUAUCA mAmCmUmUmGmAmAmAmAmAmGmUmGmGmCmAmCmCmGmAmG mU*mC*mG*mG*mU*mG*mC*mU*mU*mU*mU |
| 347 | G000543 | G211-39 | invd abasic | (invd)UUACAGCCACGUCUACAGCAGUUUUAGAmGmCmUmAmGmAmA mAmUmAmGmCAAGUUAAAAUAAGGCUAGUCCGUUAUCAmAmCmUm UmGmAmAmAmAmAmGmUmGmGmCmAmCmCmGmAmGmUmCmG mGmUmGmCmUmUmUmU(invd) |
| 348 | G000544 | G211-40 | invd abasic + 3xOMePS | (invd)mU*mU*mA*CAGCCACGUCUACAGCAGUUUAGAmGmCmUmAm GmAmAmAmUmAmGmCAAGUUAAAAUAAGGCUAGUCCGUUAUCAmA mCmUmUmGmAmAmAmAmAmGmUmGmGmCmAmCmCmGmAmGm UmCmGmGmUmGmCmU*mU*mU*mU(invd) |
| 349 | G000564 | G211-42 | 3xMOE-PS | moeU*moeU*moeA*CAGCCACGUCUACAGCAGUUUUAGAmGmCmUmA mGmAmAmAmUmAmGmCAAGUUAAAAUAAGGCUAGUCCGUUAUCAm AmCmUmUmGmAmAmAmAmAmGmUmGmGmCmAmCmCmGmAmGm UmCmGmGmUmGmCmoeU*moeU*moeU*mU |
| 350 | G000545 | G211-43 | US loop PS | mU*mU*mA*CAGCCACGUCUACAGCAGUUUUAGAmGmCmUmA*mG* mA*mA*mA*mUmAmGmCAAGUUAAAAUAAGGCUAGUCCGUUAUCAm AmCmUmUmGmAmAmAmAmAmGmUmGmGmCmAmCmCmGmAmGm UmCmGmGmUmGmCmU*mU*mU*mU |
| 351 | G000546 | G211-44 | H1 loop PS | mU*mU*mA*CAGCCACGUCUACAGCAGUUUUAGAmGmCmUmAmGmA mAmAmUmAmGmCAAGUUAAAAUAAGGCUAGUCCGUUAUCAmAmCmU mU*mG*mA*mA*mA*mAmAmGmUmGmGmCmAmCmCmGmAmGmU mCmGmGmUmGmCmU*mU*mU*mU |
| 352 | G000547 | G211-45 | H2 loop PS | mU*mU*mA*CAGCCACGUCUACAGCAGUUUUAGAmGmCmUmAmGmA mAmAmUmAmGmCAAGUUAAAAUAAGGCUAGUCCGUUAUCAmAmCmU mUmGmAmAmAmAmAmGmUmGmGmCmAmCmCmG*mA*mG*mU* mCmGmGmUmGmCmU*mU*mU*mU |
| 353 | G000548 | G211-46 | all loops PS | mU*mU*mA*CAGCCACGUCUACAGCAGUUUUAGAmGmCmUmA*mG* mA*mA*mA*mUmAmGmCAAGUUAAAAUAAGGCUAGUCCGUUAUCAm AmCmUmU*mG*mA*mA*mA*mAmAmGmUmGmGmCmAmCmCmG* mA*mG*mU*mCmGmGmUmGmCmU*mU*mU*mU |
| 354 | | | Mod6 (with modifications not shown in sequence listing) | mN*mN*mN*NNNNNNNNNNNNNNNNNGUUUUAGAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUm CmGmGmUmGmCmU*mU*mU*mU N = any nucleotide |
| 355 | | | Invariable region only | GUUUUAGAGCUAGAAAUAGCAAGUUAAAAUAAGGCUAGUCCGUUAUCA ACUUGAAAAAGUGGCACCGAGUCGGUGCUUUU |
| 356 | | | Mod6 pattern; invariable region only | GUUUUAGAmGmCmUmAmGmAmAmAmUmAmGmCAAGUUAAAAUAA GGCUAGUCCGUUAUCAmAmCmUmUmGmAmAmAmAmAmGmUmGmG mCmAmCmCmGmAmGmUmCmGmGmUmGmCmU*mU*mU*mU |
| 357 | | | Variable and invariable region | NNNNNNNNNNNNNNNNNNNNGUUUUAGAGCUAGAAAUAGCAAGUUAA AAUAAGGCUAGUCCGUUAUCAACUUGAAAAAGUGGCACCGAGUCGGUGC UUUU |
| 358 | | | Mod6 with modifications shown in sequence listing | mN*mN*mN*NNNNNNNNNNNNNNNNNGUUUUAGAmGmCmUmAmG mAmAmAmUmAmGmCAAGUUAAAAUAAGGCUAGUCCGUUAUCAmAmC mUmUmGmAmAmAmAmAmGmUmGmGmCmAmCmCmGmAmGmUm CmGmGmUmGmCmU*mU*mU*mU N = any nucleotide |

"Guide RNA" and "gRNA" are used herein interchangeably to refer collectively to either an sgRNA, a trRNA (also known as tracrRNA), or a crRNA (also known as a CRISPR RNA). The crRNA and trRNA may be associated on one RNA molecule (single guide RNA [sgRNA]) or in two separate RNA molecules (dual guide RNA [dgRNA]). "Guide RNA" or "gRNA" refers to each type.

The trRNA sequences may be naturally-occurring, or the trRNA sequence may include modifications or variations compared to naturally-occurring sequences.

"Editing efficiency" or "editing percentage" or "percent editing" as used herein is the total number of sequence reads with insertions or deletions of nucleotides into the target region of interest over the total number of sequence reads following cleavage by a Cas RNP.

"Hairpin" as used herein describes a loop of nucleic acids that is created when a nucleic acid strand folds and forms base pairs with another section of the same strand. A hairpin may form a structure that comprises a loop or a U-shape. In some embodiments, a hairpin may be comprised of a RNA loop. Hairpins can be formed with two complementary sequences in a single nucleic acid molecule bind together, with a folding or wrinkling of the molecule. In some embodiments, hairpins comprise stem or stem loop structures.

"Regions" as used herein describes conserved groups of nucleic acids. Regions may also be referred to as "modules" or "domains." Regions of a gRNA may perform particular functions, e.g., in directing endonuclease activity of the RNP, for example as described in Briner AE et al., Molecular Cell 56:333-339 (2014*).* Regions of a gRNA are described in Tables 1-3.

"Ribonucleoprotein" (RNP) or "RNP complex" as used herein describes a gRNA, for example, together with a nuclease, such as a Cas protein. In some embodiments, the RNP comprises Cas9 and gRNA.

"Stem loop" as used herein describes a secondary structure of nucleotides that form a base-paired "stem" that ends in a loop of unpaired nucleic acids. A stem may be formed when two regions of the same nucleic acid strand are at least partially complementary in sequence when read in opposite directions. "Loop" as used herein describes a region of nucleotides that do not base pair (i.e., are not complementary) that may cap a stem. A "tetraloop" describes a loop of 4 nucleotides. As used herein, the upper stem of a sgRNA may comprise a tetraloop.

In certain reference examples involving dgRNA, a "stem" region as used herein describes a secondary structure of nucleotides that forms a base-paired region between certain regions of a crRNA and trRNA (e.g., the lower and upper stem regions of each RNA). The "stem" region of a dgRNA may also be referred to in the art as a "flagpole" region.

"Treatment" as used herein covers any administration or application of a therapeutic for disease in a subject, and includes inhibiting the disease, arresting its development, relieving one or more symptoms of the disease, curing the disease, or preventing reoccurrence of one or more symptoms of the disease.

### 1. Types of Modifications

### A. 2'-O-methyl modifications

Modified sugars are believed to control the puckering of nucleotide sugar rings, a physical property that influences oligonucleotide binding affinity for complementary strands, duplex formation, and interaction with nucleases. Substitutions on sugar rings can therefore alter the confirmation and puckering of these sugars. For example, 2'-O-methyl (2'-O-Me) modifications can increase binding affinity and nuclease stability of oligonucleotides, though as shown in the Examples, the effect of any modification at a given position in an oligonucleotide needs to be empirically determined.

The terms "mA," "mC," "mU," or "mG" may be used to denote a nucleotide that has been modified with 2'-O-Me.

Modification of a ribonucleotide as 2'-O-methyl ribonucleotide can be depicted as follows:

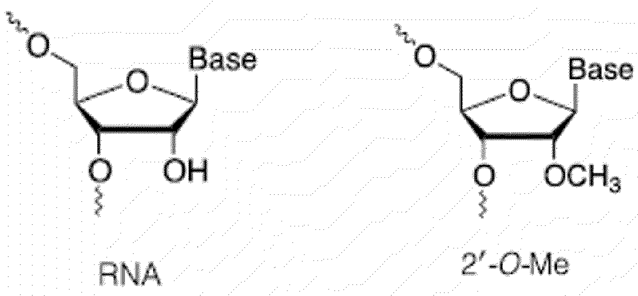

### B. 2'-O-(2-methoxyethyl) modifications

In some embodiments, the modification may be 2'-O-(2-methoxyethyl) (2'-O-moe). Modification of a ribonucleotide as a 2'-O-moe ribonucleotide can be depicted as follows:

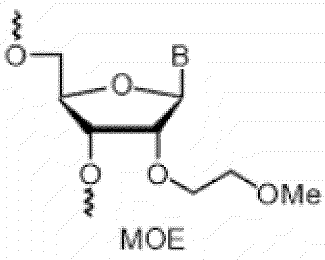

The terms "moeA," "moeC," "moeU," or "moeG" may be used to denote a nucleotide that has been modified with 2'-O-moe.

### C. 2'-fluoro modifications

Another chemical modification that has been shown to influence nucleotide sugar rings is halogen substitution. For example, 2'-fluoro (2'-F) substitution on nucleotide sugar rings can increase oligonucleotide binding affinity and nuclease stability.

In this application, the terms "fA," "fC," "fU," or "fG" may be used to denote a nucleotide that has been substituted with 2'-F.

Substitution of 2'-F can be depicted as follows:

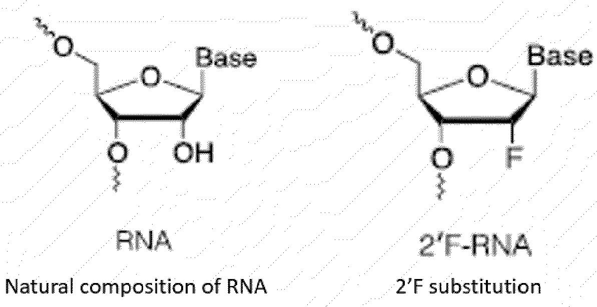

### D. Phosphorothioate modifications

Phosphorothioate (PS) linkage or bond refers to a bond where a sulfur is substituted for one nonbridging phosphate oxygen in a phosphodiester linkage, for example in the bonds between nucleotides bases. When phosphorothioates are used to generate oligonucleotides, the modified oligonucleotides may also be referred to as S-oligos.

A "*" may be used to depict a PS modification. In this application, the terms A*, C*, U*, or G* may be used to denote a nucleotide that is linked to the next (e.g., 3') nucleotide with a PS bond.

In this application, the terms "mA*," "mC*," "mU*," or "mG*" may be used to denote a nucleotide that has been substituted with 2'-O-Me and that is linked to the next (e.g., 3') nucleotide with a PS bond. Similarly, the terms "fA*," "fC*," "fU*," or "fG*" may be used to denote a nucleotide that has been substituted with 2'-F and that is linked to the next (e.g., 3') nucleotide with a PS bond. Equivalents of a PS linkage or bond are described herein.

The diagram below shows the substitution of S- into a nonbridging phosphate oxygen, generating a PS bond in lieu of a phosphodiester bond:

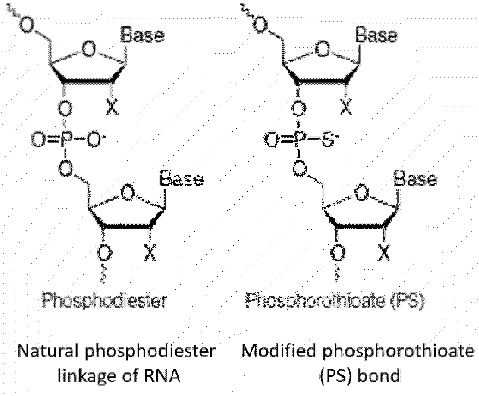

### E. G-C substitutions

In some embodiments, gRNAs are modified with sequence substitutions that do not comprise chemical modifications. In some reference examples, modified gRNAs are engineered with G-C pairings (e.g., in lower and/or upper stem regions) that are not found in the parental gRNA sequence. In some reference examples, modified gRNAs are engineered with G-U mismatches ("GU wobbles" or mismatch pairings) that are not found in the parental gRNA sequence.

### F. Inverted abasic modifications

Abasic nucleotides refer to those which lack nitrogenous bases. The figure below depicts an oligonucleotide with an abasic (also known as apurinic) site that lacks a base:

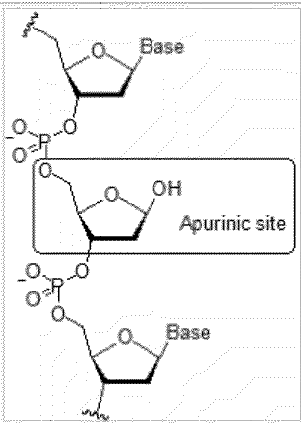

Inverted bases refer to those with linkages that are inverted from the normal 5' to 3' linkage (i.e., either a 5' to 5' linkage or a 3' to 3' linkage). For example:

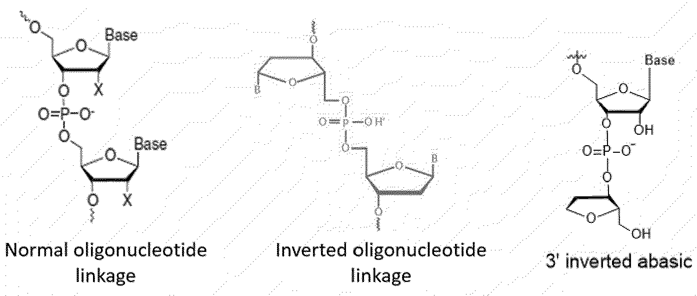

An abasic nucleotide can be attached with an inverted linkage. For example, an abasic nucleotide may be attached to the terminal 5' nucleotide via a 5' to 5' linkage, or an abasic nucleotide may be attached to the terminal 3' nucleotide via a 3' to 3' linkage. An inverted abasic nucleotide at either the terminal 5' or 3' nucleotide may also be called an inverted abasic end cap. In this application, the terms "invd" indicates an inverted abasic nucleotide linkage.

The above modifications and their equivalents are included within the scope of the reference examples described herein.

### 2. Guide RNA Compositions

Compositions comprising guide RNA are described. In some reference examples, the guide RNA comprises a trRNA. In some reference examples, the guide RNA comprises a crRNA. In some reference examples, the guide RNA comprises a crRNA and trRNA. In some reference examples, the guide RNA comprises a crRNA and trRNA on one RNA molecule as a sgRNA. In some reference examples, the guide RNA comprises a crRNA and trRNA on two RNA molecules as a dgRNA. In a dgRNA, the two RNA molecules may associate via base pairing.

In some reference examples, the guide RNA comprises a 5' terminus region. In some reference examples, the guide RNA does not comprise a 5' terminus region. In some reference examples, the 5' terminus region comprises a "spacer" region as described in Briner AE et al., Molecular Cell 56:333-339 (2014*)* for sgRNA (but applicable herein to all guide RNAs). In some reference examples, the 5' terminus region comprises a 5' end modification. A 5' terminus region with or without a spacer region may be associated with a crRNA, trRNA, sgRNA and/or dgRNA. The spacer region is also sometimes referred to herein, and by others, as a "guide region," "guide domain" or "targeting domain." A "target sequence" as used herein refers to a sequence of nucleic acid to which the guide region/domain directs a nuclease for cleavage. In some embodiments, a spyCas9 protein may be directed by a guide region/domain to a target sequence of a target nucleic acid molecule by the nucleotides present in the spacer region. In some reference examples, the guide RNA does not comprise a spacer region.

In some reference examples, the guide RNAs described herein comprise or consist of SEQ ID NOs 1-357 shown in Table 4. In an embodiment the guide RNA described herein comprises SEQ ID NO 358 shown in Table 4. Note, however, that where a sequence shows a guide/spacer region, it should be recognized that the composition may comprise this region or not. Further, guide RNAs are described that comprise the modifications of any of the sequences shown in Table 4, and identified therein by SEQ ID No. That is, the nucleotides may be the same or different, but the modification pattern shown may be the same or similar to a modification pattern of a guide sequence of Table 4. A modification pattern includes the relative position and identity of modifications of the gRNA or a region of the gRNA (e.g. 5' terminus region, lower stem region, bulge region, upper stem region, nexus region, hairpin 1 region, hairpin 2 region, 3' terminus region). In some reference examples, the modification pattern contains at least 50%, 55%, 60%, 70%, 75%, 80%, 85%, 90%, 95%, 96%, 97%, 98%, and 99% of the modifications of any one of the sequences shown in the sequence column of Table 4, or over one or more regions of the sequence. In some reference examples, the modification pattern is at least 50%, 55%, 60%, 70%, 75%, 80%, 85%, 90%, 95%, 96%, 97%, 98%, and 99% identical to the modification pattern of any one of the sequences shown in the sequence column of Table 4. In some reference examples, the modification pattern is at least 50%, 55%, 60%, 70%, 75%, 80%, 85%, 90%, 95%, 96%, 97%, 98%, and 99% identical over one or more regions of the sequence shown in Table 4, e.g., a 5' terminus region, lower stem region, bulge region, upper stem region, nexus region, hairpin 1 region, hairpin 2 region, and/or 3' terminus region. For example, in some reference examples, a guide RNA is described wherein the modification pattern is least 50%, 55%, 60%, 70%, 75%, 80%, 85%, 90%, 95%, 96%, 97%, 98%, and 99% identical to the modification pattern of a sequence over the 5' terminus region. In some reference examples, a guide RNA is described wherein the modification pattern is least 50%, 55%, 60%, 70%, 75%, 80%, 85%, 90%, 95%, 96%, 97%, 98%, and 99% identical over the lower stem. In some reference examples, a guide RNA is described wherein the modification pattern is least 50%, 55%, 60%, 70%, 75%, 80%, 85%, 90%, 95%, 96%, 97%, 98%, and 99% identical over the bulge. In some reference examples, a guide RNA is described wherein the modification pattern is least 50%, 55%, 60%, 70%, 75%, 80%, 85%, 90%, 95%, 96%, 97%, 98%, and 99% identical over the upper stem. In some reference examples, a guide RNA is described wherein the modification pattern is least 50%, 55%, 60%, 70%, 75%, 80%, 85%, 90%, 95%, 96%, 97%, 98%, and 99% identical over the nexus. In some reference examples, a guide RNA is described wherein the modification pattern is least 50%, 55%, 60%, 70%, 75%, 80%, 85%, 90%, 95%, 96%, 97%, 98%, and 99% identical over the hairpin 1. In some reference examples, a guide RNA is described wherein the modification pattern is least 50%, 55%, 60%, 70%, 75%, 80%, 85%, 90%, 95%, 96%, 97%, 98%, and 99% identical over the hairpin 2. In some reference examples, a guide RNA is described wherein the modification pattern is least 50%, 55%, 60%, 70%, 75%, 80%, 85%, 90%, 95%, 96%, 97%, 98%, and 99% identical over the 3' terminus. In some reference examples, the modification pattern differs from the modification pattern of a sequence of Table 4, or a region (e.g. 5' terminus, lower stem, bulge, upper stem, nexus, hairpin 1, hairpin 2, 3' terminus) of such a sequence, at 0, 1, 2, 3, 4, 5, or 6 nucleotides. In some reference examples, the gRNA comprises modifications that differ from the modifications of a sequence of Table 4, at 0, 1, 2, 3, 4, 5, or 6 nucleotides. In some reference examples, the gRNA comprises modifications that differ from modifications of a region (e.g. 5' terminus, lower stem, bulge, upper stem, nexus, hairpin 1, hairpin 2, 3' terminus) of a sequence of Table 4, at 0, 1, 2, 3, 4, 5, or 6 nucleotides.

In some reference examples, the gRNA comprises a 2'-O-methyl (2'-O-Me) modified nucleotide. In some reference examples, the gRNA comprises a 2'-O-(2-methoxyethyl) (2'-O-moe) modified nucleotide. In some reference examples, the gRNA comprises a 2'-fluoro (2'-F) modified nucleotide. In some reference examples, the gRNA comprises a phosphorothioate (PS) bond between nucleotides.

In some reference examples, the gRNA comprises a 5' end modification, a 3' end modification, or 5' and 3' end modifications. In some reference examples, the 5' end modification comprises a phosphorothioate (PS) bond between nucleotides. In some reference examples, the 5' end modification comprises a 2'-O-methyl (2'-O-Me), 2'-O-(2-methoxyethyl) (2'-O-moe), and/or 2'-fluoro (2'-F) modified nucleotide. In some reference examples, the 5' end modification comprises at least one phosphorothioate (PS) bond and one or more of a 2'-O-methyl (2'-O-Me) , 2'-O-(2-methoxyethyl) (2'-O-moe), and/or 2'-fluoro (2'-F) modified nucleotide. The end modification may comprise a phosphorothioate (PS), 2'-O-methyl (2'-O-Me) , 2'-O-(2-methoxyethyl) (2'-O-moe), and/or 2'-fluoro (2'-F) modification. Equivalent end modifications are also described by reference examples described herein. In some reference examples, the gRNA comprises an end modification in combination with a modification of one or more regions of the gRNA.

### A. Compositions of sgRNAs

In some reference examples, the compositions and methods of the invention comprise gRNA comprising a crRNA and trRNA that direct a nuclease such as Cas9 to a target DNA sequence. In some embodiments, the gRNAs described herein may be associated on one RNA molecule (single guide RNA or sgRNA).

Some reference examples describe a sgRNA comprising or consisting of any one of the sequences described in SEQ ID Nos: 228-332.

In some reference examples, a sgRNA comprising any one of the modified sequences of SEQ ID Nos: 235-240, 265-285, and 309-329 is described. In some reference examples, a sgRNA comprising any one of the modified sequences of SEQ ID Nos: 235-240, 265-285, and 309-329, wherein the sgRNA further comprises a 5' "spacer" sequence ("guide sequence") that is complementary to a target sequence, and directs a Cas9 to its target for cleavage is described. In some instances, the invention comprises sgRNA comprising nucleic acids having at least 99, 98, 97, 96, 95, 94, 93, 92, 91, 90, 85, 80, 75, or 70% identity to the nucleic acids of any one of SEQ ID Nos: 235-240, 265-285, and 309-329, wherein the modification pattern is identical to the modification pattern shown in the reference sequence identifier.

### 1. Domains of sgRNAs

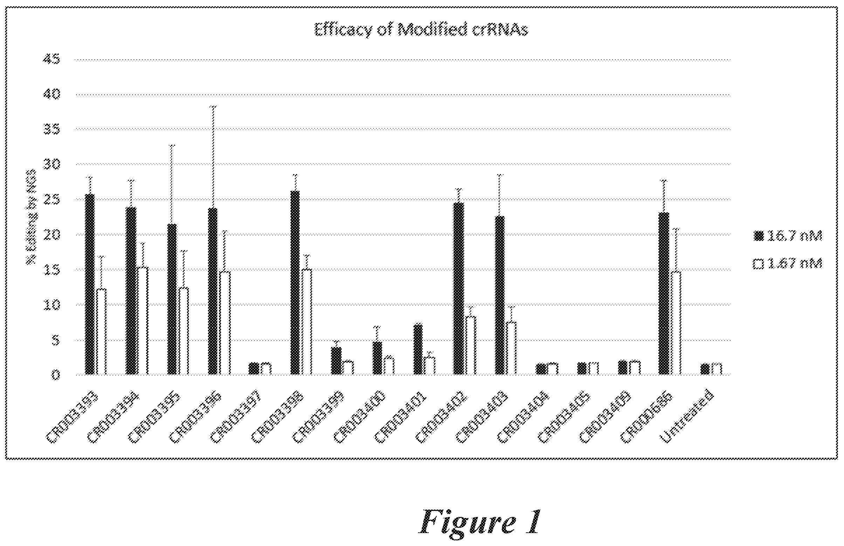

Briner AE et al., Molecular Cell 56:333-339 (2014) describes functional domains of sgRNAs, referred to herein as "domains", including the "spacer" domain responsible for targeting, the "lower stem", the "bulge", "upper stem" (which may include a tetraloop), the "nexus", and the "hairpin 1" and "hairpin 2" domains. See, Briner et al. at page 334, Figure 1A.

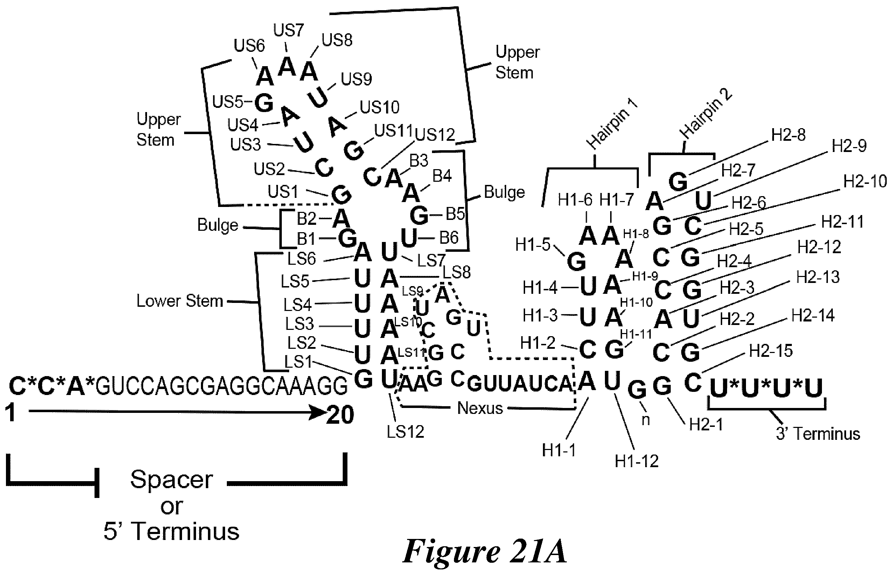

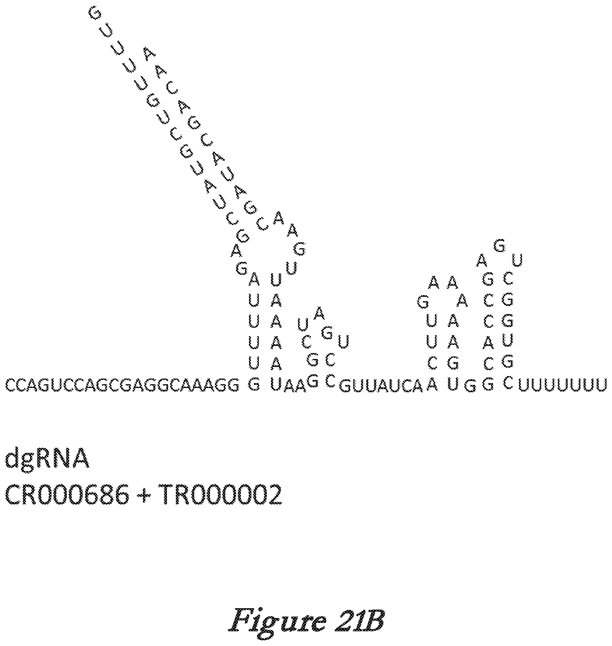

**Table 1** and FIG. 21A provide a description of the domains of a sgRNA as used herein. In Table 1, the "n" between regions represents a variable number of nucleotides, for example, from 0 to 1, 2, 3, 4, 5, 6, 7, 8, 9, 10, 11, 12, 13, 14, 15, 16, 17, 18, 19, 20, or more. In some reference examples, n equals 0. In some reference examples, n equals 1.

**Table 1: Regions of sgRNA (linear view, 5' to 3')**

| | LS1-6 | | B1 -2 | | US1-12 | | B3-6 | |
|---|---|---|---|---|---|---|---|---|
| 5' terminus (n) | lower stem | n | bulge | n | upper stem | n | bulge | n |

| LS7-12 | | N1-18 | | H1-1 thru H1-12 | | H2-1 thru H2-15 | | |
|---|---|---|---|---|---|---|---|---|
| lower stem | n | nexus | n | hairpin 1 | n | hairpin 2 | 3' terminus | |

### a) 5' terminus region

In some reference examples, the sgRNA comprises nucleotides at the 5' terminus as shown in Table 1. In some reference examples, the 5' terminus of the sgRNA comprises a spacer or guide region that functions to direct a Cas protein to a target nucleotide sequence. In some reference examples, the 5' terminus does not comprise a spacer or guide region. In some reference examples, the 5' terminus comprises a spacer and additional nucleotides that do not function to direct a Cas protein to a target nucleotide region.

In some reference examples, the guide region comprises the first 1-10, 11, 12, 13, 14, 15, 16, 17, 18, 19, or 20 nucleotides at the 5' end of the sgRNA. In some reference examples, the guide region comprises 20 nucleotides. In some reference examples, the guide region may comprise 5, 6, 7, 8, 9, 10, 11, 12, 13, 14, 15, 16, 17, 18, 19, 20, 21, 22, 23, 24, or 25 or more nucleotides. In some reference examples, the guide region may comprise 17 nucleotides. In some reference examples, the guide region may comprise 18 nucleotides. In some reference examples, the guide region may comprise 19 nucleotides.

In some reference examples, the selection of the guide region is determined based on target sequences within the gene of interest for editing. For example, in some reference examples, the sgRNA comprises a guide region that is complementary to target sequences of a gene of interest.

In some reference examples, the target sequence in the gene of interest may be complementary to the guide region of the sgRNA. In some reference examples, the degree of complementarity or identity between a guide region of a sgRNA and its corresponding target sequence in the gene of interest may be about 50%, 55%, 60%, 65%, 70%, 75%, 80%, 85%, 90%, 95%, 96%, 97%, 98%, 99%, or 100%. In some reference examples, the guide region of a sgRNA and the target region of a gene of interest may be 100% complementary or identical. In other embodiments, the guide region of a sgRNA and the target region of a gene of interest may contain at least one mismatch. For example, the guide region of a sgRNA and the target sequence of a gene of interest may contain 1, 2, 3, 4, 5, 6, 7, 8, 9, or 10 mismatches, where the total length of the target sequence is at least about 17, 18, 19, 20 or more base pairs. In some embodiments, the guide region of a sgRNA and the target region of a gene of interest may contain 1-6 mismatches where the guide sequence comprises at least about 17, 18, 19, 20 or more nucleotides. In some embodiments, the guide region of a sgRNA and the target region of a gene of interest may contain 1, 2, 3, 4, 5, or 6 mismatches where the guide sequence comprises about 20 nucleotides. The 5' terminus may comprise nucleotides that are not considered guide regions (i.e., do not function to direct a cas9 protein to a target nucleic acid).

### b) Lower stem

In some embodiments, the sgRNA comprises a lower stem (LS) region that when viewed linearly, is separated by a bulge and upper stem regions. See Table 1.

In some embodiments, the lower stem regions comprise 1-12 nucleotides, e.g. in one embodiment the lower stem regions comprise LS1-LS12. In some embodiments, the lower stem region comprises fewer nucleotides than shown in Table 1 and FIG. 21A. In some embodiments, the lower stem region comprises more nucleotides than shown in Table 1 and FIG. 21A. When the lower stem region comprises fewer or more nucleotides than shown in the schematic of Table 1 and FIG. 21A, the modification pattern, as will be apparent to the skilled artisan, should be maintained.

In some embodiments, the lower stem region has nucleotides that are complementary in nucleic acid sequence when read in opposite directions. In some embodiments, the complementarity in nucleic acid sequence of lower stem leads to a secondary structure of a stem in the sgRNA (e.g., the regions may base pair with one another). In some embodiments, the lower stem regions may not be perfectly complimentary to each other when read in opposite directions.

### c) Bulge

In some embodiments, the sgRNA comprises a bulge region comprising six nucleotides, B1-B6. When viewed linearly, the bulge region is separated into two regions. See Table 1. In some embodiments, the bulge region comprises six nucleotides, wherein the first two nucleotides are followed by an upper stem region, followed by the last four nucleotides of the bulge. In some reference examples, the bulge region comprises fewer nucleotides than shown in Table 1 and FIG. 21A. In some reference examples, the bulge region comprises more nucleotides than shown in Table 1 and FIG. 21A. When the bulge region comprises fewer or more nucleotides than shown in the schematic of Table 1 and FIG. 21A, the modification pattern, as will be apparent to the skilled artisan, should be maintained.

In some embodiments, the presence of a bulge results in a directional kink between the upper and lower stem modules in a sgRNA.

### d) Upper stem

In some embodiments, the sgRNA comprises an upper stem region comprising 12 nucleotides. In some embodiments, the upper stem region comprises a loop sequence. In some instances, the loop is a tetraloop (loop consisting of four nucleotides).

In some reference examples, the upper stem region comprises fewer nucleotides than shown in Table 1 and FIG. 21A. In some reference examples, the upper stem region comprises more nucleotides than shown in Table 1 and FIG. 21A. When the upper stem region comprises fewer or more nucleotides than shown in the schematic of Table 1 and FIG. 21A, the modification pattern, as will be apparent to the skilled artisan, should be maintained.

In some embodiments, the upper stem region has nucleotides that are complementary in nucleic acid sequence when read in opposite directions. In some embodiments, the complementarity in nucleic acid sequence of upper stem leads to a secondary structure of a stem in the sgRNA (e.g., the regions may base pair with one another). In some embodiments, the upper stem regions may not be perfectly complimentary to each other when read in opposite directions.

### e) Nexus

In some embodiments, the sgRNA comprises a nexus region that is located between the lower stem region and the hairpin 1 region. In some embodiments, the nexus comprises 18 nucleotides. In some embodiments, the nexus region comprises nucleotides N1 through N18 as shown in Table 1 and FIG. 21A.

In some reference examples, the nexus region comprises fewer nucleotides than shown in Table 1 and FIG. 21A. In some reference examples, the nexus region comprises more nucleotides than shown in Table 1 and FIG. 21A. When the nexus region comprises fewer or more nucleotides than shown in the schematic of Table 1 and FIG. 21A, the modification pattern, as will be apparent to the skilled artisan, should be maintained.

In some embodiments, the nexus region has nucleotides that are complementary in nucleic acid sequence when read in opposite directions. In some embodiments, the complementarity in nucleic acid sequence leads to a secondary structure of a stem and/or stem loop in the sgRNA (e.g., certain nucleotides in the nexus region may base pair with one another). In some embodiments, the nexus regions may not be perfectly complimentary to each other when read in opposite directions.

### f) Hairpin

In some embodiments, the sgRNA comprises one or more hairpin regions. In some embodiments, the hairpin region is downstream of (e.g., 3' to) the nexus region. In some embodiments, the region of nucleotides immediately downstream of the nexus region is termed "hairpin 1" or "H1". In some embodiments, the region of nucleotides 3' to hairpin 1 is termed "hairpin 2" or "H2". In some embodiments, the hairpin region comprises hairpin 1 and hairpin 2. In some embodiments, the sgRNA comprises only hairpin 1 or hairpin 2.

In some embodiments, the hairpin 1 region comprises 12 nucleic acids immediately downstream of the nexus region. In some embodiments, the hairpin 1 region comprises nucleotides H1-1 through H1-12 as shown in Table 1 and FIG. 21A.

In some embodiments, the hairpin 2 region comprises 15 nucleic acids downstream of the hairpin 1 region. In some embodiments, the hairpin 2 region comprises nucleotides H2-1 through H2-15 as shown in Table 1 and FIG. 21A.

In some embodiments, one or more nucleotides is present between the hairpin 1 and the hairpin 2 regions. The one or more nucleotides between the hairpin 1 and hairpin 2 region may be modified or unmodified. In some embodiments, hairpin 1 and hairpin 2 are separated by one nucleotide. In some reference examples, the hairpin regions comprise fewer nucleotides than shown in Table 1 and FIG. 21A. In some reference examples, the hairpin regions comprise more nucleotides than shown in Table 1 and FIG. 21A. When a hairpin region comprises fewer or more nucleotides than shown in the schematic of Table 1 and FIG. 21A, the modification pattern, as will be apparent to the skilled artisan, should be maintained.

In some embodiments, a hairpin region has nucleotides that are complementary in nucleic acid sequence when read in opposite directions. In some embodiments, the hairpin regions may not be perfectly complimentary to each other when read in opposite directions (e.g., the top or loop of the hairpin comprises unpaired nucleotides).

In some reference examples, the sgRNA comprises replacement of hairpin 1 with nucleotides "n", wherein "n" is an integer between 1 and 50, 40, 30, 20, 15, 10, 5, 4, 3, and 2. In some reference examples, the hairpin 1 region of a sgRNA is replaced by 2 nucleotides.

### g) 3' terminus region

In some reference examples, the sgRNA comprises nucleotides after the hairpin region(s). In some reference examples, the 3' terminus region comprises 1, 2, 3, 4, 5, 6, 7, 8, 9, 10, 15 or 20 or more nucleotides, e.g. that are not associated with the secondary structure of a hairpin. In some reference examples, the 3' terminus region comprises 1, 2, 3, or 4 nucleotides that are not associated with the secondary structure of a hairpin. In some embodiments, the 3' terminus region comprises 4 nucleotides that are not associated with the secondary structure of a hairpin. In some reference examples, the 3' terminus region comprises 1, 2, or 3 nucleotides that are not associated with the secondary structure of a hairpin.

### 2. Modifications of sgRNAs

In some reference examples, the invention comprises a sgRNA comprising one or more modifications within one or more of the following regions: the nucleotides at the 5' terminus; the lower stem region; the bulge region; the upper stem region; the nexus region; the hairpin 1 region; the hairpin 2 region; and the nucleotides at the 3' terminus.

In some reference examples, the modification comprises a 2'-O-methyl (2'-O-Me) modified nucleotide. In some reference examples, the modification comprises a 2'-O-(2-methoxyethyl) (2'-O-moe) modified nucleotide. In some reference examples, the modification comprises a 2'-fluoro (2'-F) modified nucleotide. In some reference examples, the modification comprises a phosphorothioate (PS) bond between nucleotides.

In some reference examples, the sgRNA comprises modifications at 1, 2, 3, or 4 of the first 4 nucleotides at its 5' end. In some reference examples, the first three or four nucleotides at the 5' terminus, and the last three or four nucleotides at the 3' terminus are modified. In some embodiments, the first four nucleotides at the 5' terminus, and the last four nucleotides at the 3' terminus are linked with phosphorothioate (PS) bonds. In some reference examples, the modification comprises 2'-O-Me. In some reference examples, the modification comprises 2'-F. In some reference examples, the modification comprises 2'-O-moe.

In some reference examples, the sgRNA comprises modifications at 1, 2, 3, or 4 of the first 4 nucleotides at the 5' end. In some reference examples, the sgRNA comprises modifications at 1, 2, 3, or 4 of the first 4 nucleotides at the 3' end. In some reference examples, the first four nucleotides at the 5' terminus and the last four nucleotides at the 3' terminus are linked with a PS bond, and the first three nucleotides at the 5' terminus and the last three nucleotides at the 3' terminus comprise 2'-O-Me or 2'-O-moe modifications.

In some reference examples, the first four nucleotides at the 5' terminus and the last four nucleotides at the 3' terminus are linked with a PS bond, and the first three nucleotides at the 5' terminus and the last three nucleotides at the 3' terminus comprise 2'-F modifications.

In some reference examples, a sgRNA is described wherein LS1, LS6, LS7, LS8, LS11, and LS12 are modified with 2'-O-Me. In some embodiments, each of the nucleotides in the bulge region of the sgRNA are modified with 2'-O-Me. In some embodiments, each of the nucleotides in the upper stem region of the sgRNA are modified with 2'-O-Me. In some embodiments, N16, N17, and N18 in the nexus region of the sgRNA are modified with 2'-O-Me. In some embodiments, each of the nucleotides in the hairpin 1 region of the sgRNA are modified with 2'-O-Me. In some embodiments, each of the nucleotides in the hairpin 2 region of the sgRNA are modified with 2'-O-Me.

In some reference examples, the sgRNA comprises 2'-O-Me modified nucleotides at the following nucleotides: the first three nucleotides at the 5' terminus; LS1, LS6, LS7, LS8, LS11, and LS12; B1 and B2 in the bulge region; each of the nucleotides in the upper stem region of the sgRNA; N16, N17, and N18 in the nexus region; each of the nucleotides in the hairpin 1 region; each of the nucleotides in the hairpin 2 region; and last four nucleotides at the 3' terminus.

In some embodiments, the sgRNA further comprises three phosphorothioate (PS) bonds linking the first four nucleotides at the 5' terminus and three PS bonds linking the last four nucleotides at the 3' terminus. In some reference examples, the sgRNA further comprises 2'-O-Me or 2'-F modified nucleic acids at the first three nucleotides at the 5' terminus, and 2'-O-Me or 2'-F modified nucleic acids at the last four nucleotides at the 3' terminus. In some reference examples, LS9 and LS10 are modified with 2'-F. In some reference examples, N15, N16, N17, and N18 are modified with 2'-F. In some reference examples, H2-9, H2-10, H2-11, H2-12, H2-13, HS-14, and H2-15 are modified with 2'-F. In some reference examples, the second to last, third to last, and fourth to last nucleotides at the 3' terminus are modified with 2'-F

In some reference examples, a single guide RNA (sgRNA) is described comprising 2'-F modified nucleic acids at the following nucleotides: LS9 and LS10 in the lower stem region; N15, N16, N17, and N18 in the nexus region; and H2-9, H2-10, H2-11, H2-12, H2-13, HS-14, and H2-15 in the hairpin 2 region. In some reference examples, the sgRNA further comprises 2'-F modified nucleotides at the second to last, third to last, and fourth to last nucleotides at the 3' terminus. In some embodiments, the sgRNA further comprises three phosphorothioate (PS) bonds linking the first four nucleotides at the 5' terminus and three PS bonds linking the last four nucleotides at the 3' terminus. In some reference examples, the sgRNA further comprises 2'-O-Me or 2'-F modified nucleic acids at the first three nucleotides at the 5' terminus, and 2'-O-Me or 2'-F modified nucleic acids at three of the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at LS1 and LS6; 2'-O-Me modified nucleotides at US1-US12; 2'-O-Me modified nucleotides at H1-1 - H1-12; a 2'-O-Me modified nucleotide between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-F modified nucleotides at LS1-LS6; 2'-O-Me modified nucleotides at US1-US12; 2'-O-Me modified nucleotides at H1-1 - H1-12; a 2'-O-Me modified nucleotide at "n" between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-F modified nucleotides at LS2-LS5; 2'-O-Me modified nucleotides at LS1 and LS6; 2'-O-Me modified nucleotides at US1-US12; 2'-O-Me modified nucleotides at H1-1 - H1-12; a 2'-O-Me modified nucleotide at "n" between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at US1-US12; 2'-O-Me modified nucleotides at LS7, LS8, LS11, and LS12; 2'-O-Me modified nucleotides at H1-1 - H1-12; a 2'-O-Me modified nucleotide at "n" between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at US1-US12; 2'-O-Me modified nucleotides at LS8, LS10, and LS12; 2'-O-F modified nucleotides at LS7, LS9, and LS11; 2'-O-Me modified nucleotides at H1-1 - H1-12; a 2'-O-Me modified nucleotide between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at LS1, LS6, LS7, LS8, LS11, and LS12; 2'-O-Me modified nucleotides at US1-US12; 2'-O-Me modified nucleotides at H1-1 - H1-12; a 2'-O-Me modified nucleotide between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at LS1, LS6, LS7, LS8, LS11, and LS12; 2'-F modified nucleotides at LS9 and LS10; 2'-O-Me modified nucleotides at US1-US12; 2'-O-Me modified nucleotides at H1-1 - H1-12; a 2'-O-Me modified nucleotide between Hairpin 1 and Hairpin 2; 2'-O-Me modified nucleotides at H2-1 - H2-15; and 2'-O-Me modified nucleotides at the last four nucleotides at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at US1-US12; 2'-O-Me modified nucleotides at H1-1 - H1-12; a 2'-O-Me modified nucleotide between Hairpin 1 and Hairpin 2; 2'-O-Me modified nucleotides at H2-1 - H2-8; 2'-F modified nucleotides at H2-9 - H2-15; 2'-F modified nucleotides at the second from last, third from last, and fourth from last nucleotide at the 3' terminus; and a 2'-O-Me modified nucleotide at the last nucleotide at the 3' terminus. In some reference examples, the sgRNA further comprises three phosphorothioate (PS) bonds linking the first four nucleotides at the 5' terminus and three PS bonds linking the last four nucleotides at the 3' terminus.

In some reference examples, a single guide RNA (sgRNA) is described comprising 2'-O-Me modified nucleotides at the first three nucleotides at the 5' terminus; 2'-O-Me modified nucleotides at US1-US12; 2'-O-Me modified nucleotides at H1-2, H1-4, H1-6, H1-8, H1-10, and H1-12; 2'-F modified nucleotides at H1-1, H1-3, H1-5, H1-7, H1-9, and H1-11; a 2'-F modified nucleotide between Hairpin 1 and Hairpin 2; 2'-F modified nucleotides at H2-2, H2-4, H2-6, H2-8, H2-10, H2-12; and H2-14; 2'-O-Me modified nucleotides at H2-1, H2-3, H2-5, H2-7, H2-9, H2-11; H2-13, and H2-15; 2'-F modified nucleotides at the second from last, and fourth from last nucleotide at the 3' terminus; and 2'-O-Me modified nucleotide at the third from last, and last nucleotide at the 3' terminus. In some reference examples, the sgRNA further comprises three phosphorothioate (PS) bonds linking the first four nucleotides at the 5' terminus and three PS bonds linking the last four nucleotides at the 3' terminus.

Disclosed herein, in some reference examples, is a single guide RNA (sgRNA) comprising 2'-O-Me modifications at nucleotides LS8, LS10, LS12, H1-2, H1-4, H1-6, H1-8, H1-10, H1-12, H2-1, H2-3, H2-5, H2-7, H2-9, H2-11, H2-13, and H2-15; and 2'-F modifications at LS7, LS9, LS11; H1-1, H1-3, H1-5, H1-7, H1-9, H1-11, H1-13, H2-2, H2-4, H2-6, H2-8, H2-10, H2-12, and H2-14. In some reference examples, the sgRNA further comprises three phosphorothioate (PS) bonds linking the first four nucleotides at the 5' terminus and three PS bonds linking the last four nucleotides at the 3' terminus. In some reference examples, the sgRNA further comprises 2'-O-Me modified nucleotides at the last and third to last nucleotide at the 3' terminus; and 2'-F modified nucleotides at the second to last and third to last nucleotide at the 3' terminus.

Disclosed herein, in some reference examples, is a sgRNA comprising the nucleic acids of any one of SEQ ID Nos: 228-232. Disclosed herein, in some reference examples, is a sgRNA comprising the nucleic acids of any one of SEQ ID Nos: 235-240, 265-285, and 309-329. Disclosed herein, in some reference examples, is a sgRNA comprises nucleic acids having at least 99, 98, 97, 96, 95, 94, 93, 92, 91, 90, 85, 80, 75, or 70% identity to the nucleic acids of any one of SEQ ID Nos: 235-240, 265-285, and 309-329, wherein the modification pattern is identical to the modification pattern shown in the reference sequence identifier. In some reference examples, the sgRNA further comprises three phosphorothioate (PS) bonds linking the first four nucleotides at the 5' terminus and three PS bonds linking the last four nucleotides at the 3' terminus.

In some reference examples, a sgRNA comprising a 5' end modification and one or more modifications in one or more of: the upper stem region; the hairpin 1 region; and the hairpin 2 region is described, wherein the 5' end modification comprises at least two phosphorothioate linkages within the first seven nucleotides of the 5' terminus.

In some reference examples, a sgRNA comprising a 5' end modification and one or more modifications in one or more of: the upper stem region; the hairpin 1 region; and the hairpin 2 region is described, wherein the 5' end modification comprises one or more phosphorothioate linkages at the 5' end of the RNA. In some reference examples, one or more phorphorothioate bonds link the 5' terminal nucleotides.

In some reference examples, a sgRNA comprising a 5' end modification and one or more modifications in one or more of: the upper stem region; the hairpin 1 region; and the hairpin 2 region is described, wherein the 5' end modification comprises one or more phosphorothioate linkages within the first seven nucleotides of the 5' terminus.

In some reference examples, a sgRNA comprising any one of the modified sgRNA sequences of SEQ ID Nos: 228-332 is described.

In some reference examples, a sgRNA comprising or consisting of any one of the modified sgRNA sequences of SEQ ID Nos: 235-240, 265-285, and 309-329 is described.

In some reference examples, the invention comprises a sgRNA comprising any one of the modified sequences of SEQ ID Nos: 235-240, 265-285, and 309-329, wherein the sgRNA further comprises a 5' spacer sequence that is at least partially complementary to a target sequence, and directs a Cas9 to its target for cleavage.

In some reference examples, the invention comprises a sgRNA comprising nucleotides having at least 99, 98, 97, 96, 95, 94, 93, 92, 91, 90, 85, 80, 75, or 70% identity to the nucleotides of any one of SEQ ID Nos: 235-240, 265-285, and 309-329, wherein the modification pattern is identical to the modification pattern shown in the reference sequence identifier. That is, the nucleotides A, U, C, and G may differ by 99, 98, 97, 96, 95, 94, 93, 92, 91, 90, 85, 80, 75, or 70% compared to what is shown in in the sequences, but the modification remains unchanged.

In some reference examples, the invention comprises a sgRNA comprising one or more modifications within one or more of the following regions: the nucleotides at the 5' terminus; the lower stem region; the bulge region; the upper stem region; the; the nexus region; the hairpin 1 region; the hairpin 2 region; and the nucleotides at the 3' terminus.

In some embodiments, the modification comprises a 2'-O-methyl (2'-O-Me) modified nucleotide. In some reference examples, the modification comprises a 2'-fluoro (2'-F) modified nucleotide. In some embodiments, the modification comprises a phosphorothioate (PS) bond between nucleotides. In some reference examples, the modification comprises an inverted abasic nucleotide.

In some reference examples, a sgRNA is described comprising 2'-O-Me modified nucleotides at: the first three nucleotides in the 5' terminus; LS1, LS6, LS7, LS8, LS11, and LS12 in the lower stem; B1 and B2 in the bulge region; each of the nucleotides in the upper stem region; N16, N17, and N18 in the nexus region; each of the nucleotides in the hairpin 1 region; one nucleotide between hairpin 1 and hairpin 2; each of the nucleotides in the hairpin 2 region; and the last four nucleotides at the 3' terminus. In one reference example, the sgRNA further comprises three PS bonds between the first four nucleotides at the 5' terminus and three PS bonds between the last four nucleotides at the 3' terminus.

In some reference examples, a sgRNA is described comprising 2'-O-Me modified nucleotides at: the first three nucleotides in the 5' terminus; LS1, LS6, LS7, LS8, LS11, and LS12 in the lower stem; B1-B6 in the bulge region; each of the nucleotides in the upper stem region; N16, N17, and N18 in the nexus region; each of the nucleotides in the hairpin 1 region; one nucleotide between hairpin 1 and hairpin 2; each of the nucleotides in the hairpin 2 region; and the last four nucleotides at the 3' terminus. In one reference example, the sgRNA further comprises three PS bonds between the first four nucleotides at the 5' terminus and three PS bonds between the last four nucleotides at the 3' terminus.

In some reference examples, a sgRNA is described comprising 2'-F modified nucleotides at: LS9 and LS10 in the lower stem; 15-N18 in the nexus region; H2-9-HS-15 in the hairpin 2 region; and the second to last, third to last, and fourth to last nucleotide in the 3' terminus region.

In some reference examples, a sgRNA is described comprising 2'-F modified nucleotides at: each nucleotide in the lower stem; 15-N18 in the nexus region; H2-9-HS-15 in the hairpin 2 region; and the second to last, third to last, and fourth to last nucleotide in the 3' terminus region.

In some reference examples, a single guide RNA (sgRNA) is described comprising 2'-O-Me modified nucleotides at LS8, LS10, LS12, H1-2, H1-4, H1-6, H1-8, H1-10, H1-12, H2-1, H2-3, H2-5, H2-7, H2-9, H2-11, H2-13, H2-15, and the last and third to last nucleotides at the 3' terminus; and 2'-F modifications at LS7, LS9, LS11; H1-1, H1-3, H1-5, H1-7, H1-9, H1-11, H1-13, H2-2, H2-4, H2-6, H2-8, H2-10, H2-12, H2-14, and the second to last and fourth to last nucleotide at the 3' terminus.

### B. Compositions of dgRNAs

In some reference examples, the compositions and methods of the invention comprise gRNA comprising a crRNA and trRNA that direct a nuclease such as Cas9 to a target DNA sequence. In some reference examples, the gRNAs are associated, but on two separate RNA molecules (dual guide RNA or dgRNA).

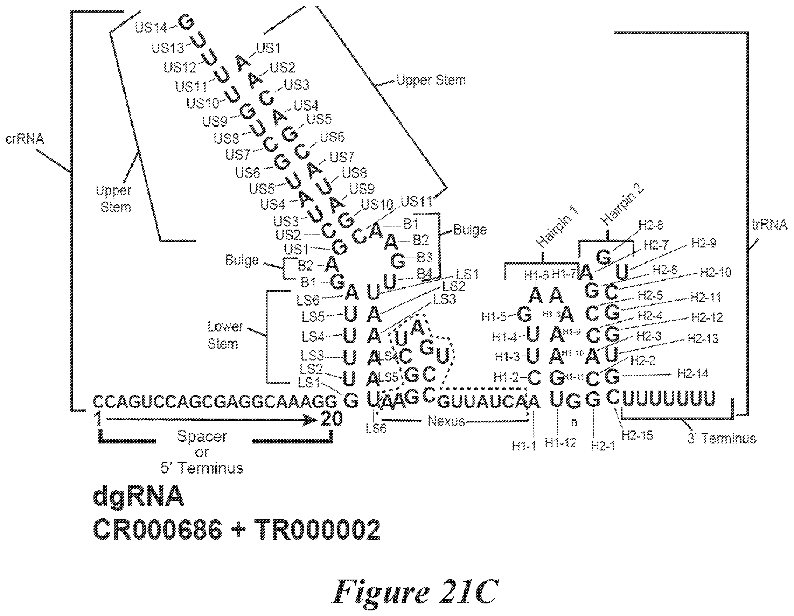

**Table 2** and FIG. 21C provides a description of domains of a crRNA as used herein. The 5' terminus region may comprise a spacer region at or near the 5' terminus of the crRNA and functions to direct a Cas9 to a target region in the DNA, e.g., as described herein. In Table 2, the "n" between regions represents a variable number of nucleotides, for example, from 0 to 1, 2, 3, 4, 5, 6, 7, 8, 9, 10, 11, 12, 13, 14, 15, 16, 17, 18, 19, 20, or more. In some reference examples, n equals 0. Any of the dgRNAs described herein may include an "n" between any domain.

**Table 3** and FIG. 21C provide a description of domains of a trRNA as used herein. In Table 3, the "n" between regions represents a variable number of nucleotides, for example, from 0 to 1, 2, 3, 4, 5, 6, 7, 8, 9, 10, 11, 12, 13, 14, 15, 16, 17, 18, 19, 20, or more. In some reference examples, n equals 0. Any of the dgRNAs described herein may include an "n" between any domain.

### 1. Domains of dgRNAs

As described in *Briner 2014,* dgRNAs can be developed based on specific functional domains, referred to herein as "domains", including the spacer responsible for targeting, the lower stem, the bulge, the upper stem, the nexus, and the hairpin domains. In dgRNAs, the crRNA comprises some components of the gRNA and the trRNA comprises some components of the gRNA.

Regions of crRNAs are described in **Table 2** and **FIG 21C****.** Regions of trRNAs are described in **Table 3** and **FIG 21C.** FIG 21C shows a schematic of an exemplary dgRNA.

**Table 2: Regions of crRNA (linear view, 5' to 3')**

| | LS1-6 | | B1-2 | | US1-14 | |
|---|---|---|---|---|---|---|
| 5' terminus (n) | lower stem | n | bulge | n | upper stem | 3' terminus |

**Table 3: Regions of trRNA (linear view, 5' to 3')**

| | US1-11 | | B1-4 | | LS1-6 | | N1-18 | | H1-1 thru H1-12 | | H2-1 thru H2-15 | |
|---|---|---|---|---|---|---|---|---|---|---|---|---|
| 5' terminus (n) | upper stem | n | bulge | n | lower stem | n | nexus | n | hairpin 1 | n | hairpin 2 | 3' terminus |

### a) 5' Terminus Region

In some reference examples, the dgRNA comprises nucleotides at the 5' terminus of the crRNA and trRNA as shown in Tables 2-3 and FIG 21C.

In some reference examples, the 5' terminus of the crRNA comprises a spacer or guide region that functions to direct a Cas protein to a target nucleotide sequence. In some reference examples, the 5' terminus does not comprise a spacer or guide region. In some reference examples, the 5' terminus comprises a spacer and additional nucleotides that do not function to direct a Cas protein to a target nucleotide region.

In some reference examples, the guide region comprises the first 1-10, 11, 12, 13, 14, 15, 16, 17, 18, 19, or 20 nucleotides at the 5' end of the crRNA. In some embodiments, the guide region comprises 20 nucleotides. In some reference examples, the guide region may comprise 5, 6, 7, 8, 9, 10, 11, 12, 13, 14, 15, 16, 17, 18, 19, 20, 21, 22, 23, 24, or 25 or more nucleotides. In some reference examples, the guide region may comprise 17 nucleotides. In some reference examples, the guide region may comprise 18 nucleotides. In some reference examples, the guide region may comprise 19 nucleotides.

In some embodiments, the selection of the guide region is determined based on target sequences within the gene of interest for editing. For example, in some reference examples, the crRNA comprises a guide region that is complementary to target sequences of a gene of interest.

In some reference examples, the target sequence in the gene of interest may be complementary to the guide region of the crRNA. In some reference examples, the degree of complementarity or identity between a guide region of a crRNA and its corresponding target sequence in the gene of interest may be about 50%, 55%, 60%, 65%, 70%, 75%, 80%, 85%, 90%, 95%, 96%, 97%, 98%, 99%, or 100%. In some reference examples, the guide region of a crRNA and the target region of a gene of interest may be 100% complementary or identical. In other reference examples, the guide region of a crRNA and the target region of a gene of interest may contain at least one mismatch. For example, the guide region of a crRNA and the target sequence of a gene of interest may contain 1, 2, 3, 4, 5, 6, 7, 8, 9, or 10 mismatches, where the total length of the target sequence is at least about 17, 18, 19, 20 or more base pairs. In some reference examples, the guide region of a crRNA and the target region of a gene of interest may contain 1-6 mismatches where the guide sequence comprises at least about 17, 18, 19, 20 or more nucleotides. In some reference examples, the guide region of a crRNA and the target region of a gene of interest may contain 1, 2, 3, 4, 5, or 6 mismatches where the guide sequence comprises about 20 nucleotides.

In some reference examples, the trRNA comprises a 5' terminus. In some reference examples, the trRNA comprises a 5' terminus which forms, in part, the upper stem of a dgRNA. The 5' terminus of the trRNA is not complementary to a region of the target gene.

### b) Lower stem

In some reference examples, the dgRNA comprises a lower stem (LS) region. The lower stem region comprises a crRNA lower stem region and a trRNA lower stem region that associate as depicted in FIG 21C. In some reference examples, the lower stem region of the crRNA is at least partially complementary to the lower stem region of the trRNA. In some reference examples, the lower stem region of the crRNA is fully complementary to the lower stem region of the trRNA.

In some reference examples, the lower stem region of the crRNA and trRNA each comprise 6 nucleotides. In some reference examples, the lower stem region of the crRNA and trRNA each comprise fewer nucleotides than shown in Tables 2 and 3 and FIG. 21C. In some reference examples, the lower stem region comprises more nucleotides than shown in Tables 2 and 3 and FIG. 21C. When the lower stem region comprises fewer or more nucleotides than shown in the schematic of Tables 2 and 3 and FIG. 21C, the modification patterns, as will be apparent to the skilled artisan, are maintained. In some reference examples, the number of nucleotides in the lower stem of the crRNA differs from the number of nucleotides in the lower stem of the trRNA.

### c) Bulge

In some reference examples, the dgRNA comprises a bulge (B) region. In some reference examples, the crRNA comprises one bulge region and the trRNA comprises one bulge region. In some reference examples, each bulge region comprises 1-4 nucleotides. In some reference examples, the bulge region of the crRNA comprises two nucleotides, and the bulge region of the trRNA comprises four nucleotides.

In some reference examples, the crRNA bulge region is located between the lower stem region and the upper stem region of the crRNA. In some reference examples, the bulge region of the crRNA comprises two nucleotides. In some reference examples, the bulge region of the crRNA comprises nucleotides B1 and B2 as shown Table 2 and FIG 21C.

In some reference examples, the trRNA bulge region is located between the upper stem region and the lower stem region of the trRNA. In some reference examples, the bulge region of the trRNA comprises four nucleotides. In some reference examples, the bulge region of the trRNA comprises nucleotides B1 through B4 as shown Table 3 and FIG 21C.

In some reference examples, the presence of a bulge results in a directional kink between the upper and lower stems modules in a dgRNA. The crRNA bulge and trRNA bulge may be partially complementary. The crRNA bulge and trRNA bulge may have no complementary.

In some reference examples, the bulge regions of the crRNA and trRNA comprise more nucleotides than shown in Tables 2 and 3 and FIG. 21C. When the bulge region comprises fewer or more nucleotides than shown in the schematic of Tables 2 and 3 and FIG. 21C, the modification patterns, as will be apparent to the skilled artisan, are maintained. In some reference examples, the number of nucleotides in the bulge of the crRNA differs from the number of nucleotides in the bulge of the trRNA.

### d) Upper stem

In some reference examples, the dgRNA comprises an upper stem (US) region. The upper stem region comprises a crRNA upper stem region and a trRNA upper stem region that associate as depicted in FIG 21C. In some reference examples, the upper stem region of the crRNA is at least partially complementary to the upper stem region of the trRNA. In some reference examples, the upper stem region of the crRNA is fully complementary to the upper stem region of the trRNA.

In some reference examples, the upper stem region of the crRNA comprises fourteen nucleotides. In some reference examples, the upper stem region of the trRNA comprises eleven nucleotides. In some reference examples, the upper stem regions of the crRNA and trRNA each comprise fewer nucleotides than shown in Tables 2 and 3 and FIG. 21C. In some reference examples, the upper stem regions of the crRNA and trRNA comprise more nucleotides than shown in Tables 2 and 3 and FIG. 21C. When the upper stem region comprises fewer or more nucleotides than shown in the schematic of Tables 2 and 3 and FIG. 21C, the modification patterns, as will be apparent to the skilled artisan, are maintained.

In some reference examples, the upper stem of the crRNA comprises nucleotides US1 through US14 as shown in Table 2 and FIG. 21C.

In some reference examples, the upper stem of the trRNA comprises nucleotides US1 through US 11 as shown in Table 3 and FIG. 21C.

### e) Nexus

In some reference examples, the dgRNA comprises a trRNA comprising a nexus region. In some reference examples, the nexus is between the lower stem region and the hairpin 1 region of the trRNA. In some reference examples, the nexus is located immediately downstream of the lower stem of the trRNA. In some reference examples, the nexus comprises eighteen nucleotides. In some reference examples, the nexus region of the trRNA comprises nucleotides N1-N18 as shown in Table 3 and FIG 21C. In some reference examples, the nexus comprises fewer nucleotides than shown in Table 3 and FIG 21C. In some reference examples, the nexus region of the trRNA comprises more nucleotides than shown in Table 3 and FIG. 21C. When the nexus region comprises fewer or more nucleotides than shown in Table 3 and FIG. 21C, the modification patterns, as will be apparent to the skilled artisan, are maintained.

In some reference examples, the nexus region has nucleotides that are complementary in nucleic acid sequence when read in opposite directions. In some reference examples, the complementarity in nucleic acid sequence leads to a secondary structure of a stem and/or stem loop in the sgRNA (e.g., certain nucleotides in the nexus region may base pair with one another). In some reference examples, the nexus regions may not be perfectly complimentary to each other when read in opposite directions.

### f) Hairpin

In some reference examples, the hairpin region of the trRNA is downstream of the nexus region. In some reference examples, the region of nucleotides immediately downstream of the nexus region is termed "hairpin 1." In some reference examples, the region of nucleotides immediately downstream of the hairpin 1 region is termed "hairpin 2." In some reference examples, the hairpin region comprises hairpin 1 and hairpin 2. In some instances, hairpin 1 and hairpin 2 are separated by one or more nucleotide "n." In some reference examples, n=1. In some reference examples, the trRNA comprises only hairpin 1 or hairpin 2.

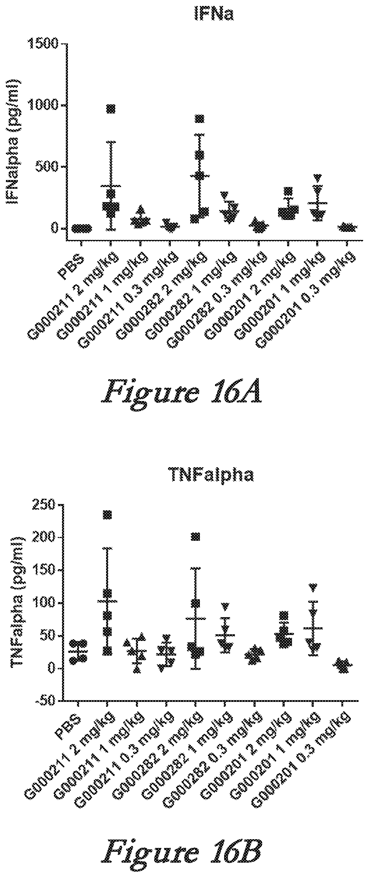

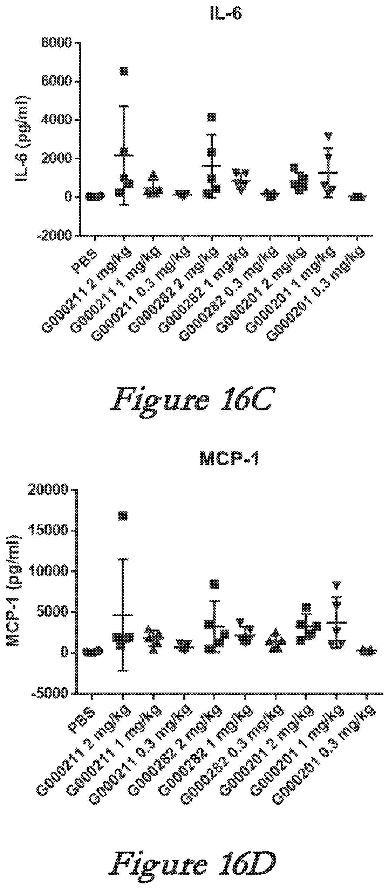

Replacement of the hairpin 1 region of a trRNA with 2 nucleotides has been shown to allow editing activity of a Cas RNP (see US20150376586, Fig. 16). In some reference examples, the trRNA comprises replacement of hairpin 1 with nucleotides "n", wherein "n" is an integer between 1 and 50, 40, 30, 20, 15, 10, 5, 4, 3, and 2. In some reference examples, the hairpin 1 region of a trRNA is replaced by 2 nucleotides.

In some reference examples, hairpin 1 of the trRNA comprises twelve nucleotides immediately downstream of the nexus region. In some reference examples, the hairpin 1 region of the trRNA comprises nucleotides H1-1 through H1-12 as shown in Table 3 and FIG 21C.

In some reference examples, non-hairpin nucleotides are present between the hairpin 1 and the hairpin 2 regions of the trRNA. In some reference examples, one to two non-hairpin nucleotides reside between hairpin 1 and hairpin 2.

In some reference examples, hairpin 2 of the trRNA comprises fifteen nucleotides after (3' to) hairpin 1. In some reference examples, the hairpin 2 region of the trRNA comprises nucleotides H2-1 through H2-15 as shown in Table 3 and FIG 21C. In some reference examples, the hairpin 2 region of the trRNA comprises nucleotides H2-1 through H2-15 as shown in Table 3, and the "n" between hairpin 1 and hairpin 2 is 1 or 2.

In some reference examples, a hairpin region of the trRNA comprises more nucleotides than shown in Table 3 and FIG. 21C. When a hairpin region comprises fewer or more nucleotides than shown in Table 3 and FIG. 21C, the modification patterns, as will be apparent to the skilled artisan, are maintained.

In some reference examples, a hairpin region has nucleotides that are complementary in nucleic acid sequence when read in opposite directions. In some reference examples, the hairpin regions may not be perfectly complimentary to each other when read in opposite directions (e.g., the top or loop of the hairpin comprises unpaired nucleotides).

In some reference examples, the trRNA comprises replacement of hairpin 1 with nucleotides "n", wherein "n" is an integer between 1 and 50, 40, 30, 20, 15, 10, 5, 4, 3, and 2. In some reference examples, the hairpin 1 region of a trRNA is replaced by 2 nucleotides.

### g) 3' terminus

In some reference examples, the dgRNA comprises a trRNA comprising a 3' terminus region comprising additional nucleotides after (3' to) the hairpin region(s). In some reference examples, the 3' terminus region comprises 1, 2, 3, 4, 5, 6, 7, 8, 9, 10, 15 or 20 or more nucleotides that are not associated with the secondary structure of a hairpin. In some reference examples, the 3' terminus region comprises 1, 2, 3, or 4 nucleotides that are not associated with the secondary structure of a hairpin. In some reference examples, the 3' terminus region comprises 4 nucleotides that are not associated with the secondary structure of a hairpin. In some reference examples, the 3' terminus region comprises 1, 2, or 3 nucleotides that are not associated with the secondary structure of a hairpin.

### 2. Modifications of dgRNAs

In some reference examples, a dgRNA comprises a modified crRNA and an unmodified trRNA. In some reference examples, a dgRNA comprises an unmodified crRNA and a modified trRNA. In some reference examples, both the crRNA and trRNA of a dgRNA comprise modifications.

In some reference examples, the gRNAs described herein are in two separate RNA molecules (dual guide or dgRNA). See, Tables 2, 3, and FIG. 21C.

In some reference examples, the invention comprises a dgRNA comprising or consisting of a) any one of the crRNA sequences of SEQ ID Nos: 1-187; and b) any one of the trRNA sequences described in SEQ ID Nos: 188-227.

In some reference examples, a dgRNA comprising any one of the modified crRNA sequences of 1-187 is described.

In some reference examples, a dgRNA comprising any one of the modified trRNA sequences of 188-227 is described.

In some reference examples, a dgRNA comprising any one of the modified crRNA sequences of SEQ ID Nos: 19-31, 53-73, and 104-130 is described. In some reference examples, the invention comprises a dgRNA comprising any one of the modified sequences of SEQ ID Nos: 19-31, 53-73, and 104-130, wherein the crRNA further comprises a 5' spacer sequence that is at least partially complementary to a target sequence, and directs a Cas9 to its target for cleavage.

In some reference examples, the invention comprises a crRNA comprising any one of the sequences described in SEQ ID Nos: 1-187. In some reference examples, the invention comprises a crRNA comprising or consisting of any one of the sequences described in SEQ ID Nos: 19-31, 53-73, and 104-130. In some reference examples, the invention comprises a crRNA comprising any one of the sequences described in SEQ ID Nos: 19-31, 53-73, and 104-130 and a spacer region.

In some reference examples, the invention comprises a trRNA comprising or consisting of any one of the sequences described in SEQ ID Nos: 188-277.

In some reference examples, the invention comprises a crRNA comprising nucleotides having at least 99, 98, 97, 96, 95, 94, 93, 92, 91, 90, 85, 80, 75, or 70% identity to the nucleotides of any one of SEQ ID Nos: 1-187, wherein the modification pattern is identical to the modification pattern shown in the reference sequence identifier. That is, the nucleotides A, U, C, and G may differ by 99, 98, 97, 96, 95, 94, 93, 92, 91, 90, 85, 80, 75, or 70% compared to what is shown in in the sequences, but the modification remains unchanged.

In some reference examples, the invention comprises a trRNA comprising nucleotides having at least 99, 98, 97, 96, 95, 94, 93, 92, 91, 90, 85, 80, 75, or 70% identity to the nucleotides of any one of SEQ ID Nos: 188-277, wherein the modification pattern is identical to the modification pattern shown in the reference sequence identifier. That is, the nucleotides A, U, C, and G may differ by 99, 98, 97, 96, 95, 94, 93, 92, 91, 90, 85, 80, 75, or 70% compared to what is shown in the sequences, but the modification on each nucleotide remains unchanged.

### 3. crRNAs, trRNAs, and dgRNAs with modifications

In some reference examples, the crRNA comprises one or more modified nucleotides within one or more of the 5' terminus, lower stem, bulge, upper stem, and 3' terminus.

In some reference examples, the modification comprises 2'-O-Me.

In some reference examples, the modification comprises 2'-F.

In some reference examples, the modification comprises a phosphorothioate (PS) bond linking one or more nucleotides. In some reference examples, the modification is three PS bonds linking the first four nucleotides at the 5' terminus and three PS bonds linking the last four nucleotides at the 3' terminus.

In some reference examples, the modification comprises an inverted abasic nucleotide.

In some reference examples, a crRNA is described comprising 2'-O-Me modified nucleotides at each nucleotide in the upper stem. In some reference examples, US-1 through US-14 of the crRNA are each modified with 2'-O-Me. In some reference examples, LS1 and LS6 of the crRNA are modified with 2'-O-Me. In some reference examples, LS5 of the crRNA is modified with 2'-O-Me.

In some reference examples, a crRNA comprising 2'-O-Me modified nucleotides at each of the nucleotides in the upper stem, and LS1 and LS6 in the lower stem is described. In some reference examples, the crRNA further comprises one or more 2'-O-Me or 2'-O-moe modified nucleotides in the 5' and/or 3' terminus region, e.g. in a 5' and/or 3' end modification.

In some reference examples, a crRNA comprising 2'-O-Me modified nucleotides at each of the nucleotides in the upper stem, LS1, LS5, and LS6 in the lower stem is described. In some reference examples, the crRNA further comprises one or more 2'-O-Me or 2'-O-moe modified nucleotides in the 5' and/or 3' terminus region, e.g. in a 5' and/or 3' end modification.

In some reference examples, the invention comprises a crRNA comprising 2'-F modified nucleotides at LS1, LS2, and LS6 in the lower stem. In some reference examples, the crRNA further comprises 2'-F modified nucleotides at each of B1 and B2 in the bulge region. In some reference examples, the invention comprises a crRNA comprising 2'-F modified nucleotides at LS1, LS2, and LS6 in the lower stem, and at each of B1 and B2 in the bulge region. In some reference examples, the crRNA further comprises one or more 2'-O-Me or 2'-O-moe modified nucleotides in the 5' and/or 3' terminus region, e.g. in a 5' and/or 3' end modification.

In some reference examples, the crRNA comprises 2'-O-Me modified nucleotides at nucleotides LS1 and LS6 in the lower stem region; each of the nucleic acids in the bulge region; and each of the nucleic acids in the upper stem region. In some reference examples, the LS5 nucleotide of the crRNA is also modified with 2'-O-Me. In some reference examples, LS2, LS3, and LS4 of the crRNA are not modified. In some reference examples, the crRNA further comprises one or more 2'-O-Me or 2'-O-moe modified nucleotides in the 5' and/or 3' terminus region, e.g. in a 5' and/or 3' end modification.

In some reference examples, the crRNA comprises 2'-fluoro (2'-F) modified nucleotides at LS1, LS2, and LS6 in the lower stem region, and each of the nucleotides in the bulge region. In some reference examples, the crRNA comprises 2'-fluoro (2'-F) modified nucleotides at LS1, LS2, and LS6 in the lower stem region, and at B2 and B2 in the bulge region. In some reference examples, the crRNA comprises 2'-fluoro (2'-F) modified nucleotides at LS1- LS6 in the lower stem region, and each of the nucleotides in the bulge region. In some reference examples, the crRNA further comprises one or more 2'-O-Me or 2'-O-moe modified nucleotides in the 5' and/or 3' terminus region, e.g. in a 5' and/or 3' end modification.

In some reference examples, the invention comprises a trRNA comprising one or more modified nucleotides within one or more of the following regions: the 5' terminus, the upper stem region; the bulge region; the lower stem region; the nexus region; the hairpin 1 region; the intervening region between the hairpin 1 and hairpin 2 regions; the hairpin 2 region; and the 3' terminus region.

In some embodiments, the modification comprises 2'-O-Me.

In some reference examples, the modification comprises 2'-F.

In some reference examples, the modification comprises a phosphorothioate (PS) bond linking one or more nucleotides. In some reference examples, the modification is three PS bonds linking the first four nucleotides at the 5' terminus and three PS bonds linking the last four nucleotides at the 3' terminus.

In some reference examples, the modification comprises an inverted abasic nucleotide.

In some reference examples, the trRNA comprises 2'-O-Me modified nucleotides at each nucleic acid in the upper stem; B1 and B2 in the bulge region; LS1 and LS2 in the lower stem region; N3, N4, N5, N15, N16, N17, and N18 in the nexus region; each nucleotide in the hairpin 1 region; one nucleotide between the hairpin 1 and hairpin 2 region; and each nucleotide in the hairpin 2 region. In some reference examples, the trRNA further comprises one or more 2'-O-Me or 2'-O-moe modified nucleotides in the 5' and/or 3' terminus region, e.g. in a 5' and/or 3' end modification.

In some reference examples, the trRNA comprises 2'-O-Me modified nucleotides at each nucleic acid in the upper stem; each nucleotide in the bulge region; LS1, LS2, LS5, and LS6 in the lower stem region; N3- N5, N10-N18 in the nexus region; each nucleotide in the hairpin 1 region; one nucleotide between the hairpin 1 and hairpin 2 region; and each nucleotide in the hairpin 2 region. In some reference examples, the crRNA further comprises one or more 2'-O-Me or 2'-O-moe modified nucleotides in the 5' and/or 3' terminus region, , e.g. in a 5' and/or 3' end modification.

In some reference examples, the trRNA comprises 2'-F modified nucleotides at N15 through N18 in the nexus region. In some reference examples, the trRNA further comprises one or more 2'-F modified nucleotides in the 5' and/or 3' terminus region, e.g. in a 5' and/or 3' end modification.

In some reference examples, the trRNA comprises 2'-F modified nucleotides at LS4 and LS5 in the lower stem region, and N13-N18 in the nexus region. In some reference examples, the trRNA further comprises one or more 2'-F modified nucleotides in the 5' and/or 3' terminus region, e.g. in a 5' and/or 3' end modification.

In some reference examples, the trRNA comprises 2'-F modified nucleotides at LS1, LS3, and LS5 in the lower stem, and 2'-O-Me modified nucleotides at LS2, LS4, and LS6 in the lower stem.

Disclosed herein, in some reference examples, is a crispr RNA (crRNA) comprising one or more modifications within one or more of the following regions: the first five nucleotides at the 5' terminus; the lower stem region; the bulge region; the upper stem region; and the last five nucleotides at the 3' terminus. In some reference examples, the modification comprises a 2'-O-methyl (2'-O-Me) modified nucleotide. In some reference examples, the modification comprises a 2'-fluoro (2'-F) modified nucleotide. In some reference examples, the modification comprises a phosphorothioate (PS) bond between nucleotides. In some reference examples, the first three nucleotides at the 5' terminus, and the last three nucleotides at the 3' terminus are modified. In some embodiments, the first four nucleotides at the 5' terminus, and the last four nucleotides at the 3' terminus are linked with phosphorothioate (PS) bonds. In some embodiments, the modification comprises 2'-O-Me. In some reference examples, the modification comprises 2'-F. In some embodiments, the first four nucleotides at the 5' terminus and the last four nucleotides at the 3' terminus are linked with a PS bond, and wherein the first three nucleotides at the 5' terminus and the last three nucleotides at the 3' terminus comprise 2'-O-Me modifications. In some reference examples, the first four nucleotides at the 5' terminus and the last four nucleotides at the 3' terminus are linked with a PS bond, and wherein the first three nucleotides at the 5' terminus and the last three nucleotides at the 3' terminus comprise 2'-F modifications. In some reference examples, LS1 and LS6 are modified with 2'-O-Me. In some embodiments, each of the nucleotides in the upper stem region are modified with 2'-O-Me.

In some reference examples, the invention comprises a crispr RNA (crRNA) comprising 2'-O-Me modified nucleic acids at the following nucleotides: LS1 and LS6 in the lower stem region; and each nucleotide in the upper stem region. In some reference examples, the crRNA further comprises three phosphorothioate (PS) bonds linking the first four nucleotides at the 5' terminus and three PS bonds linking the last four nucleotides at the 3' terminus. In some reference examples, the crRNA further comprises 2'-O-Me or 2'-F modified nucleic acids at the first three nucleotides at the 5' terminus, and 2'-O-Me or 2'-F modified nucleic acids at the last three nucleotides at the 3' terminus. In some reference examples, LS1, LS2, and LS6 are modified with 2'-F. In some reference examples, each nucleotide in the bulge region is modified with 2'-F.

Disclosed herein, in some reference examples, is a crispr RNA (crRNA) comprising 2'-F modified nucleic acids at the following nucleotides: LS1, LS2, and LS6 in the lower stem region; and each nucleotide in the bulge region. In some reference examples, the crRNA further comprises three phosphorothioate (PS) bonds linking the first four nucleotides at the 5' terminus and three PS bonds linking the last four nucleotides at the 3' terminus. In some reference examples, the crRNA further comprises 2'-O-Me or 2'-F modified nucleic acids at the first three nucleotides at the 5' terminus, and 2'-O-Me or 2'-F modified nucleic acids at the last three nucleotides at the 3' terminus.

In some reference examples, a crRNA comprising the nucleic acids of any one of SEQ ID Nos: 1 - 187 is described. In some reference examples, a crRNA comprising the nucleic acids of any one of SEQ ID Nos: 19-31, 53-73, 104-130, and 161-187 is described. In some reference examples, a crRNA comprising nucleic acids having at least 99, 98, 97, 96, 95, 94, 93, 92, 91, 90, 85, 80, 75, or 70% identity to the nucleic acids of any one of SEQ ID Nos: 19-31, 53-73, 104-130, and 161-187, wherein the modification pattern is identical to the modification pattern shown in the reference sequence identifier, is described. In some reference examples, the crRNA further comprises three phosphorothioate (PS) bonds linking the first four nucleotides at the 5' terminus and three PS bonds linking the last four nucleotides at the 3' terminus.

Also described is a tracr RNA (trRNA) comprising one or more modifications within one or more of the following regions: the first five nucleotides at the 5' terminus; the upper stem region; the bulge region; the lower stem region; the nexus region; the hairpin 1 region; the hairpin 2 region; and the last five nucleotides at the 3' terminus. In some reference examples, the modification comprises a 2'-O-methyl (2'-O-Me) modified nucleotide. In some reference examples, the modification comprises a 2'-fluoro (2'-F) modified nucleotide. In some reference examples, the modification comprises a phosphorothioate (PS) bond between nucleotides. In some reference examples, the first four nucleotides at the 5' terminus, and the last four nucleotides at the 3' terminus are linked with phosphorothioate (PS) bonds. In some reference examples, the first three nucleotides at the 5' terminus, and the last three nucleotides at the 3' terminus are modified. In some reference examples, the modification comprises 2'-O-Me. In some reference examples, the modification comprises 2'-F. In some reference examples, the first four nucleotides at the 5' terminus and the last four nucleotides at the 3' terminus are linked with a PS bond, and wherein the first three nucleotides at the 5' terminus and the last three nucleotides at the 3' terminus comprise 2'-O-Me modifications. In some reference examples, the first four nucleotides at the 5' terminus and the last four nucleotides at the 3' terminus are linked with a PS bond, and wherein the first three nucleotides at the 5' terminus and the last three nucleotides at the 3' terminus comprise 2'-F modifications. In some embodiments, each nucleotide in the upper stem region is modified with 2'-O-Me. In some reference examples, B1 and B2 within the bulge region are modified with 2'-O-Me. In some reference examples, N3, N4, N5, N15, N16, N17, and N18 in the nexus region are modified with 2'-O-Me. In some embodiments, each nucleotide in the hairpin 1 region is modified with 2'-O-Me. In some embodiments, each nucleotide in the hairpin 2 region is modified with 2'-O-Me.

In some reference examples, the invention comprises a tracr RNA (trRNA) comprising 2'-O-Me modified nucleic acids at the following nucleotides: each nucleotide in the upper stem; B1 and B2 within the bulge region; N3, N4, N5, N15, N16, N17, and N18 in the nexus region; each nucleotide in the hairpin 1 region; and each nucleotide in the hairpin 2 region. In some reference examples, the trRNA further comprises three phosphorothioate (PS) bonds linking the first four nucleotides at the 5' terminus and three PS bonds linking the last four nucleotides at the 3' terminus. In some reference examples, the trRNA further comprises 2'-O-Me or 2'-F modified nucleotides at the first three nucleotides at the 5' terminus, and 2'-O-Me or 2'-F modified nucleic acids at the last three nucleotides at the 3' terminus. In some reference examples, N15, N16, N17, and N18 are modified with 2'-F. In some reference examples, LS1, LS3, and LS5 are modified with 2'-F, and LS2, LS4, and LS6 are modified with 2'-O-Me. In some reference examples, the trRNA further comprises three phosphorothioate (PS) bonds linking the first four nucleotides at the 5' terminus and three PS bonds linking the last four nucleotides at the 3' terminus. In some reference examples, the trRNA further comprises 2'-O-Me or 2'-F modified nucleic acids at the first three nucleotides at the 5' terminus, and 2'-O-Me or 2'-F modified nucleic acids at the last three nucleotides at the 3' terminus.

In some reference examples, a trRNA comprising the nucleic acids of any one of SEQ ID Nos: 188-227 is described. In some reference examples, a trRNA comprising nucleic acids having at least 99, 98, 97, 96, 95, 94, 93, 92, 91, 90, 85, 80, 75, or 70% identity to the nucleic acids of any one of SEQ ID Nos: 188-227, wherein the modification pattern is identical to the modification pattern shown in the reference sequence identifier, is described. In some reference examples, the trRNA further comprises three phosphorothioate (PS) bonds linking the first four nucleotides at the 5' terminus and three PS bonds linking the last four nucleotides at the 3' terminus.

In some instances, a dual guide comprising a crRNA and a trRNA is described, wherein the crRNA comprises the nucleic acids of any one of SEQ ID Nos: 1-187, and wherein the trRNA comprises the nucleic acids of any one of SEQ ID Nos: 188-227.

A dual guide comprising a crRNA disclosed herein and a trRNA disclosed herein is described, as is a dual guide comprising a crRNA disclosed herein and an unmodified trRNA. In some reference examples, a dual guide comprising an unmodified crRNA and a modified trRNA disclosed herein is described.

### C. Modifications to terminal nucleotides

In some reference examples, the 5' or 3' terminal nucleotides of any of the guide RNAs described herein are modified. In some reference examples, the terminal (i.e., last) 1, 2, 3, 4, 5, 6, or 7 nucleotides in 3' terminus region of guide RNA, including, for example, the sgRNA, the dgRNA, the crRNA, trRNA, or both crRNA and trRNA are modified. In some reference examples, the terminal (i.e., last) 1, 2, 3, 4, 5, 6, or 7 nucleotides in 3' terminus region of guide RNA comprise more than one modification. In some reference examples, at least one of the terminal (i.e., last) 1, 2, 3, 4, 5, 6, or 7 nucleotides at the 3' terminus region are modified. In some reference examples, at least two of the terminal (i.e., last) 1, 2, 3, 4, 5, 6, or 7 nucleotides in 3' terminus region are modified. In some reference examples, at least three of the terminal (i.e., last) 1, 2, 3, 4, 5, 6, or 7 nucleotides in 3' terminus region are modified. some embodiments, the modification comprises a PS linkage.

In some reference examples, the 5' end of the 5' terminus region is modified, for example, the first 1, 2, 3, 4, 5, 6, or 7 nucleotides of the sgRNA, the dgRNA, crRNA, trRNA, or both crRNA and trRNA are modified. In some reference examples, the first 1, 2, 3, 4, 5, 6, or 7 nucleotides in 3' terminus region of guide RNA comprise more than one modification. In some reference examples, at least one of the terminal (i.e., first) 1, 2, 3, 4, 5, 6, or 7 nucleotides at the 5' end are modified. In some reference examples, at least two of the terminal 1, 2, 3, 4, 5, 6, or 7 nucleotides at the 5' end are modified. In some reference examples, at least three of the terminal 1, 2, 3, 4, 5, 6, or 7 nucleotides at the 5' end are modified. some reference examples, the modification comprises a PS linkage.

n some reference examples, both the 5' and 3' termini (e.g., ends) of the guide RNA, e.g., sgRNA, dgRNA, crRNA, trRNA, or both crRNA and trRNA are modified. In some reference examples, only the 5' terminus of the guide RNA, e.g., sgRNA, dgRNA, crRNA, trRNA, or both crRNA and trRNA is modified. In some reference examples, only the 3' terminus of the guide RNA, e.g., sgRNA, dgRNA, crRNA, trRNA, or both crRNA and trRNA is modified.

In some reference examples, the gRNA comprises modifications at 1, 2, 3, 4, 5, 6, or 7 of the first 7 nucleotides at a 5' end of the gRNA. In some reference examples, the gRNA comprises modifications at 1, 2, 3, 4, 5, 6, or 7 of the 7 terminal nucleotides at a 3' end. In some reference examples, 2, 3, or 4 of the first 4 nucleotides at the 5' end, and/or 2, 3, or 4 of the terminal 4 nucleotides at the 3' end are modified. In some reference examples, 2, 3, or 4 of the first 4 nucleotides at the 5' end are linked with phosphorothioate (PS) bonds.

In some reference examples, the modification to the 5' terminus and/or 3' terminus comprises a 2'-O-methyl (2'-O-Me) or 2'-O-(2-methoxyethyl) (2'-O-moe) modification to a nucleotide. In some reference examples, the modification comprises a 2'-fluoro (2'-F) modification to a nucleotide. In some embodiments, the modification comprises a phosphorothioate (PS) linkage between nucleotides. In some reference examples, the modification comprises an inverted abasic nucleotide. In some reference examples, the modification comprises a more than one modification selected from 2'-O-Me, 2'-O-moe, 2'-fluoro (2'-F), a phosphorothioate (PS) linkage between nucleotides, and an inverted abasic nucleotide. In some reference examples, an equivalent modification is described.

In some reference examples, the guide RNA, e.g., sgRNA, dgRNA, crRNA, trRNA, or both crRNA and trRNA comprises one or more phosphorothioate (PS) linkages between the first one, two, three, four, five, six, or seven nucleotides at the 5' terminus. In some reference examples, the guide RNA, e.g., sgRNA, dgRNA, crRNA, trRNA, or both crRNA and trRNA comprises one or more PS linkages between the last one, two, three, four, five, six, or seven nucleotides at the 3' terminus. In some reference examples, the guide RNA, e.g., sgRNA, dgRNA, crRNA, trRNA, or both crRNA and trRNA comprises one or more PS linkages between the last one, two, three, four, five, six, or seven nucleotides at both the 5' terminus and the 3' terminus. In some reference examples, in addition to PS linkages, the 5' and 3' terminal nucleotides may comprise 2'-O-Me, 2'-O-moe, or 2'-F modified nucleotides.

In some reference examples, the guide RNA, e.g., sgRNA, dgRNA, crRNA, trRNA, or both crRNA and trRNA comprises modified nucleotides at the 5' and 3' terminus, and modified nucleotides in one or more other regions described in Tables 1-3 and FIG 21A or 21C.

In some reference examples, the crRNA, trRNA, or both crRNA and trRNA comprises modified nucleotides that are not at the 5' or 3' ends. Specific patterns of modifications are described below and in Table 4.

### 3. Delivery of gRNAs and Cas Protein

In some embodiments, in addition to the at least one gRNA, the compositions provided herein further comprise a nuclease. In some embodiments, the nuclease is a Cas protein. In some embodiments, the gRNA together with a Cas protein is called a Cas RNP. In some embodiments, the Cas protein is from the Type-II CRISPR/Cas system. In some embodiments, the Cas protein is Cas9. In some embodiments, the Cas9 protein is a wild type Cas9. In some embodiments, the Cas9 protein is derived from the *Streptococcus pyogenes* Cas9 protein, e.g., a S. *pyogenes* Cas9. In some embodiments, the Cas9 protein is not derived from S. *pyogenes,* but functions in the same way as S. *pyogenes* Cas9 such that gRNA that is specific to S. *pyogenes* Cas9 will direct the non-S. *pyogenes* Cas9 to its target site. In some embodiments, the Cas induces a double strand break in target DNA. Equivalents of S. *pyogenes* Cas9 protein are encompassed by the embodiments described herein.

Cas9 encompasses modified and variants thereof. Modified versions of Cas9 having one catalytic domain, either RuvC or HNH, that is inactive are termed "nickases." Nickases cut only one strand on the target DNA, thus creating a single-strand break. A single-strand break may also be known as a "nick." In some embodiments, the compositions and methods comprise nickases. In some embodiments, the compositions and methods comprise a nickase Cas9 that induces a nick rather than a double strand break in the target DNA.

In some embodiments, the Cas protein may be modified to contain only one functional nuclease domain. For example, the Cas protein may be modified such that one of the nuclease domains is mutated or fully or partially deleted to reduce its nucleic acid cleavage activity. In some embodiments, a nickase Cas is used having a RuvC domain with reduced activity. In some embodiments, a nickase Cas is used having an inactive RuvC domain. In some embodiments, a nickase Cas is used having an HNH domain with reduced activity. In some embodiments, a nickase Cas is used having an inactive HNH domain.

In some embodiments, a conserved amino acid within a Cas protein nuclease domain is substituted to reduce or alter nuclease activity. In some embodiments, a Cas protein may comprise an amino acid substitution in the RuvC or RuvC-like nuclease domain. Exemplary amino acid substitutions in the RuvC or RuvC-like nuclease domain include D10A (based on the S. *pyogenes* Cas9 protein). In some embodiments, the Cas protein may comprise an amino acid substitution in the HNH or HNH-like nuclease domain. Exemplary amino acid substitutions in the HNH or HNH-like nuclease domain include E762A, H840A, N863A, H983A, and D986A (based on the S. *pyogenes* Cas9 protein).

In some embodiments, the RNP complex described herein comprises a nickase and a pair of guide RNAs that are complementary to the sense and antisense strands of the target sequence, respectively. In this embodiment, the guide RNAs direct the nickase to a target sequence and introduce a double stranded break (DSB) by generating a nick on opposite strands of the target sequence (i.e., double nicking). In some embodiments, use of double nicking may improve specificity and reduce off-target effects. In some embodiments, a nickase Cas is used together with two separate guide RNAs targeting opposite strands of DNA to produce a double nick in the target DNA. In some embodiments, a nickase Cas is used together with two separate guide RNAs that are selected to be in close proximity to produce a double nick in the target DNA.

In some embodiments, chimeric Cas proteins are used, where one domain or region of the protein is replaced by a portion of a different protein. In some embodiments, a Cas nuclease domain may be replaced with a domain from a different nuclease such as Fok1. In some embodiments, a Cas protein may be a modified nuclease.

In some embodiments, the Cas protein comprises a fusion protein comprising a catalytically inactive Cas9 linked to a heterologous functional domain (see, e.g., WO2014152432). In some embodiments, the catalytically inactive Cas9 is from *S. pyogenes.* In some embodiments, the catalytically inactive Cas9 comprises mutations that inactivate the Cas9. In some embodiments, the heterologous functional domain is a domain that modifies gene expression, histones, or DNA. In some embodiments, the heterologous functional domain is a transcriptional activation domain or a transcriptional repressor domain.

### A. PAM

In some embodiments, the target sequence may be adjacent to the PAM. In some embodiments, the PAM may be adjacent to or within 1, 2, 3, or 4, nucleotides of the 3' end of the target sequence. The length and the sequence of the PAM may depend on the Cas protein used. For example, the PAM may be selected from a consensus or a particular PAM sequence for a specific Cas9 protein or Cas9 ortholog, including those disclosed in Figure 1 of Ran et al., Nature 520:186-191 (2015). In some embodiments, the PAM may comprise 2, 3, 4, 5, 6, 7, 8, 9, or 10 nucleotides in length. Non-limiting exemplary PAM sequences include NGG, NAG, NGA, NGAG, NGCG, NNGRRT, TTN, NGGNG, NG, NAAAAN, NNAAAAW, NNNNACA, GNNNCNNA, and NNNNGATT (wherein N is defined as any nucleotide, and W is defined as either A or T, and R is defined as either A or G). In some embodiments, the PAM sequence may be NGG. In some embodiments, the PAM sequence may be NGGNG. In some embodiments, the PAM sequence may be NNAAAAW.

### B. Delivery of Modified gRNA

Lipid nanoparticles (LNPs) are a well-known means for delivery of nucleotide and protein cargo, and may be used for delivery of the gRNA, mRNA, Cas9, and RNPs disclosed herein. In some embodiments, the LNPs deliver nucleic acid, protein, or nucleic acid together with protein.

In some embodiments, the invention comprises a method for delivering any one of the gRNAs disclosed herein to a subject, wherein the gRNA is associated with an LNP. In some embodiments, the gRNA/LNP is also associated with a Cas9 or an mRNA encoding Cas9.

In some embodiments, the invention comprises a composition comprising any one of the gRNAs disclosed and an LNP. In some embodiments, the composition further comprises a Cas9 or an mRNA encoding Cas9.

In some embodiments, the LNPs comprise cationic lipids. In some embodiments, the LNPs comprise (9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate). In some embodiments, the LNPs comprise molar ratios of a cationic lipid amine to RNA phosphate (N:P) of about 4.5.

In some embodiments, LNPs associated with the gRNAs disclosed herein are for use in preparing a medicament for treating a disease or disorder.

Electroporation is a well-known means for delivery of cargo, and any electroporation methodology may be used for delivery of any one of the gRNAs disclosed herein. In some embodiments, electroporation may be used to deliver any one of the gRNAs disclosed herein and Cas9 or an mRNA encoding Cas9.

In some embodiments, the invention comprises a method for delivering any one of the gRNAs disclosed herein to an ex vivo cell, wherein the gRNA is associated with an LNP or not associated with an LNP. In some embodiments, the gRNA/LNP or gRNA is also associated with a Cas9 or an mRNA encoding Cas9.

### 4. Methods of Gene Modulation

In some embodiments, the invention comprises a pharmaceutical formulation comprising any one of the gRNAs of the invention disclosed herein together with a pharmaceutically acceptable carrier. In some embodiments, the invention comprises a pharmaceutical formulation comprising any one of the gRNAs of the invention disclosed herein and an LNP together with a pharmaceutically acceptable carrier. In some embodiments, the invention comprises a pharmaceutical formulation comprising any one of the gRNAs of the invention disclosed herein, a Cas9 protein or an mRNA encoding a Cas9 protein, and a LNP together with a pharmaceutically acceptable carrier. In some embodiments, the pharmaceutical formulation is for use in preparing a medicament for treating a disease or disorder. Disclosed herein is a method of treating a human patient comprising administering any one of the gRNAs or pharmaceutical formulations described herein.

Disclosed herein is a method or use of modifying a target DNA comprising, administering or delivering a Cas protein or Cas mRNA and any one or more of the gRNAs disclosed herein.

Disclosed herein is a method or use for modulation of a target gene comprising, administering or delivering a Cas protein or Cas mRNA and any one or more of the gRNAs disclosed herein. In some instances, the modulation is editing of the target gene. In some instances, the modulation is a change in expression of the protein encoded by the target gene.

In some instances, the method or use results in gene editing. In some instances, the method or use results in a double-stranded break within the target gene. In some instances, the method or use results in formation of indel mutations during non-homologous end joining of the DSB. In some instances, the method or use results in an insertion or deletion of nucleotides in a target gene. In some instances, the insertion or deletion of nucleotides in a target gene leads to a frameshift mutation or premature stop codon that results in a non-functional protein. In some instances, the insertion or deletion of nucleotides in a target gene leads to a knockdown or elimination of target gene expression. In some instances, the method or use comprises homology directed repair of a DSB. In some instances, the method or use further comprises delivering to the cell a template, wherein at least a part of the template incorporates into a target DNA at or near a double strand break site induced by the Cas protein.

In some instances, the method or use results in gene modulation. In some instances, the gene modulation is an increase or decrease in gene expression, a change in methylation state of DNA, or modification of a histone subunit. In some instances, the method or use results in increased or decreased expression of the protein encoded by the target gene.

The sgRNA of the invention may be useful in preparing a medicament for treating a disease or disorder. As reference examples the other types of gRNA disclosed herein may be useful in preparing a medicament for treating a disease or disorder.

### A. Measures of Gene Modulation

The efficacy of modified gRNAs can be tested in vitro and in vivo. The invention comprises one or more of the sgRNAs disclosed herein, wherein the sgRNA results in gene modulation when provided to a cell together with Cas9. The efficacy of an sgRNA can be measured in *in vitro* or *in vivo* assays.

### 1. In vitro measurement of Cas efficacy

In some examples, the activity of a Cas RNP comprising a modified sgRNA is compared to the activity of a Cas RNP comprising an unmodified sgRNA.

In some reference examples, the activity of a Cas RNP comprising a dgRNA comprising a modified trRNA is compared to the activity of a Cas RNP comprising a dgRNA comprising an unmodified trRNA.

In some reference examples, the activity of a Cas RNP comprising a dgRNA comprising a modified crRNA is compared to the activity of a Cas RNP comprising a dgRNA comprising an unmodified crRNA.

In some reference examples, the activity of a Cas RNP comprising a dgRNA comprising a modified crRNA and a modified trRNA is compared to the activity of a Cas RNP comprising an unmodified crRNA and an unmodified trRNA.

In some examples, the efficiency of an sgRNA in increasing or decreasing target protein expression is determined by measuring the amount of target protein. In some embodiments, the invention comprises any one of the sgRNAs claimed herein, wherein the sgRNA increases or decreases the amount of protein produced from the targeted gene. Also disclosed herein is a method of modulating protein expression comprising administering any one of the sgRNAs claimed herein to a subject, wherein the gRNA directs Cas9 to the gene encoding the target protein, and the target protein expression is increased or decreased as compared to a gRNA control that does not target Cas9 to that gene.

In some embodiments, the efficiency of editing with a claimed sgRNA is determined by the editing present at the target location in the genome following delivery of Cas9 and the sgRNA. In reference examples, the efficiency of editing with a dgRNA comprising a crRNA and trRNA is determined by the editing present at the target location in the genome following delivery of Cas9 and the sgRNA). In some examples, the efficiency of editing with specific gRNAs is measured by next-generation sequencing. In some examples, the editing percentage of the target region of interest is determined. In some examples, the total number of sequence reads with insertions or deletions of nucleotides into the target region of interest over the total number of sequence reads is measured following delivery of a sgRNA and Cas9. Disclosed herein is a method of increasing the efficiency of gene editing comprising, administering or delivering any one of the modified gRNAs described herein to a cell, wherein the percentage of gene editing is increased as compared to a control gRNA that is not similarly modified.

In some examples disclosed herein, the efficiency of editing with specific gRNAs is measured by the presence of insertions or deletions of nucleotides introduced by successful gene editing. Disclosed herein is a method of creating insertions or deletions of nucleotides in genes comprising, administering or delivering any one of the modified gRNAs described herein to a cell, wherein the nucleotides are inserted or deleted as compared to a control gRNA that is not similarly modified. In some examples, activity of a Cas9 and gRNAs is tested in biochemical assays. In some examples, activity of a Cas9 and gRNAs is tested in a cell-free cleavage assay. In some examples, activity of a Cas9 and gRNAs is tested in Neuro2A cells.

In some embodiments, Cas 9 and sgRNA shows similar, greater, or reduced activity compared to the unmodified sgRNA. In reference examples, Cas9 and dgRNA comprising modified crRNA and/or trRNA shows similar, greater, or reduced activity compared to the unmodified dgRNA comprising unmodified crRNA and trRNA. In some embodiments, Cas9 and modified sgRNA shows enhanced activity compared to the unmodified sgRNA. In reference examples, Cas9 and modified dgRNA comprising modified crRNA and trRNA shows enhanced activity compared to the unmodified crRNA and trRNA.

### 2. In vivo measurement of Cas efficacy

In some examples, the activity of modified gRNAs is measured after in vivo dosing of LNPs comprising modified gRNAs and Cas protein or mRNA encoding Cas protein.

In some examples, in vivo efficacy of a gRNA or composition provided herein is determined by editing efficacy measured in DNA extracted from tissue (e.g., liver tissue) after administration of gRNA and Cas9.

### 3. In vivo measurement of immune system activation

Modifications to gRNA as disclosed herein may reduce the subject's immune response to in vivo dosing of gRNAs. In some embodiments, activation of the subject's immune response is measured by serum concentrations of cytokine(s) following in vivo dosing of sgRNA together with Cas9 mRNA or protein (e.g., formulated in a LNP). In reference examples, activation of the subject's immune response is measured by serum concentrations of cytokine(s) following in vivo dosing of dgRNA comprising trRNA and crRNA together with Cas9 mRNA or protein (e.g., formulated in a LNP). In some embodiments, the cytokine is interferon-alpha (IFN-alpha), interleukin 6 (IL-6), monocyte chemotactic protein 1 (MCP-1), and/or tumor necrosis factor alpha (TNF-alpha). Disclosed herein is a method of reducing a subject's immune response to delivery of an sgRNA comprising, administering any one of the sgRNAs of the invention, wherein the sgRNA produces a reduced response by the subject's immune system following administration. In some embodiments, the invention comprises reducing activation of the subject's immune system following administration as compared to a control sgRNA that is not similarly modified.

In some embodiments, administration of Cas RNP or Cas9 mRNA together with the modified sgRNA produces lower serum concentration(s) of immune cytokines compared to administration of unmodified sgRNA. In some embodiments, the invention comprises reducing a subject's serum concentration of immune cytokines comprising, administering any one of the sgRNAs of the invention, wherein the sgRNA produces a lower concentration of immune cytokines in a subject's serum as compared to a control gRNA that is not similarly modified.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

### EXAMPLES

The following examples are provided to illustrate the present disclosure and are not to be construed as limiting the scope of this disclosure in any way.

### Example 1 - Materials and Methods

### A. Synthetic guide RNA (gRNA)

gRNA in both dual (dgRNA, i.e., crRNA and trRNA) and single guide (sgRNA) format were chemically synthesized by commercial vendors with modified nucleotides and linkages as provided in Table 4.

### B. In vitro transcription ("IVT") of Cas9 mRNA

Capped and polyadenylated Cas9 mRNA containing N1-methyl pseudo-U was generated by *in vitro* transcription using a linearized plasmid DNA template and T7 RNA polymerase. Plasmid DNA containing a T7 promoter and a 100 nucleotide (nt) poly(A/T) region was linearized by XbaI and obtained from a commercial manufacturer. The IVT reaction to generate Cas9 modified mRNA was incubated at 37 °C for 4 hours in the following conditions: 50 ng/µL linearized plasmid; 2 mM each of GTP, ATP, CTP, and N1-methyl pseudo-UTP (Trilink); 10 mM ARCA (Trilink); 5 U/µL T7 RNA polymerase (NEB); 1 U/µL Murine RNase inhibitor (NEB); 0.004 U/µL Inorganic *E. coli* pyrophosphatase (NEB); and 1x reaction buffer. After the 4 hr incubation, TURBO DNase (ThermoFisher) was added to a final concentration of 0.01 U/µL, and the reaction was incubated for an additional 30 minutes to remove the DNA template. The Cas9 mRNA was purified from enzyme and nucleotides using standard protocols, including silica binding columns such as a MegaClear Transcription Clean-up kit (ThermoFisher) or precipitation steps using LiCl followed by EtOH with NaOAc. The transcript concentration was determined by measuring the light absorbance at 260 nm (Nanodrop), and the transcript was analyzed by capillary electrophoresis by Bioanlayzer (Agilent).

### C. Cas9 mRNA and gRNA Transfections in Neuro2A Cells

The mouse cell line Neuro2A was cultured in DMEM media supplemented with 10% fetal bovine serum and was plated at a density of 15,000 cells/well in a 96-well plate 24 hours prior to transfection. On the day of transfection, the media was aspirated from cells and replaced with fresh media. Lipofectamine-2000 (Invitrogen) was diluted 1:50 (v/v) in Opti-MEM (Invitrogen). Cas9 mRNA and single guide RNA were diluted separately in Opti-MEM. For the dual guide format, crRNA and trRNA were diluted together in 1: 1 molar ratio in Opti-MEM. Both Cas9 mRNA and gRNA were mixed separately 1:1 (v/v) with diluted Lipofectamine-2000, producing two lipoplexes. After 5 minutes of incubation, lipoplexes were added in succession to cells, for a final concentration of 100 ng Cas9 mRNA/well and 0.4 µL total lipofection reagent. Guides were tested at two dose levels for each experiment, including 25 nM and 2.5 nM, 16.7 nM and 1.67 nM, 10 nM and 1 nM, 8.3 nM and 0.83 nM, and 3 nM and 0.3 nM. For dual guide, this concentration includes equimolar amounts of crRNA and trRNA, such that, for example, 25 nM crRNA and 25 nM trRNA produce 25 nM total dual guide. Cells were lysed 24 hours post transfection, and lysates were used directly in the PCR reaction that was analyzed for editing by NGS.
Cas9 mRNA with 1xNLS (SEQ ID NO: 359): 
GGGUCCCGCAGUCGGCGUCCAGCGGCUCUGCUUGUUCGUGUGUGUCGUUGCAGGCCUUAUUCGGAU
CCAUGGAUAAGAAGUACUCAAUCGGGCUGGAUAUCGGAACUAAUUCCGUGGGUUGGGCAGUGAUCACG
GAUGAAUACAAAGUGCCGUCCAAGAAGUUCAAGGUCCUGGGGAACACCGAUAGACACAGCAUCAAGAA
AAAUCUCAUCGGAGCCCUGCUGUUUGACUCCGGCGAAACCGCAGAAGCGACCCGGCUCAAACGUACCG
CGAGGCGACGCUACACCCGGCGGAAGAAUCGCAUCUGCUAUCUGCAAGAGAUCUUUUCGAACGAAAUG
GCAAAGGUCGACGACAGCUUCUUCCACCGCCUGGAAGAAUCUUUCCUGGUGGAGGAGGACAAGAAGCA
UGAACGGCAUCCUAUCUUUGGAAACAUCGUCGACGAAGUGGCGUACCACGAAAAGUACCCGACCAUCU
ACCAUCUGCGGAAGAAGUUGGUUGACUCAACUGACAAGGCCGACCUCAGAUUGAUCUACUUGGCCCUC
GCCCAUAUGAUCAAAUUCCGCGGACACUUCCUGAUCGAAGGCGAUCUGAACCCUGAUAACUCCGACGU
GGAUAAGCUUUUCAUUCAACUGGUGCAGACCUACAACCAACUGUUCGAAGAAAACCCAAUCAAUGCUA
GCGGCGUCGAUGCCAAGGCCAUCCUGUCCGCCCGGCUGUCGAAGUCGCGGCGCCUCGAAAACCUGAUC
GCACAGCUGCCGGGAGAGAAAAAGAACGGACUUUUCGGCAACUUGAUCGCUCUCUCACUGGGACUCAC
UCCCAAUUUCAAGUCCAAUUUUGACCUGGCCGAGGACGCGAAGCUGCAACUCUCAAAGGACACCUACG
ACGACGACUUGGACAAUUUGCUGGCACAAAUUGGCGAUCAGUACGCGGAUCUGUUCCUUGCCGCUAAG
AACCUUUCGGACGCAAUCUUGCUGUCCGAUAUCCUGCGCGUGAACACCGAAAUAACCAAAGCGCCGCU
UAGCGCCUCGAUGAUUAAGCGGUACGACGAGCAUCACCAGGAUCUCACGCUGCUCAAAGCGCUCGUGA
 
GACAGCAACUGCCUGAAAGUACAAGGAGAUCUUCUUCGACCAGUCCAAGAAUGGGUACGCAGGGUAC
AUCGAUGGAGGCGCUAGCCAGGAAGAGUUCUAUAAGUUCAUCAAGCCAAUCCUGGAAAAGAUGGACGG
AACCGAAGAACUGCUGGUCAAGCUGAACAGGGAGGAUCUGCUCCGGAAACAGAGAACCUUUGACAACG
GAUCCAUUCCCCACCAGAUCCAUCUGGGUGAGCUGCACGCCAUCUUGCGGCGCCAGGAGGACUUUUAC
CCAUUCCUCAAGGACAACCGGGAAAAGAUCGAGAAAAUUCUGACGUUCCGCAUCCCGUAUUACGUGGG
CCCACUGGCGCGCGGCAAUUCGCGCUUCGCGUGGAUGACUAGAAAAUCAGAGGAAACCAUCACUCCUU
GGAAUUUCGAGGAAGUUGUGGAUAAGGGAGCUUCGGCACAAAGCUUCAUCGAACGAAUGACCAACUUC
GACAAGAAUCUCCCAAACGAGAAGGUGCUUCCUAAGCACAGCCUCCUUUACGAAUACUUCACUGUCUA
CAACGAACUGACUAAAGUGAAAUACGUUACUGAAGGAAUGAGGAAGCCGGCCUUUCUGUCCGGAGAAC
AGAAGAAAGCAAUUGUCGAUCUGCUGUUCAAGACCAACCGCAAGGUGACCGUCAAGCAGCUUAAAGAG
GACUACUUCAAGAAGAUCGAGUGUUUCGACUCAGUGGAAAUCAGCGGGGUGGAGGACAGAUUCAACGC
UUCGCUGGGAACCUAUCAUGAUCUCCUGAAGAUCAUCAAGGACAAGGACUUCCUUGACAACGAGGAGA
ACGAGGACAUCCUGGAAGAUAUCGUCCUGACCUUGACCCUUUUCGAGGAUCGCGAGAUGAUCGAGGAG
AGGCUUAAGACCUACGCUCAUCUCUUCGACGAUAAGGUCAUGAAACAACUCAAGCGCCGCCGGUACAC
UGGUUGGGGCCGCCUCUCCCGCAAGCUGAUCAACGGUAUUCGCGAUAAACAGAGCGGUAAAACUAUCC
UGGAUUUCCUCAAAUCGGAUGGCUUCGCUAAUCGUAACUUCAUGCAAUUGAUCCACGACGACAGCCUG
ACCUUUAAGGAGGACAUCCAAAAAGCACAAGUGUCCGGACAGGGAGACUCACUCCAUGAACACAUCGC
GAAUCUGGCCGGUUCGCCGGCGAUUAAGAAGGGAAUUCUGCAAACUGUGAAGGUGGUCGACGAGCUGG
UGAAGGUCAUGGGACGGCACAAACCGGAGAAUAUCGUGAUUGAAAUGGCCCGAGAAAACCAGACUACC
CAGAAGGGCCAGAAAAACUCCCGCGAAAGGAUGAAGCGGAUCGAAGAAGGAAUCAAGGAGCUGGGCAG
CCAGAUCCUGAAAGAGCACCCGGUGGAAAACACGCAGCUGCAGAACGAGAAGCUCUACCUGUACUAUU
UGCAAAAUGGACGGGACAUGUACGUGGACCAAGAGCUGGACAUCAAUCGGUUGUCUGAUUACGACGUG
GACCACAUCGUUCCACAGUCCUUUCUGAAGGAUGACUCGAUCGAUAACAAGGUGUUGACUCGCAGCGA
CAAGAACAGAGGGAAGUCAGAUAAUGUGCCAUCGGAGGAGGUCGUGAAGAAGAUGAAGAAUUACUGGC
GGCAGCUCCUGAAUGCGAAGCUGAUUACCCAGAGAAAGUUUGACAAUCUCACUAAAGCCGAGCGCGGC
GGACUCUCAGAGCUGGAUAAGGCUGGAUUCAUCAAACGGCAGCUGGUCGAGACUCGGCAGAUUACCAA
GCACGUGGCGCAGAUCUUGGACUCCCGCAUGAACACUAAAUACGACGAGAACGAUAAGCUCAUCCGGG
AAGUGAAGGUGAUUACCCUGAAAAGCAAACUUGUGUCGGACUUUCGGAAGGACUUUCAGUUUUACAAA
GUGAGAGAAAUCAACAACUACCAUCACGCGCAUGACGCAUACCUCAACGCUGUGGUCGGUACCGCCCU
GAUCAAAAAGUACCCUAAACUUGAAUCGGAGUUUGUGUACGGAGACUACAAGGUCUACGACGUGAGGA
AGAUGAUAGCCAAGUCCGAACAGGAAAUCGGGAAAGCAACUGCGAAAUACUUCUUUUACUCAAACAUC
AUGAACUUUUUCAAGACUGAAAUUACGCUGGCCAAUGGAGAAAUCAGGAAGAGGCCACUGAUCGAAAC
UAACGGAGAAACGGGCGAAAUCGUGUGGGACAAGGGCAGGGACUUCGCAACUGUUCGCAAAGUGCUCU
CUAUGCCGCAAGUCAAUAUUGUGAAGAAAACCGAAGUGCAAACCGGCGGAUUUUCAAAGGAAUCGAUC
CUCCCAAAGAGAAAUAGCGACAAGCUCAUUGCACGCAAGAAAGACUGGGACCCGAAGAAGUACGGAGG
AUUCGAUUCGCCGACUGUCGCAUACUCCGUCCUCGUGGUGGCCAAGGUGGAGAAGGGAAAGAGCAAAA
AGCUCAAAUCCGUCAAAGAGCUGCUGGGGAUUACCAUCAUGGAACGAUCCUCGUUCGAGAAGAACCCG
AUUGAUUUCCUCGAGGCGAAGGGUUACAAGGAGGUGAAGAAGGAUCUGAUCAUCAAACUCCCCAAGUA
CUCACUGUUCGAACUGGAAAAUGGUCGGAAGCGCAUGCUGGCUUCGGCCGGAGAACUCCAAAAAGGAA
AUGAGCUGGCCUUGCCUAGCAAGUACGUCAACUUCCUCUAUCUUGCUUCGCACUACGAAAAACUCAAA
GGGUCACCGGAAGAUAACGAACAGAAGCAGCUUUUCGUGGAGCAGCACAAGCAUUAUCUGGAUGAAAU
CAUCGAACAAAUCUCCGAGUUUUCAAAGCGCGUGAUCCUCGCCGACGCCAACCUCGACAAAGUCCUGU
CGGCCUACAAUAAGCAUAGAGAUAAGCCGAUCAGAGAACAGGCCGAGAACAUUAUCCACUUGUUCACC
CUGACUAACCUGGGAGCCCCAGCCGCCUUCAAGUACUUCGAUACUACUAUCGAUCGCAAAAGAUACAC
GUCCACCAAGGAAGUUCUGGACGCGACCCUGAUCCACCAAAGCAUCACUGGACUCUACGAAACUAGGA
UCGAUCUGUCGCAGCUGGGUGGCGAUGGCGGUGGAUCUCCGAAAAAGAAGAGAAAGGUGUAAUGAGCU
AGCCAUCACAUUUAAAAGCAUCUCAGCCUACCAUGAGAAUAAGAGAAAGAAAAUGAAGAUCAAUAGCU
UAUUCAUCUCUUUUUCUUUUUCGUUGGUGUAAAGCCAACACCCUGUCUAAAAAACAUAAAUUUCUUUA
AUCAUUUUGCCUCUUUUCUCUGUGCUUCAAUUAAUAAAAAAUGGAAAGAACCUCGAGAAAAAAAAAAA
AAAAAAAAAAAAAAAAAAAAAAAAAAAAAAAAAAAAAAAAAAAAAAAAAAAAAAAAAAAAAAAAAAAA
AAAAAAAAAAAAAAAAAAAAAUCUAG
Cas9 mRNA with 2xNLS and HA tag (SEQ ID NO: 360):

GGGUCCCGCAGUCGGCGUCCAGCGGCUCUGCUUGUUCGUGUGUGUGUCGUUGCAGGCCUUAUUCGGAU
CCAUGGAUAAGAAGUACUCAAUCGGGCUGGAUAUCGGAACUAAUUCCGUGGGUUGGGCAGUGAUCACG
GAUGAAUACAAAGUGCCGUCCAAGAAGUUCAAGGUCCUGGGGAACACCGAUAGACACAGCAUCAAGAA
AAAUCUCAUCGGAGCCCUGCUGUUUGACUCCGGCGAAACCGCAGAAGCGACCCGGCUCAAACGUACCG
CGAGGCGACGCUACACCCGGCGGAAGAAUCGCAUCUGCUAUCUGCAAGAGAUCUUUUCGAACGAAAUG
GCAAAGGUCGACGACAGCUUCUUCCACCGCCUGGAAGAAUCUUUCCUGGUGGAGGAGGACAAGAAGCA
UGAACGGCAUCCUAUCUUUGGAAACAUCGUCGACGAAGUGGCGUACCACGAAAAGUACCCGACCAUCU
ACCAUCUGCGGAAGAAGUUGGUUGACUCAACUGACAAGGCCGACCUCAGAUUGAUCUACUUGGCCCUC
GCCCAUAUGAUCAAAUUCCGCGGACACUUCCUGAUCGAAGGCGAUCUGAACCCUGAUAACUCCGACGU
GGAUAAGCUUUUCAUUCAACUGGUGCAGACCUACAACCAACUGUUCGAAGAAAACCCAAUCAAUGCUA
GCGGCGUCGAUGCCAAGGCCAUCCUGUCCGCCCGGCUGUCGAAGUCGCGGCGCCUCGAAAACCUGAUC
GCACAGCUGCCGGGAGAGAAAAAGAACGGACUUUUCGGCAACUUGAUCGCUCUCUCACUGGGACUCAC
UCCCAAUUUCAAGUCCAAUUUUGACCUGGCCGAGGACGCGAAGCUGCAACUCUCAAAGGACACCUACG
ACGACGACUUGGACAAUUUGCUGGCACAAAUUGGCGAUCAGUACGCGGAUCUGUUCCUUGCCGCUAAG
AACCUUUCGGACGCAAUCUUGCUGUCCGAUAUCCUGCGCGUGAACACCGAAAUAACCAAAGCGCCGCU
UAGCGCCUCGAUGAUUAAGCGGUACGACGAGCAUCACCAGGAUCUCACGCUGCUCAAAGCGCUCGUGA
GACAGCAACUGCCUGAAAAGUACAAGGAGAUCUUCUUCGACCAGUCCAAGAAUGGGUACGCAGGGUAC
AUCGAUGGAGGCGCUAGCCAGGAAGAGUUCUAUAAGUUCAUCAAGCCAAUCCUGGAAAAGAUGGACGG
AACCGAAGAACUGCUGGUCAAGCUGAACAGGGAGGAUCUGCUCCGGAAACAGAGAACCUUUGACAACG
GAUCCAUUCCCCACCAGAUCCAUCUGGGUGAGCUGCACGCCAUCUUGCGGCGCCAGGAGGACUUUUAC
CCAUUCCUCAAGGACAACCGGGAAAAGAUCGAGAAAAUUCUGACGUUCCGCAUCCCGUAUUACGUGGG
CCCACUGGCGCGCGGCAAUUCGCGCUUCGCGUGGAUGACUAGAAAAUCAGAGGAAACCAUCACUCCUU
GGAAUUUCGAGGAAGUUGUGGAUAAGGGAGCUUCGGCACAAAGCUUCAUCGAACGAAUGACCAACUUC
GACAAGAAUCUCCCAAACGAGAAGGUGCUUCCUAAGCACAGCCUCCUUUACGAAUACUUCACUGUCUA
CAACGAACUGACUAAAGUGAAAUACGUUACUGAAGGAAUGAGGAAGCCGGCCUUUCUGUCCGGAGAAC
AGAAGAAAGCAAUUGUCGAUCUGCUGUUCAAGACCAACCGCAAGGUGACCGUCAAGCAGCUUAAAGAG
GACUACUUCAAGAAGAUCGAGUGUUUCGACUCAGUGGAAAUCAGCGGGGUGGAGGACAGAUUCAACGC
UUCGCUGGGAACCUAUCAUGAUCUCCUGAAGAUCAUCAAGGACAAGGACUUCCUUGACAACGAGGAGA
ACGAGGACAUCCUGGAAGAUAUCGUCCUGACCUUGACCCUUUUCGAGGAUCGCGAGAUGAUCGAGGAG
AGGCUUAAGACCUACGCUCAUCUCUUCGACGAUAAGGUCAUGAAACAACUCAAGCGCCGCCGGUACAC
UGGUUGGGGCCGCCUCUCCCGCAAGCUGAUCAACGGUAUUCGCGAUAAACAGAGCGGUAAAACUAUCC
UGGAUUUCCUCAAAUCGGAUGGCUUCGCUAAUCGUAACUUCAUGCAAUUGAUCCACGACGACAGCCUG
ACCUUUAAGGAGGACAUCCAAAAAGCACAAGUGUCCGGACAGGGAGACUCACUCCAUGAACACAUCGC
GAAUCUGGCCGGUUCGCCGGCGAUUAAGAAGGGAAUUCUGCAAACUGUGAAGGUGGUCGACGAGCUGG
UGAAGGUCAUGGGACGGCACAAACCGGAGAAUAUCGUGAUUGAAAUGGCCCGAGAAAACCAGACUACC
CAGAAGGGCCAGAAAAACUCCCGCGAAAGGAUGAAGCGGAUCGAAGAAGGAAUCAAGGAGCUGGGCAG
CCAGAUCCUGAAAGAGCACCCGGUGGAAAACACGCAGCUGCAGAACGAGAAGCUCUACCUGUACUAUU
UGCAAAAUGGACGGGACAUGUACGUGGACCAAGAGCUGGACAUCAAUCGGUUGUCUGAUUACGACGUG
GACCACAUCGUUCCACAGUCCUUUCUGAAGGAUGACUCGAUCGAUAACAAGGUGUUGACUCGCAGCGA
CAAGAACAGAGGGAAGUCAGAUAAUGUGCCAUCGGAGGAGGUCGUGAAGAAGAUGAAGAAUUACUGGC
GGCAGCUCCUGAAUGCGAAGCUGAUUACCCAGAGAAAGUUUGACAAUCUCACUAAAGCCGAGCGCGGC
GGACUCUCAGAGCUGGAUAAGGCUGGAUUCAUCAAACGGCAGCUGGUCGAGACUCGGCAGAUUACCAA
GCACGUGGCGCAGAUCUUGGACUCCCGCAUGAACACUAAAUACGACGAGAACGAUAAGCUCAUCCGGG
AAGUGAAGGUGAUUACCCUGAAAAGCAAACUUGUGUCGGACUUUCGGAAGGACUUUCAGUUUUACAAA
GUGAGAGAAAUCAACAACUACCAUCACGCGCAUGACGCAUACCUCAACGCUGUGGUCGGUACCGCCCU
GAUCAAAAAGUACCCUAAACUUGAAUCGGAGUUUGUGUACGGAGACUACAAGGUCUACGACGUGAGGA
AGAUGAUAGCCAAGUCCGAACAGGAAAUCGGGAAAGCAACUGCGAAAUACUUCUUUUACUCAAACAUC
AUGAACUUUUUCAAGACUGAAAUUACGCUGGCCAAUGGAGAAAUCAGGAAGAGGCCACUGAUCGAAAC
UAACGGAGAAACGGGCGAAAUCGUGUGGGACAAGGGCAGGGACUUCGCAACUGUUCGCAAAGUGCUCU
CUAUGCCGCAAGUCAAUAUUGUGAAGAAAACCGAAGUGCAAACCGGCGGAUUUUCAAAGGAAUCGAUC
CUCCCAAAGAGAAAUAGCGACAAGCUCAUUGCACGCAAGAAAGACUGGGACCCGAAGAAGUACGGAGG
AUUCGAUUCGCCGACUGUCGCAUACUCCGUCCUCGUGGUGGCCAAGGUGGAGAAGGGAAAGAGCAAAA
AGCUCAAAUCCGUCAAAGAGCUGCUGGGGAUUACCAUCAUGGAACGAUCCUCGUUCGAGAAGAACCCG
AUUGAUUUCCUCGAGGCGAAGGGUUACAAGGAGGUGAAGAAGGAUCUGAUCAUCAAACUCCCCAAGUA
CUCACUGUUCGAACUGGAAAAUGGUCGGAAGCGCAUGCUGGCUUCGGCCGGAGAACUCCAAAAAGGAA
AUGAGCUGGCCUUGCCUAGCAAGUACGUCAACUUCCUCUAUCUUGCUUCGCACUACGAAAAACUCAAA

GGGUCACCGGAAGAUAACGAACAGAAGCAGCUUUCGUGGAGCAGCACAAGCAUUAUCUGGAUGAAAU
CAUCGAACAAAUCUCCGAGUUUUCAAAGCGCGUGAUCCUCGCCGACGCCAACCUCGACAAAGUCCUGU
CGGCCUACAAUAAGCAUAGAGAUAAGCCGAUCAGAGAACAGGCCGAGAACAUUAUCCACUUGUUCACC
CUGACUAACCUGGGAGCCCCAGCCGCCUUCAAGUACUUCGAUACUACUAUCGAUCGCAAAAGAUACAC
GUCCACCAAGGAAGUUCUGGACGCGACCCUGAUCCACCAAAGCAUCACUGGACUCUACGAAACUAGGA
UCGAUCUGUCGCAGCUGGGUGGCGAUGGCUCGGCUUACCCAUACGACGUGCCUGACUACGCCUCGCUC
GGAUCGGGCUCCCCCAAAAAGAAACGGAAGGUGGACGGAUCCCCGAAAAAGAAGAGAAAGGUGGACUC
CGGAUGAGAAUUAUGCAGUCUAGCCAUCACAUUUAAAAGCAUCUCAGCCUACCAUGAGAAUAAGAGAA
AGAAAAUGAAGAUCAAUAGCUUAUUCAUCUCUUUUUCUUUUUCGUUGGUGUAAAGCCAACACCCUGUC
UAAAAAACAUAAAUUUCUUUAAUCAUUUUGCCUCUUUUCUCUGUGCUUCAAUUAAUAAAAAAUGGAAA
GAACCUCGAGAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAUCUAG

### D. Primary liver hepatocytes

Primary mouse liver hepatocytes (PMH) (Gibco) were cultured per the manufacturer's protocol (Invitrogen, protocol 11.28.2012). In brief, the cells were thawed and resuspended in hepatocyte thawing medium with supplements (Gibco, Cat. CM7000) followed by centrifugation at 100 g for 10 minutes. The supernatant was discarded and the pelleted cells resuspended in hepatocyte plating medium plus supplement pack (Invitrogen, Cat. A1217601 and CM3000). Cells were counted and plated on Bio-coat collagen I coated 96-well plates (ThermoFisher, Cat. 877272) at a density of 15,000 cells/well and incubated for 5 hours at 37°C and 5% CO2 atmosphere to allow for monolayer formation. After 5 hours, the plating media was removed and replaced with supplemented hepatocyte culture medium (Invitrogen, Cat. A1217601 and CM4000) containing LNP formulated Cas9 mRNA and guide RNA plus 3% mouse serum. LNPs were diluted from a starting dose level of 100ng Cas9 mRNA and approximately 30nM guide RNA per well, carrying out serial dilutions down to 0.1ng mRNA and 0.03nM guide per well. Cells were incubated for approximately 48 hours at 37°C and 5% CO₂ atmosphere before cell lysis and NGS analysis as described herein.

### E. Lipid Nanoparticle ("LNP") Formulation

LNPs were formulated with a cationic lipid amine to RNA phosphate (N:P) molar ratio of about 4.5. The lipid nanoparticle components were dissolved in 100% ethanol with the following molar ratios: 45 mol-% (12.7 mM) cationic lipid *(e.g.,* (9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate); 44 mol-% (12.4 mM) helper lipid *(e.g.,* cholesterol); 9 mol-% (2.53 mM) neutral lipid (*e.g.,* DSPC); and 2 mol-% (.563 mM) PEG (*e.g.,* PEG2k-DMG). The RNA cargo were prepared in 25 mM sodium acetate buffer, pH 4.5, resulting in a concentration of RNA cargo of approximately 0.45 mg/mL.

The LNPs were formed by microfluidic mixing of the lipid and RNA solutions using a Precision Nanosystems NanoAssemblr^{™} Benchtop Instrument, according to the manufacturer's protocol. A 2:1 ratio of aqueous to organic solvent was maintained during mixing using differential flow rates.

LNP Formulation Procedure A: After mixing, the LNPs were collected, diluted in phosphate buffered saline (PBS, approximately 1:1), and then remaining buffer was exchanged into PBS (100-fold excess of sample volume), overnight at 4°C under gentle stirring using a 10 kDa Slide-a-LyzerTM G2 Dialysis Cassette (ThermoFisher Scientific). The LNPs were concentrated using 10kDa Amicon spin filter (centrifugation at 4000g at 4°C) to achieve the desired concentration. The resulting mixture was then filtered using a 0.2 µm sterile filter. The resulting filtrate was stored at 2-8 °C.

LNP Formulation Procedure B: After mixing, the LNPs were collected, diluted in 50mM Tris at pH 7.5 (approximately 1:1), and then LNPs were exchanged into 50mM Tris at pH 7.5 (100-fold excess of sample volume), overnight at 4°C under gentle stirring using a 10 kDa Slide-a-LyzerTM G2 Dialysis Cassette (ThermoFisher Scientific). The LNPs were concentrated using 10kDa Amicon spin filter (centrifugation at 4000g at 4°C) to achieve twice the desired concentration. These concentrated LNPs were mixed 1:1 with 50mM Tris, 90mM NaCl, 10% sucrose at pH 7.5 (2X TSS). The resulting mixture was then filtered using a 0.2 µM sterile filter. The resulting filtrate was stored at -80°C.

LNP Formulation Procedure C: The RNA cargo were prepared in 25mM sodium citrate, 100mM sodium chloride at pH 5 resulting in a concentration of RNA cargo of approximately 0.45 mg/mL. After mixing, the LNPs were collected in water at the ratio of 3:1. The LNPs were incubated for an hour at room temperature and mixed 1:1 with water. Then they were buffer-exchanged into 1X TSS (50mM Tris, 45mM NaCl, 5% sucrose at pH 7.5) on PD-10 columns (GE Healthcare), using manufacturer's protocol. The LNPs were concentrated using 10kDa Amicon spin filter (centrifugation at 4000g at 4°C) to achieve the desired concentration. The resulting mixture was then filtered using a 0.2 µm sterile filter. The resulting filtrate was stored at -80°C.

### F. Next-Generation Sequencing ("NGS") and Analysis for On-Target Cleavage Efficiency

To quantitatively determine the efficiency of editing at the target location in the genome, deep sequencing was utilized to identify the presence of insertions and deletions introduced by gene editing.

PCR primers were designed around the target site *(e.g.,* TTR, FVII), and the genomic area of interest was amplified. Primer sequences are provided below in Table 5.

**Table 5**

| **Guide** | **Gene** | **Forward Primer (5'-3')** | **SEQ ID** | **Reverse Primer (5'-3')** | **SEQ ID** |
|---|---|---|---|---|---|
| For experiments with guides based on CR000686/G000209 targeting domains | TTR | AGTCAATAATCAGAATCAGCAGGT | 333 | AGAAGGCACTTCTTCTTTATCTAAGGT | 337 |
| For experiments with guides based on CR000705/G000211 targeting domains | TTR | GTTTTGTTCCAGAGTCTATCACCG | 334 | ACACGAATAAGAGCAAATGGGAAC | 338 |
| For experiments with guides based on G000269/ G000285 targeting domains | TTR | ATTACCAGCTTAGCATCCTGTGAA | 335 | ACACGGTTTATAGAGCAAGAACAC | 339 |
| For experiments with guides based on CR000657/G000208 targeting domains | FVII | AGCACATGAGACCTTCTGTTTCTC | 336 | GACATAGGTGTGACCCTCACAATC | 340 |

Additional PCR was performed according to the manufacturer's protocols (Illumina) to add the necessary chemistry for sequencing. The amplicons were sequenced on an Illumina MiSeq instrument. The reads were aligned to the human reference genome (*e.g*., hg38) after eliminating those having low quality scores. The resulting files containing the reads were mapped to the reference genome (BAM files), where reads that overlapped the target region of interest were selected and the number of wild type reads versus the number of reads which contain an insertion, substitution, or deletion was calculated.

The editing percentage (*e.g*., the "editing efficiency" or "percent editing") is defined as the total number of sequence reads with insertions or deletions over the total number of sequence reads, including wild type.

### G. LNP Delivery in vivo

CD-1 female mice, ranging 6-10 weeks of age were used in each study. Animals were weighed and grouped according to body weight for preparing dosing solutions based on group average weight. LNPs were dosed via the lateral tail vein in a volume of 0.2 mL per animal (approximately 10 mL per kilogram body weight). The animals were observed at approximately 6 hours post dose for adverse effects. Body weight was measured at twenty-four hours post-administration, and animals were euthanized at various time points by exsanguination via cardiac puncture under isoflourane anesthesia. Blood was collected into serum separator tubes or into tubes containing buffered sodium citrate for plasma as described herein. For studies involving *in vivo* editing, liver tissue was collected from the median lobe from each animal for DNA extraction and analysis.

### H. Cytokine induction analysis

For this analysis, approximately 50-100 µL of blood was collected by tail vein nick for serum cytokine measurements. Blood was allowed to clot at room temperature for approximately 2 hours, and then centrifuged at 1000xg for 10 minutes before collecting the serum. A Luminex based magnetic bead multiplex assay (Affymetrix ProcartaPlus, catalog number Exp040-00000-801) measuring IL-6, TNF-alpha, IFN-alpha, and MCP-1 was used for cytokine analysis in collected in samples. Kit reagents and standards were prepared as directed in the manufacturer's protocol. 25 µL of mouse serum was added to wells containing 25 µL of the diluted antibody coated magnetic beads. The plate was incubated for 2 hours at room temperature and then washed. Diluted biotin antibody (50 µL) was added to the beads and incubated for 1 hour at room temperature. The beads were washed again before adding 50 µL of diluted streptavidin-PE to each well, followed by incubation for 30 minutes. The beads were washed once again and then suspended in 100 µL of wash buffer and read on the Bio-Plex 200 instrument (Bio-Rad). The data was analyzed using Bioplex Manager ver. 6.1 analysis package with cytokine concentrations calculated off a standard curve using a five parameter logistic curve fit.

### I. Genomic DNA Isolation

For the *in vivo* studies, genomic DNA was extracted from 10 mg of tissue using a bead based extraction kit, MagMAX-96 DNA Multi-Sample Kit (ThermoFisher, Cat #4413020) according to manufacturer's protocol, which includes homogenizing the tissue in lysis buffer (approximately 400 µL/10 mg tissue). All DNA samples were normalized to 100 ng/µL concentration for PCR and subsequent NGS analysis, as described herein.

### J. Transthyretin (TTR) ELISA analysis

Blood was collected and the serum was isolated as indicated. The total TTR serum levels were determined using a Mouse Prealbumin (Transthyretin) ELISA Kit (Aviva Systems Biology, Cat. OKIA00111). Kit reagents and standards were prepared according to the manufacture's protocol. Mouse serum was diluted to a final dilution of 10,000-fold with 1x assay diluent. This was done by carrying out two sequential 50-fold dilutions resulting in a 2500-fold dilution. A final 4-fold dilution step was carried out for a total sample dilution of 10,000-fold. Both standard curve dilutions (100 µL each) and diluted serum samples were added to each well of the ELISA plate pre-coated with capture antibody. The plate was incubated at room temperature for 30 minutes before washing. Enzyme-antibody conjugate (100 µL per well) was added for a 20-minute incubation. Unbound antibody conjugate was removed and the plate was washed again before the addition of the chromogenic substrate solution. The plate was incubated for 10 minutes before adding 100 µL of the stop solution, e.g., sulfuric acid (approximately 0.3 M). The plate was read on a SpectraMax M5 plate reader at an absorbance of 450 nm. Serum TTR levels were calculated by SoftMax Pro software ver. 6.4.2 using a four parameter logistic curve fit off the standard curve. Final serum values were adjusted for the assay dilution.

### Example 2 - Engineering modified gRNA and in vitro testing

Modified gRNAs were designed in the dual guide format (dgRNA), as shown in Table 4. Accordingly, both modified crRNAs and trRNAs were designed and chemically synthesized to allow for the pairing of modified and unmodified components forming dgRNA. These pairings were transfected into Neuro2A cells at concentrations as indicated in the figures and editing efficiency (e.g., percent editing) was measured by NGS, as described in Example 1.

Certain modified crRNAs from Table 4 targeting the mouse TTR gene were transfected with Cas9 mRNA and unmodified trRNA (TR000002). Tested guides included SEQ ID Nos: 1- 18. As shown in Figure 1, some of the modified crRNAs (together with unmodified trRNA) conferred similar or enhanced activity as compared to the unmodified control, while other modified crRNAs decreased activity.

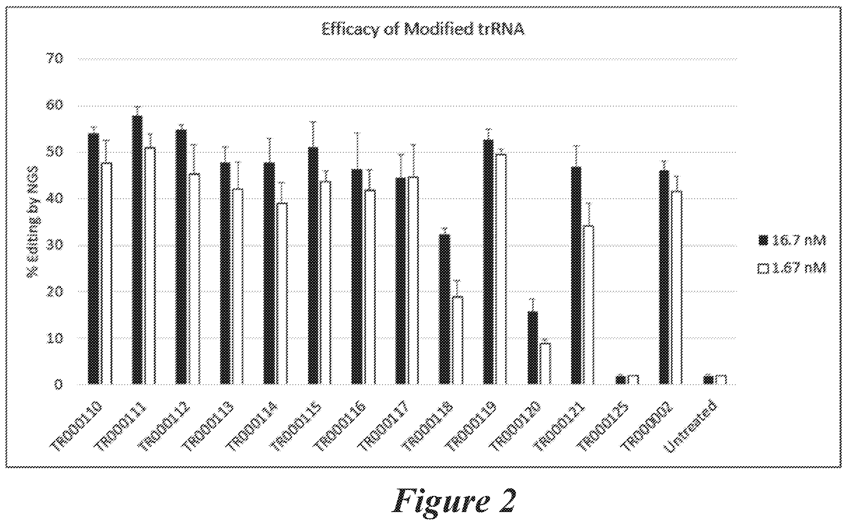

In parallel, modified trRNAs from Table 4 were transfected with Cas9 mRNA along with an unmodified crRNA (CR000686) targeting the same sequence of the mouse TTR gene. Tested guides included SEQ ID Nos: 188 - 200, and 204. As shown in Figure 2, many of the modified trRNAs (together with unmodified crRNA) conferred similar or enhanced activity as compared to the unmodified control, while some of the modified trRNAs decreased activity.

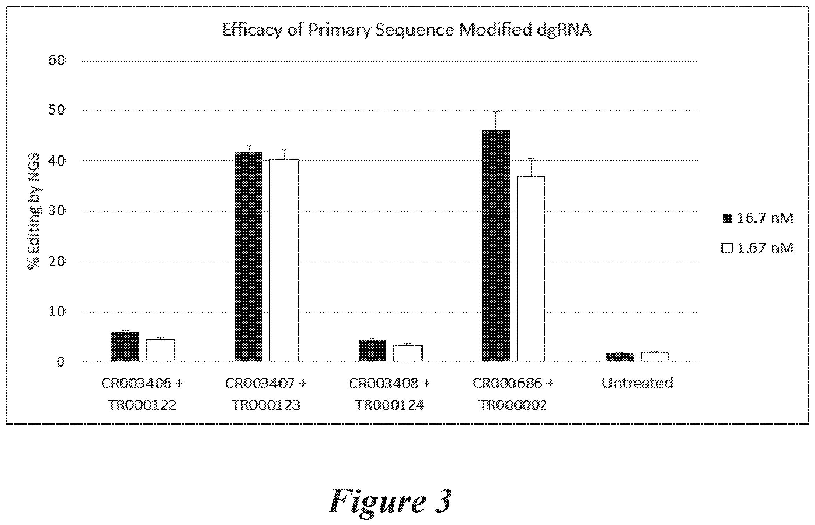

In addition to substituting chemically modified nucleotides, some of the crRNA and trRNA pairings tested were also engineered with sequence substitutions, e.g., resulting in G-C pairings not found in the parental sequences. Tested guides included SEQ ID Nos: 15 and 201; 16 and 202; 1 and 188. As shown in Figure 3, one such pairing (SEQ ID Nos: 16 and 202) resulted in similar or enhanced activity as compared to the unmodified control, while two of the pairings decreased activity.

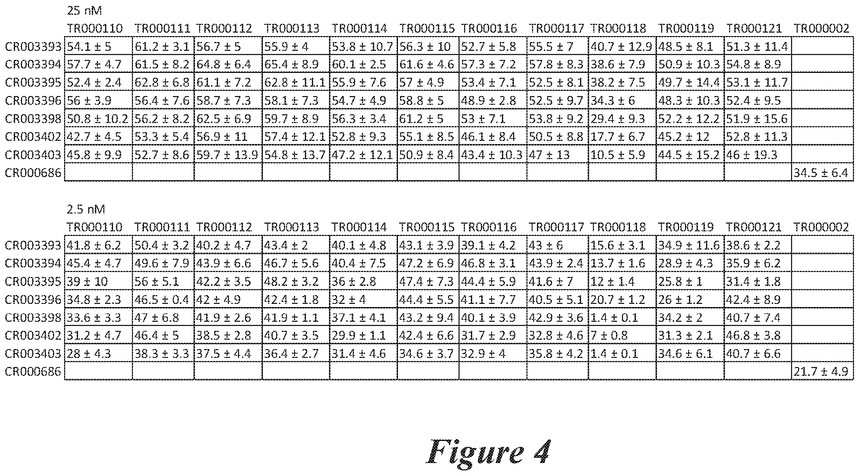

Next, pairings of modified crRNAs and modified trRNAs from Table 4 were tested. As shown in Figure 4, some of the pairings of modified crRNA with modified trRNA conferred similar or enhanced activity as compared to the unmodified controls, while some of the pairings decreased activity. In Figure 4, the column headings depict different trRNA used in the experiment, and the row headings depict different crRNA used. To determine the combination used in the experiment, you match column to row. TR000002 and CR000686 are the unmodified controls (see lower right cells).

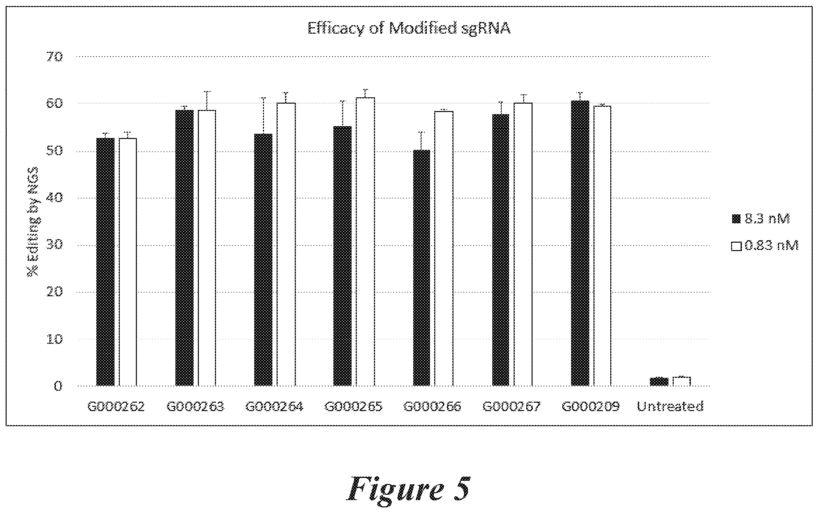

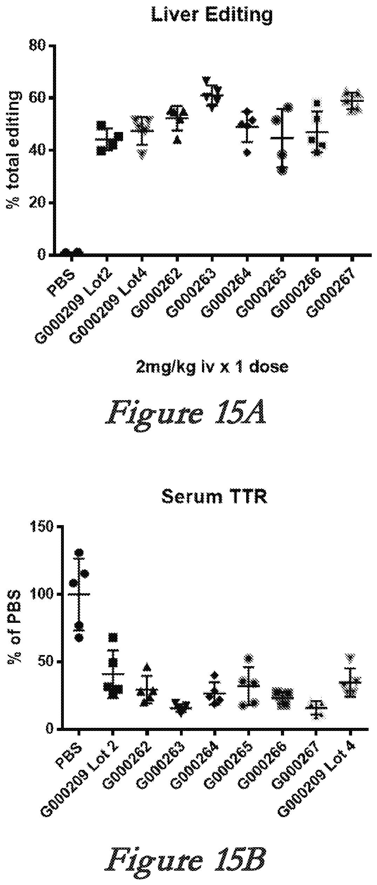

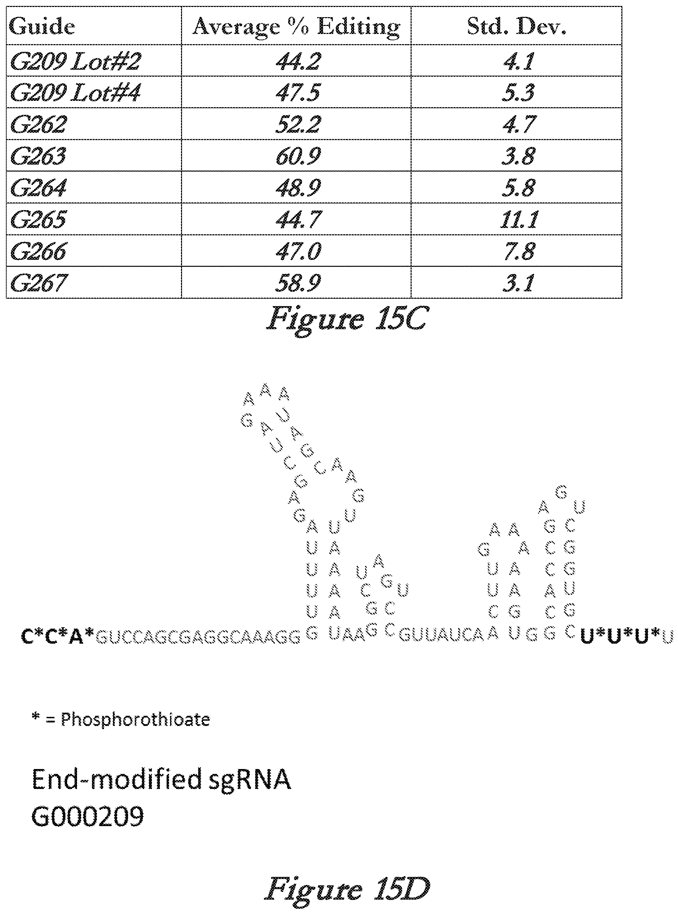

Based on the dgRNA designs, corresponding single guide RNAs (sgRNAs) were engineered featuring aspects of some of the modified crRNAs and trRNAs, as depicted in Table 4 and Figure 15D. These sgRNAs, SEQ ID Nos: 228 - 234, were also tested in Neuro2A cells, and as shown in Figure 5, each of the modified sgRNAs displayed activities comparable to the controls containing only 5' and 3' end modifications (G0000209; SEQ ID NO: 228).

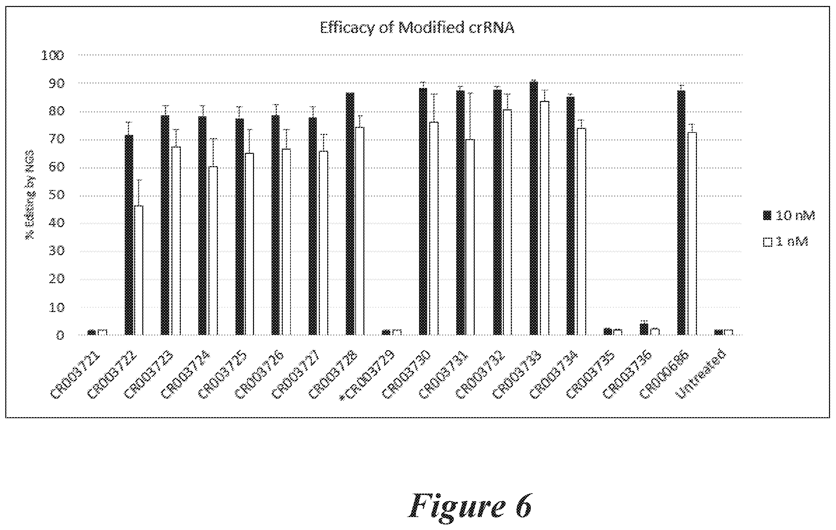

A similar set of experiments were conducted for additional dgRNAs guides depicted in Table 4 and Figure 6. Tested guides included SEQ ID Nos: 32 - 47, and 1. Modified crRNAs also targeting the mouse TTR gene were transfected with Cas9 mRNA and unmodified trRNA (TR000002). As shown in Figure 6, some of the modified crRNAs (together with unmodified trRNA) conferred similar or enhanced activity as compared to the unmodified control (CR000686), while other modified crRNAs decreased activity.

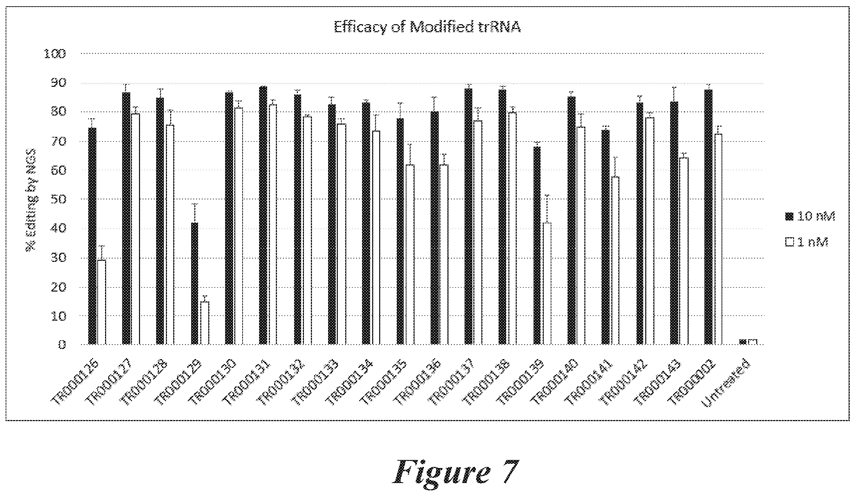

In parallel, as shown in Figure 7, modified trRNAs from Table 4 were transfected with Cas9 mRNA along with an unmodified crRNA (CR000686) targeting the same sequence of the mouse TTR gene. Tested guides included SEQ ID Nos: 205 - 222, and 1. As shown in Figure 7, many of the modified trRNAs (together with unmodified crRNA) conferred similar or enhanced activity as compared to the unmodified control (TR000002), while some of the modified trRNAs decreased activity.

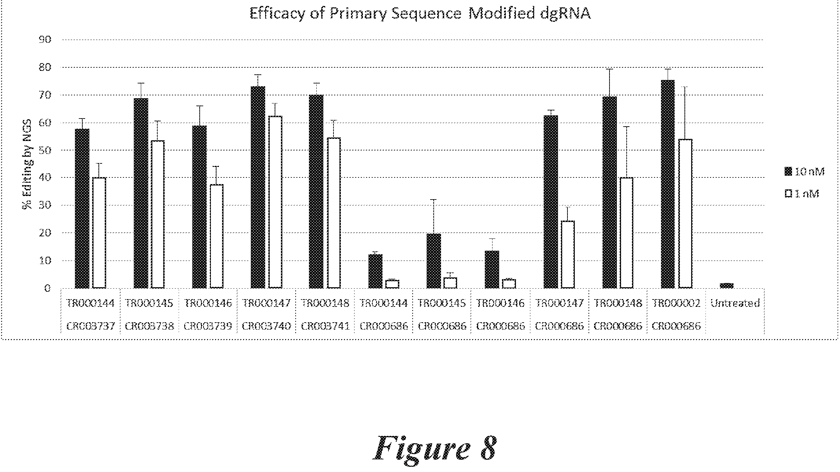

In addition to substituting chemically modified nucleotides, some of the crRNA and trRNA pairings tested from Table 4 were also engineered with sequence substitutions, e.g., resulting in G-C pairings or G-U mismatches ("GU wobbles") not found in the parental sequences. As shown in Figure 8, some of the modifications and pairings conferred similar or enhanced activity as compared to the unmodified control, while some (e.g., the "GU wobble" or mismatch pairings) decreased activity. Figure 8 shows results using trRNA guides shown in SEQ ID Nos: 223-227 and 188 with crRNA guides shown in SEQ ID Nos: 48-52, and 1.

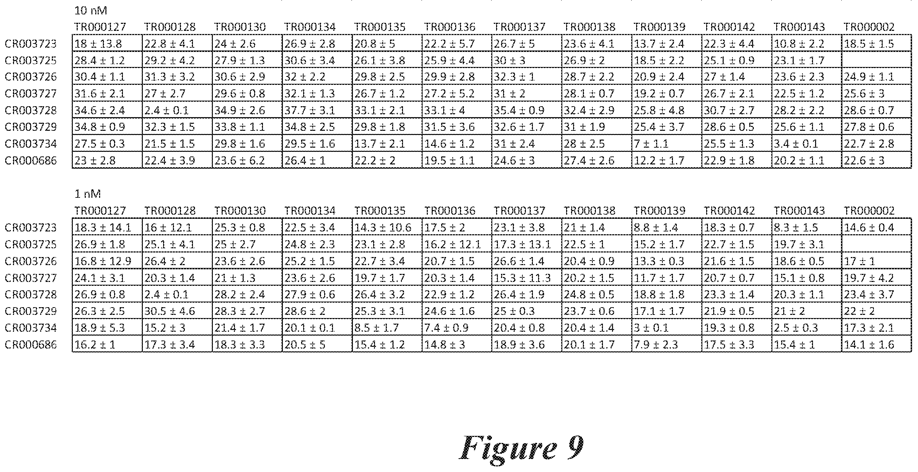

Next, select pairings of the modified crRNAs and modified trRNAs from Table 4 were tested as shown in Figure 9. Some of the pairings of modified crRNA with modified trRNA conferred similar or enhanced activity as compared to the unmodified controls, while some of the pairings decreased activity. In Figure 9, the column headings depict different trRNA used in the experiment, and the row headings depict different crRNA used. To determine the combination used in the experiment, you match column to row. Unmodified controls are TR000002, and CR000686.

Some of the modified gRNAs (dgRNAs and sgRNAs) from Table 4 were also tested in a purely biochemical assay (i.e., cell free cleavage assay). Interestingly, many of the modified gRNAs that were largely inactive in the Neuro2A cells were active in the biochemical assay, indicating that such biochemical assays may not be predictive of modified gRNA activity in cells (data not shown).

### Example 3. Further testing of modified gRNAs to other targets

Having established that certain modifications affected gRNA activity, it was next tested whether these modifications would affect the activity when targeting (1) a separate sequence in the same gene or (2) a sequence in a different gene. Accordingly, gRNAs targeting another sequence in the mouse TTR gene as well as a sequence in the mouse Factor-VII (FVII) gene were engineered and synthesized having certain modification patterns tested in Example 2 (see Table 4). These gRNAs were transfected into Neuro2A cells at the concentrations indicated in the figures and editing efficiency (e.g., percent editing) was measured by NGS, as described in Example 1.

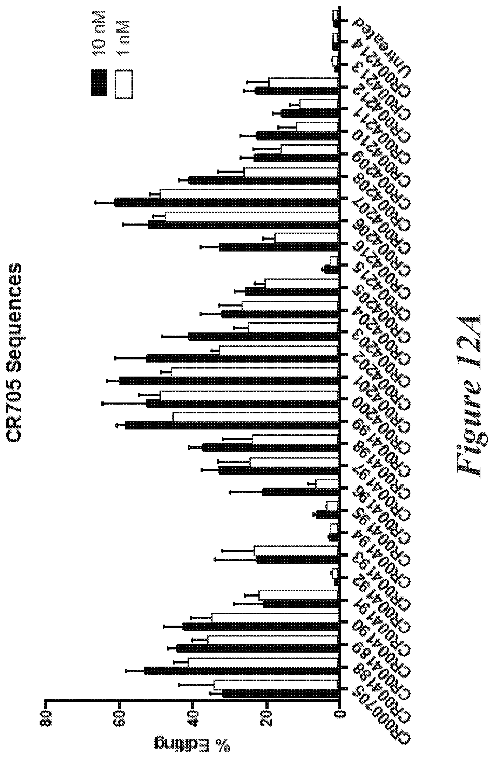

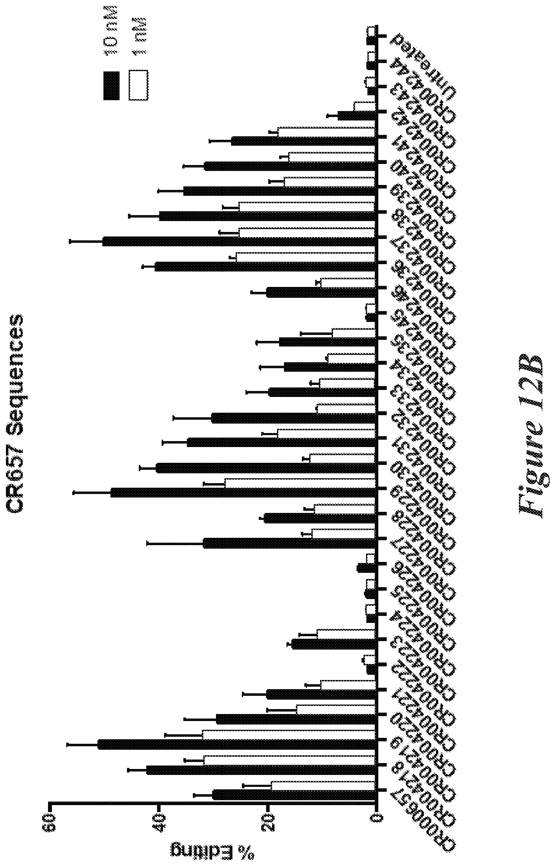

Modified crRNAs from Table 4 targeting either the mouse TTR gene (different sequence as targeted in Example 2) or the mouse FVII gene, were transfected with Cas9 mRNA and unmodified trRNA (TR000002). Tested guides included those shown in Figures 12A and 12B. Some of the modified crRNAs (together with unmodified trRNA) conferred similar or enhanced activity as compared to the unmodified controls, while other modified crRNAs decreased activity.

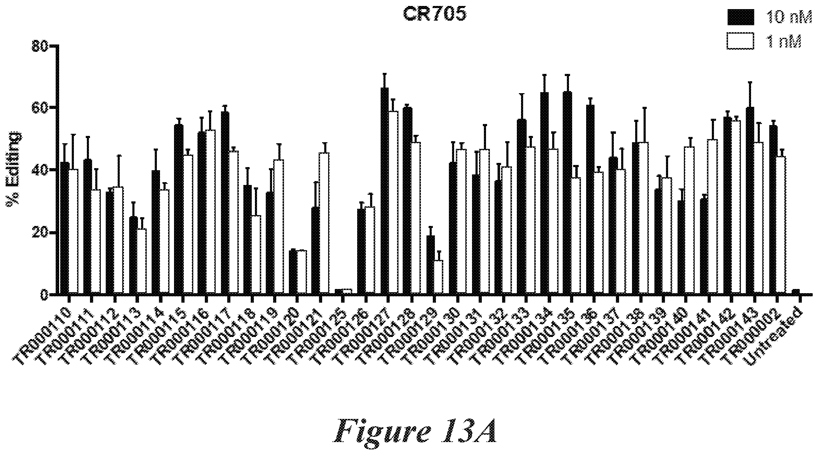

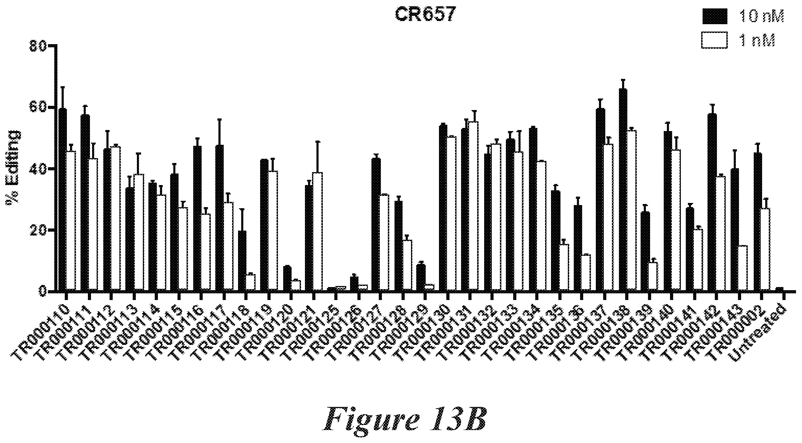

In parallel, modified trRNAs from Table 4 were transfected with Cas9 mRNA along with an unmodified crRNA targeting the same sequence of the mouse TTR gene (CR000705; different sequence as targeted in Example 2) or the same sequence as the mouse FVII gene (CR000657). As shown in Figures 13A and 13B, many of the modified trRNAs (together with unmodified crRNAs) conferred similar or enhanced activity as compared to the unmodified controls, while some of the modified trRNAs decreased activity. This data shows that certain modification patterns tended to have similar effects over the different sequences.

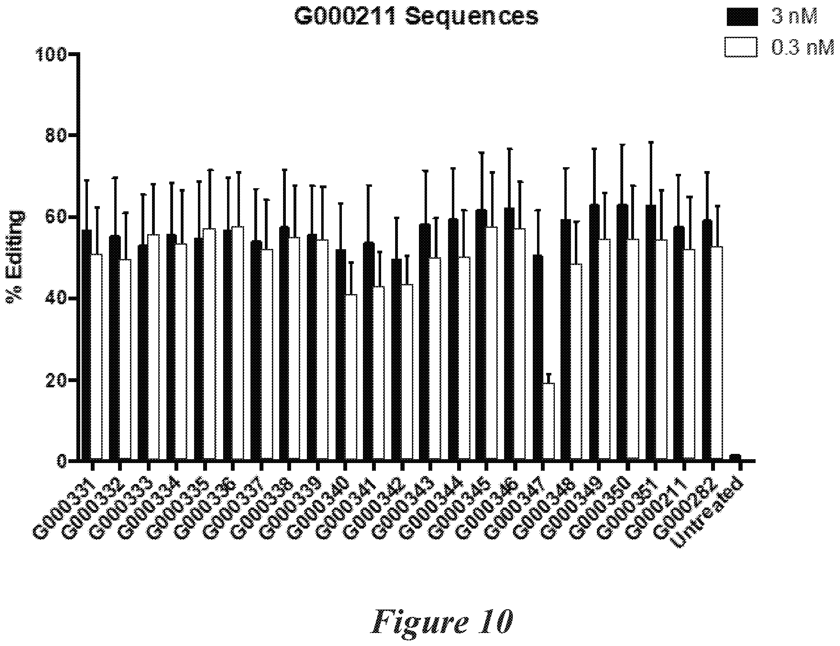

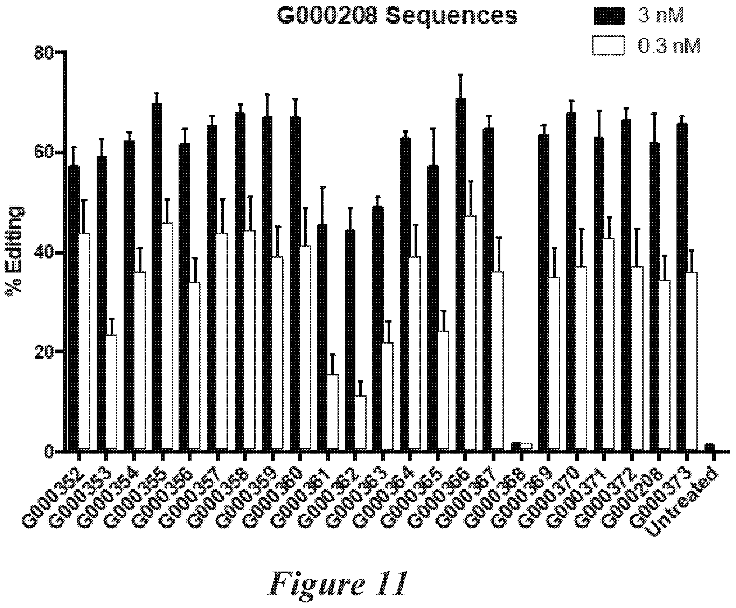

Based on the dgRNA designs described above, corresponding single guide RNAs (sgRNAs) were engineered featuring aspects of some of the modified crRNAs and trRNAs. *See,* Table 4. These sgRNAs were also tested in Neuro2A cells. Results are shown in Figure 10 (mouse TTR) and Figure 11 (mouse FVII). These experiments show that some modification patterns result in similar effects even when targeting different genes.

### Example 4. Testing of modified gRNA in vivo

Following the *in vitro* testing, modified sgRNAs were delivered to animals in six separate studies in order to determine whether the modifications conferred any benefits for editing *in vivo.*

LNPs were formulated with IVT Cas9 mRNA together with chemically modified sgRNA (targeting TTR or FVII), as described in Example 1. The ratio of mRNA:sgRNA was approximately 1:1, by weight of the RNA components. Unless otherwise indicated, the Cas9 mRNA used in the studies described in this example had the sequence of SEQ ID NO: 360 and the LNPs were formulated using LNP Formulation Procedure A described above.

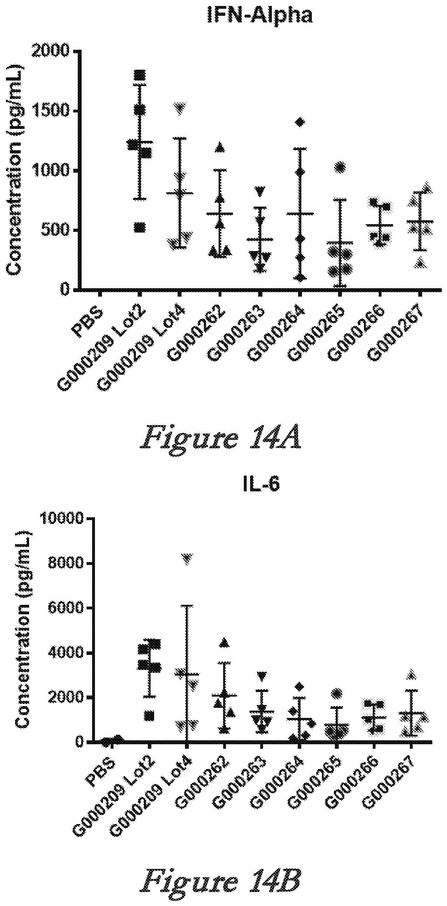

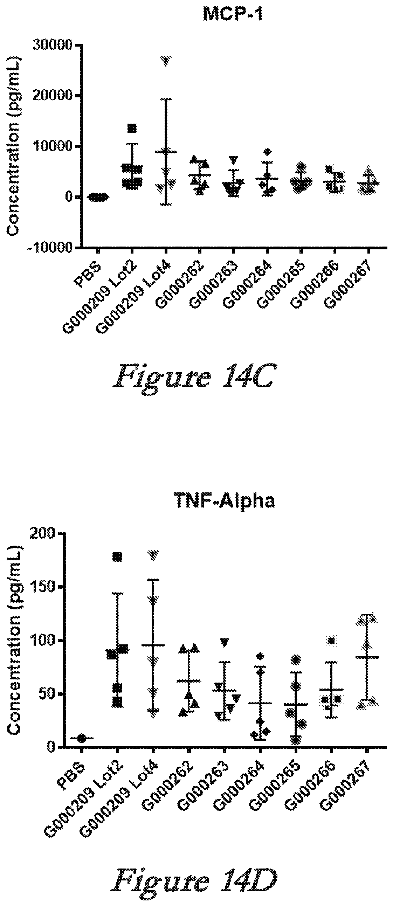

In one experiment, mice (n=5 per group) were administered a single dose of LNP at 2mg/kg and blood was collected four hours post dose for serum cytokine analysis. 7 days post dose at necropsy, livers and blood were collected for NGS measurements of editing efficiency and serum TTR analysis, respectively. Each of the sgRNAs in this experiment targeted the same sequence in the TTR gene, the only difference between the sgRNAs being the modifications made to each (See Figures 14A-D and 15A-E; Table 4 SEQ ID Nos: 228 - 234). G000209 (two lots tested) served as the less modified control, having only 2'-O-methyl modifications and phosphorothioate linkages at and between the three terminal nucleotides at both the 5' and 3' termini of the sgRNA, respectively. (See Figure 15D).

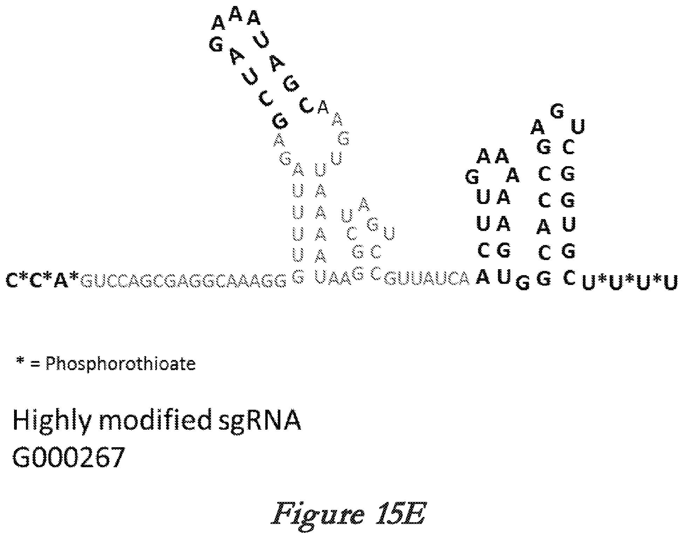

The results shown in Figures 14A-D, show that the more heavily modified sgRNAs tended to induce less of a response for each the cytokines assayed, as compared to the less modified G000209 controls. The more heavily modified sgRNAs also conferred larger editing efficiencies in the livers of treated animals, with percent editing reaching ~60% for two of the more heavily modified sgRNAs (e.g., G000263 and G000267) as compared to ~44-47% for the less modified controls (G000209 lots) (Figure 15A). Importantly, the editing efficiencies correlated with phenotypic changes as serum knockdown of TTR levels were comparable or significantly greater than the less modified controls (See e.g., G000263 and G000267 vs G000209 lots in Figures 15A-15B). The differences between the end-modified G000209 and highly-modified G000267 are summarized in Figure 15D and 15E (2'-O-Me modified nucleotides are shown in bold, and * represents phosphorothioate linkages).

In another *in vivo* study, three sgRNAs targeting a separate sequence in the mouse TTR gene were tested. Mice (n=5 per group) were administered a single dose of LNP at 2mg/kg, 1mg/kg, or 0.3mg/kg. Blood was collected four hours post dose for serum cytokine analysis. 7 days post dose at necropsy, livers and blood were collected for NGS measurements of editing efficiency and serum TTR analysis, respectively. In this study, each of the sgRNAs targeted the same sequence in the TTR gene (a different sequence from what was targeted in the previous *in vivo* study) with one sgRNA being completely unmodified (G000201 (SEQ ID NO: 243)), another having only end modifications (G000211 (SEQ ID NO: 241)), with 2'-O-methyl modifications and phosphorothioate linkages at and between the three terminal nucleotides at both the 5' and 3' termini of the sgRNA, respectively), and a third sgRNA having the same modification pattern as G000267 in the previous *in vivo* study (G000282 (SEQ ID NO: 242)).

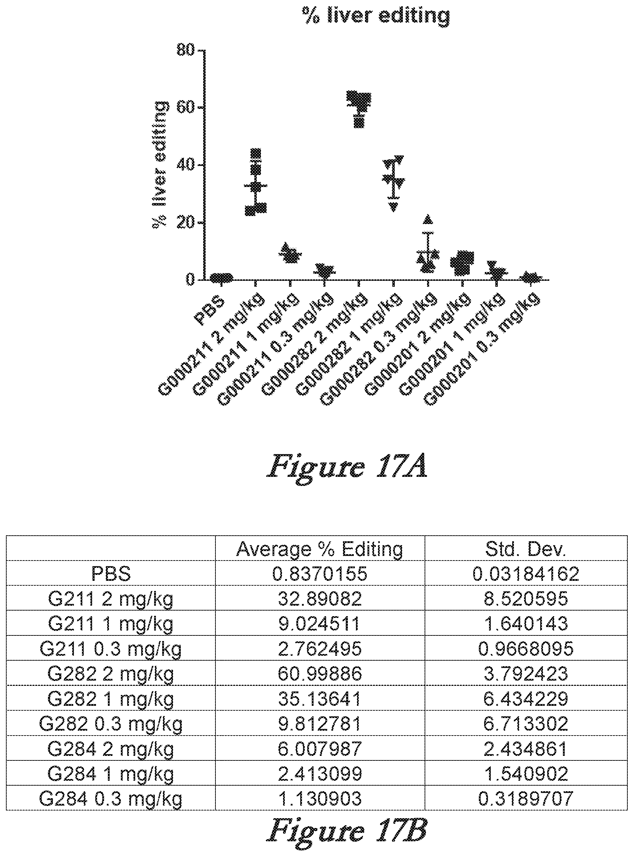

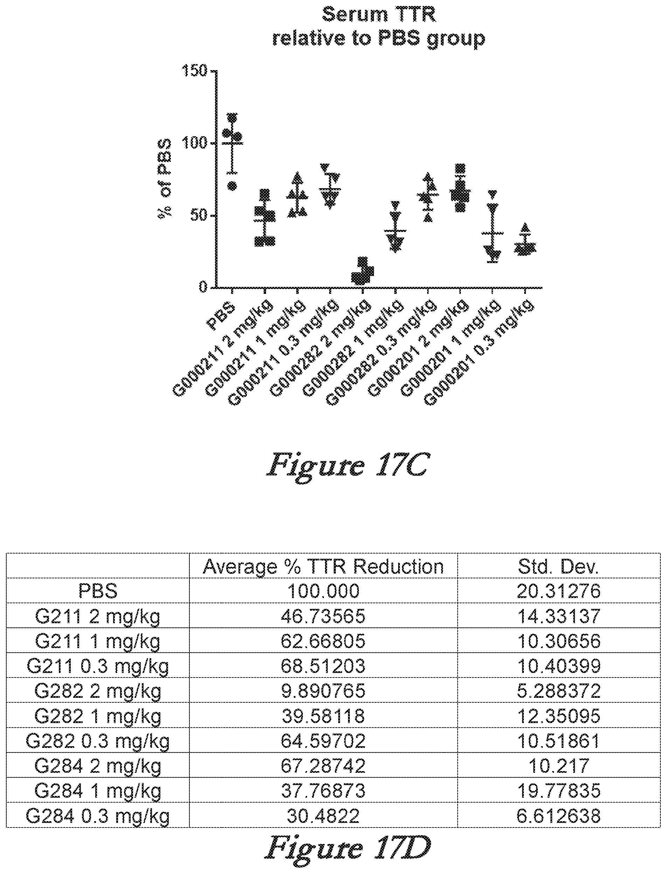

As shown in Figures 16A-16D, each of the sgRNAs resulted in similar responses in a dose dependent manner for each of the cytokines tested. For editing efficiency, the unmodified sgRNA (G000201(SEQ ID NO: 243)) conferred little *in vivo* editing, while the heavily modified sgRNA (G000282 (SEQ ID NO: 242)) conferred levels reaching ~60% with a dose of 2mg/kg, which was significantly greater than the levels achieved with the less modified sgRNA (G000211 (SEQ ID NO: 241)) (Figure 17A and B). As with the previous *in vivo* study, the levels of editing correlated with the amount of serum TTR knockdown (Figure 17C and D).

A similar study as the second *in vivo study* was next conducted with another set of three sgRNAs targeting yet a different TTR sequence in the mouse TTR gene (targeting a different sequence then what was targeted in the two previous in vivo studies). Mice (n=5 per group) were administered a single dose of LNP at 2mg/kg, 1mg/kg, or 0.3mg/kg. Blood was collected four hours post dose for serum cytokine analysis. 7 days post dose at necropsy, livers and blood were collected for NGS measurements of editing efficiency and serum TTR analysis, respectively. In this study, each of the sgRNAs targeted the same sequence in the TTR gene (a different sequence from what was targeted in the previous two *in vivo* studies) with one sgRNA being completely unmodified (G000285; (SEQ ID NO: 332)), another having only end modifications (G000269 (SEQ ID NO: 330)), with 2'-O-methyl modifications and phosphorothioate linkages at and between the three terminal nucleotides at both the 5' and 3' ends of the sgRNA, respectively), and a third sgRNA having the same modification pattern as G000267 and G000282 in the previous two *in vivo* studies (G000283 (SEQ ID NO: 331)).

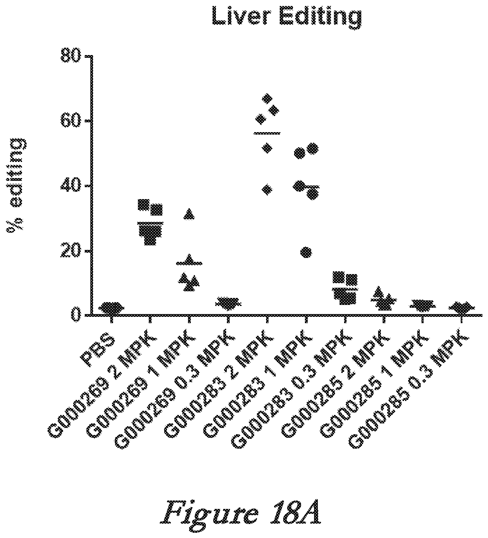

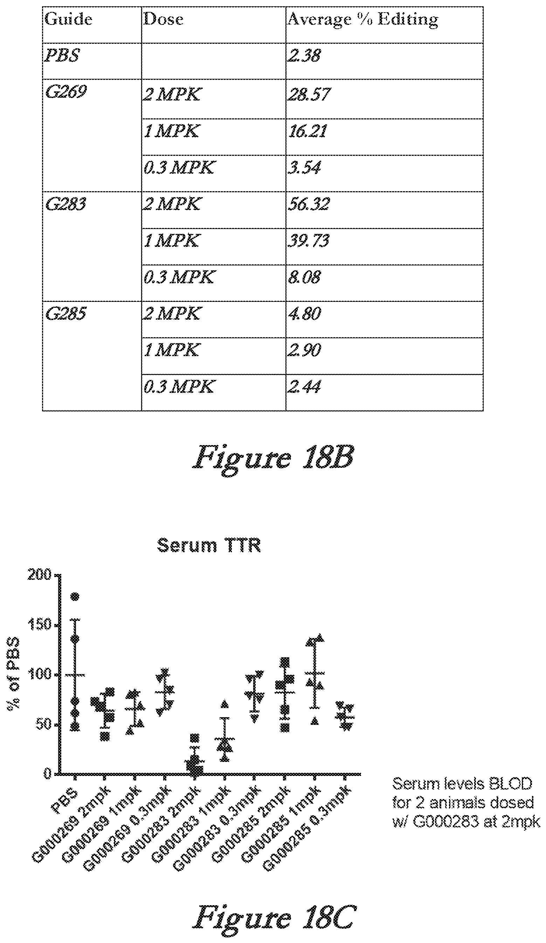

In this study, the unmodified sgRNA (G000285 (SEQ ID NO: 332)) conferred little *in vivo* editing, while the heavily modified sgRNA (G000283 (SEQ ID NO: 331)) conferred levels reaching ~60% with a dose of 2mg/kg, which was significantly greater than the levels achieved with the less modified sgRNA (G000269 (SEQ ID NO: 330)) (Figures 18A-18B). As with the previous *in vivo* studies, the levels of editing correlated with the amount of serum TTR knockdown (Figure 18C).

In a fourth *in vivo* study, the effects of modifications to gRNAs was evaluated for another gene (FVII). For in-study comparison, two of the sgRNAs tested in the first *in vivo* study were included (G000209 and G000267). Mice (n=5 per group) were administered a single dose of LNP at 2mg/kg, 1mg/kg, or 0.3mg/kg, and blood was collected four hours post dose for serum cytokine analysis. 6 days post dose at necropsy, livers were collected for NGS measurements of editing efficiency. In this study, each of the sgRNAs targeted the same sequence in the TTR or FVII genes, with one sgRNA for each having only end modifications (G000208 (SEQ ID NO: 286)) for FVII, G000209 for TTR, both having 2'-O-methyl modifications and phosphorothioate linkages at and between the three terminal nucleotides at both the 5' and 3' ends of the sgRNA, respectively), and a second sgRNA having the same modification patterns as G000267, G000282, and G000283 in the previous *in vivo* studies (G000373 (SEQ ID NO: 287) for FVII; G000267 (SEQ ID NO: 234) for TTR).

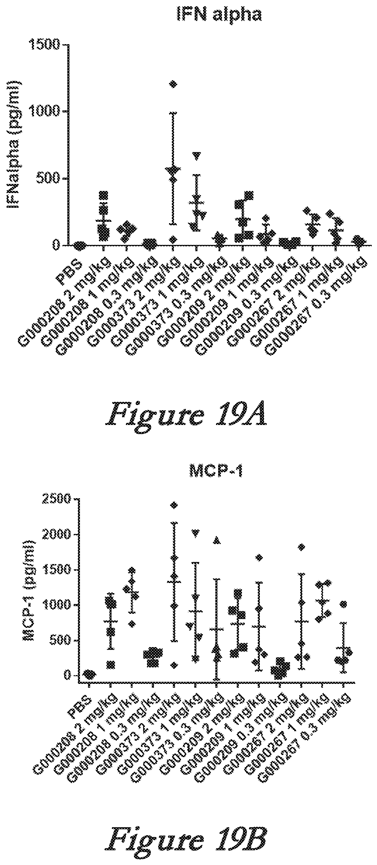

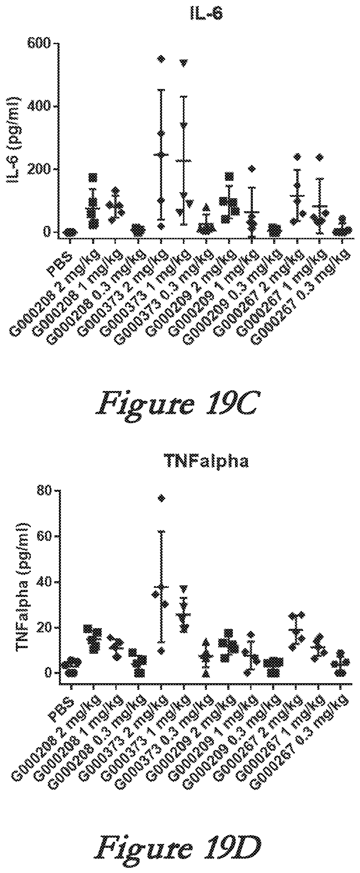

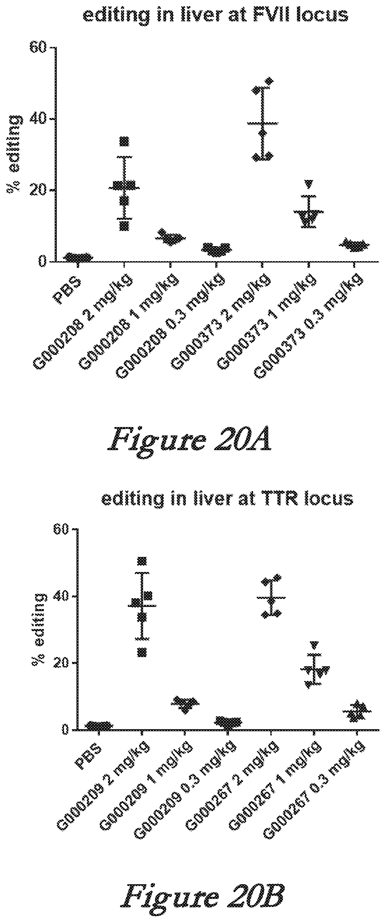

As shown in Figures 19A-19D, each of the sgRNAs resulted in similar responses in a dose dependent manner for each of the cytokines tested. For editing efficiency, the more heavily modified sgRNA targeting FVII (G000373 (SEQ ID NO: 287)) had an increase in editing efficiency as compared to the less modified version (G000208 (SEQ ID NO: 286)) across each of the doses tested (Figure 18A). These results were also observed for the sgRNAs targeting TTR (Figures 20A-20B).

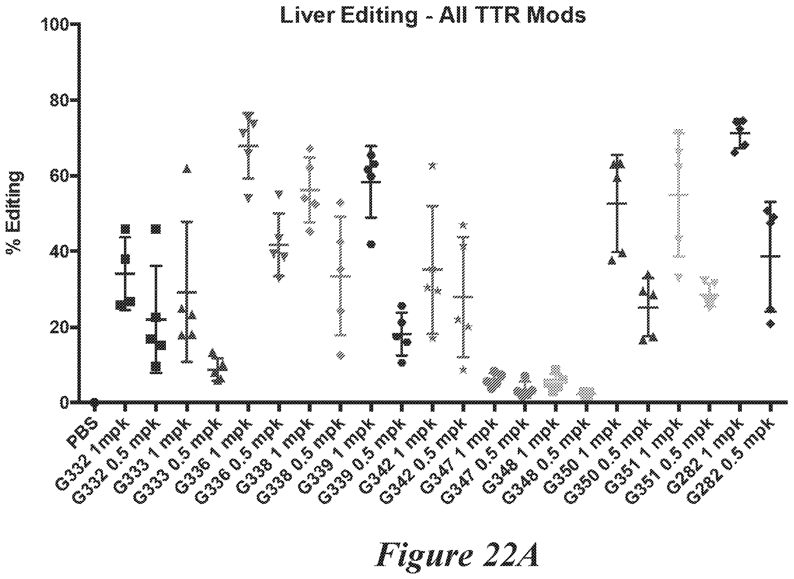

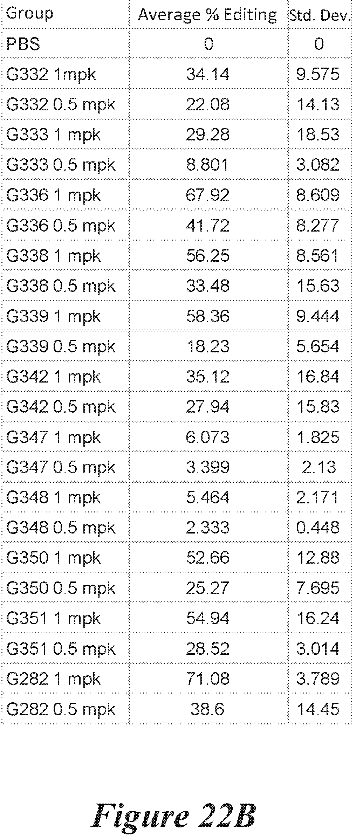

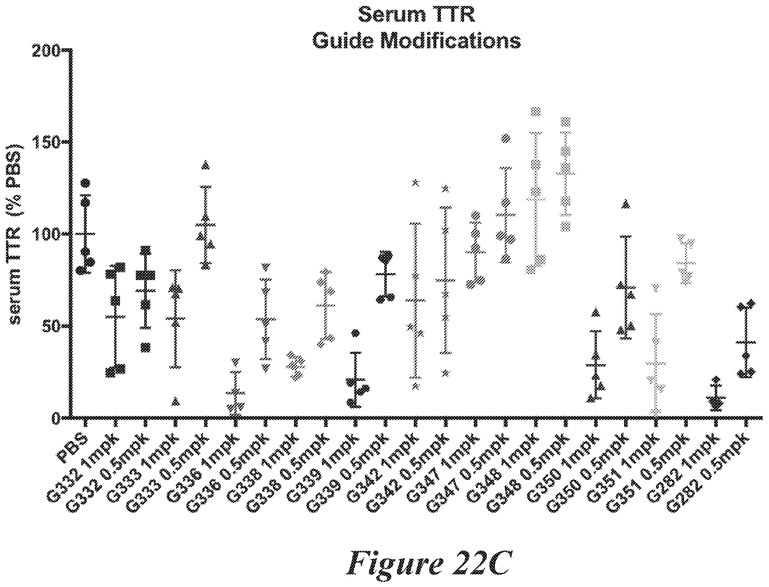

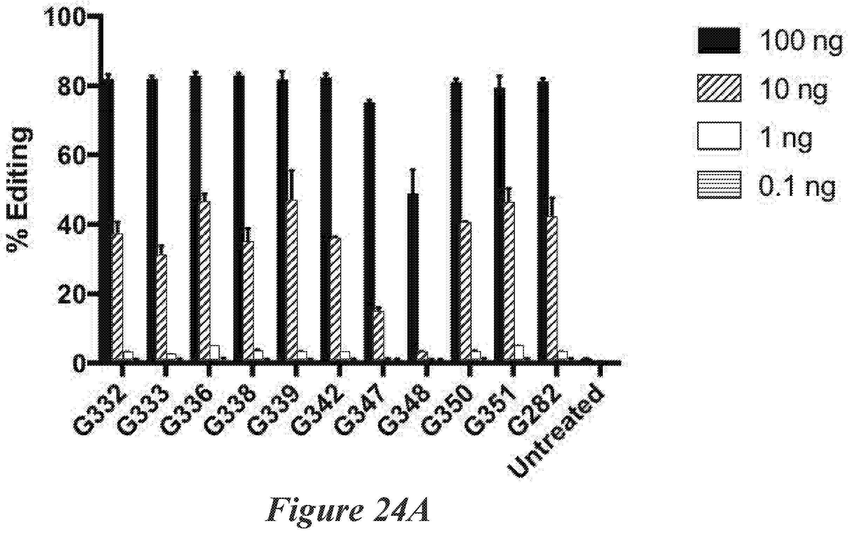

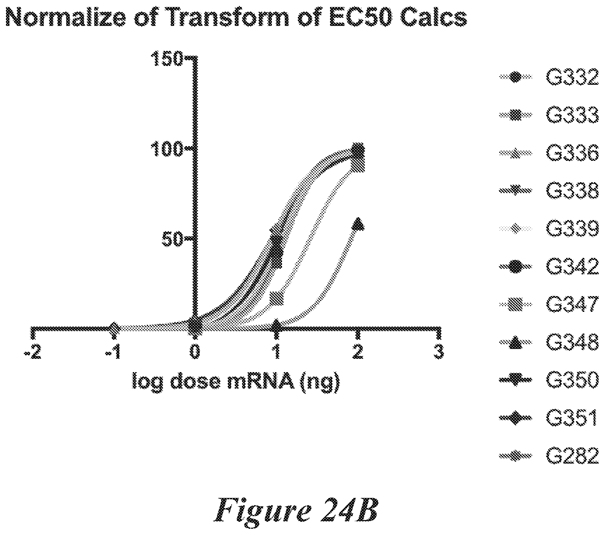

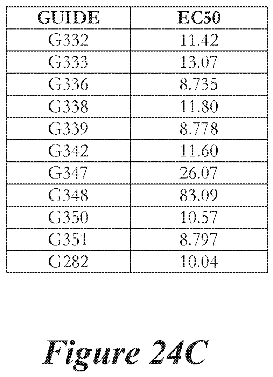

In another *in vivo* study, ten additional sgRNAs targeting the same sequence in the mouse TTR gene as G000282 were tested. G000282 was also included in the study for comparative purposes. Mice (n=5 per group) were administered a single dose of LNP at 1mg/kg or 0.5mg/kg. The LNPs used in this study were formulated using LNP Formulation Procedure B described above. Seven (7) days post dose at necropsy, livers and blood were collected for NGS measurements of editing efficiency and serum TTR analysis, respectively. In this study, each of the sgRNAs targeted the same sequence in the TTR gene. The modification pattern of each sgRNA tested varied and included 2'-OMe, 2'-F, and PS modifications in the 5' terminus, 3' terminus, hairpin 1, hairpin 2, nexus, lower stem, bulge, and upper stem of the sgRNA. The results of this study are shown in Figures 22A-22C, including % editing (Figure 22A), average editing and standard deviation (Figure 22B), and serum TTR levels (Figure 22C). These same sgRNAs were tested in primary mouse hepatocytes as per the methods described herein. The results of this dose response TTR editing study are shown in Figures 24A-24C, including % editing (Figure 24A), dose response curves (Figure 24B), and EC50 values (Figure 24C).

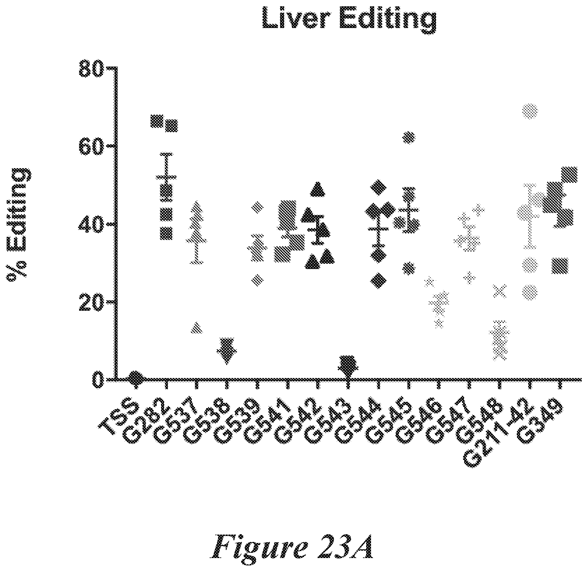

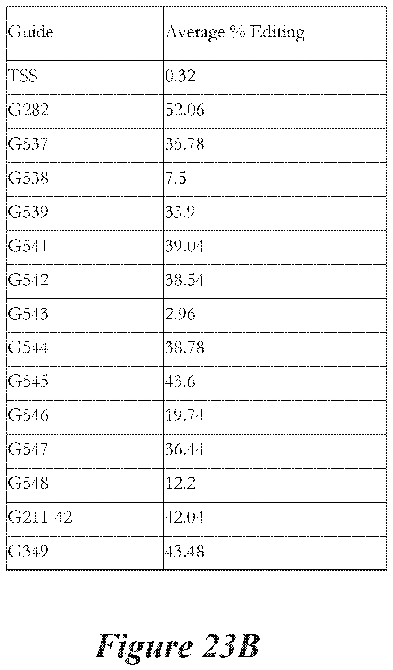

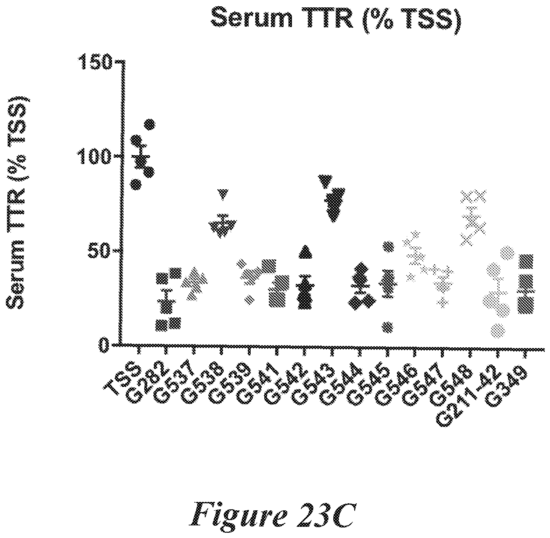

In another *in vivo* study, thirteen sgRNAs targeting the same sequence in the mouse TTR gene as G000282 were tested. G000282 was also included in the study for comparative purposes. Mice (n=5 per group) were administered a single dose of LNP at 1mg/kg. The LNPs used in this study were formulated using LNP Formulation Procedure C described above. The Cas9 mRNA used in this study had the sequence of SEQ ID NO: 359. Blood was collected four hours post dose for serum cytokine analysis. 7 days post dose at necropsy, livers and blood were collected for NGS measurements of editing efficiency and serum TTR analysis, respectively. In this study, each of the sgRNAs targeted the same sequence in the TTR gene. The sgRNAs tested include additional 2'-OMe and PS modifications in the 5' terminus, 3' terminus, hairpin 1, hairpin 2, and upper stem of the sgRNA. The results of this study are shown in Figures 23A-23C, including % editing (Figure 23A), average % editing (Figure 23B), and serum TTR levels (Figure 23C).

## Claims

1. A single guide RNA (sgRNA) comprising
mN*mN*mN*NNNNNNNNNNNNNNNNNGUUUUAGAmGmCmUmAmGmAmA mAmUmAmGmCAAGUUAAAAUAAGGCUAGUCCGUUAUCAmAmCmUmUm GmAmAmAmAmAmGmUmGmGmCmAmCmCmGmAmGmUmCmGmGmUmGm CmU*mU*mU*mU (SEQ ID NO: 358), wherein N denotes any one of A, C, G, and U; * denotes a phosphorothioate (PS) linkage; and mN, mA, mC, mG, and mU respectively denote N, A, C, G, and U modified with 2'-O-methyl (2'-O-Me).

2. A composition comprising the sgRNA of claim 1 and a lipid nanoparticle (LNP).

3. A composition comprising the sgRNA of claim 1 or the composition of claim 2, further comprising a nuclease or an mRNA encoding the nuclease.

4. The composition of claim 3, wherein the nuclease is a Cas protein.

5. The composition of claim 4, wherein the Cas protein is a Cas9.

6. The composition of claim 5, wherein the Cas9 is *Streptococcus pyogenes* Cas9.

7. A pharmaceutical formulation comprising the sgRNA of claim 1 or the composition of any one of claims 2-6 and a pharmaceutically acceptable carrier.

8. The sgRNA, the composition, or the pharmaceutical formulation of any one of claims 1-7 for use in treatment of a disease or disorder.

## Patentansprüche

1. Einzelne Guide-RNA (sgRNA), umfassend mN*mN*mN*NNNNNNNNNNNNNNNNNGUUUUAGAmGmCmUmAmGmAmA mAmUmAmGmCAAGUUAAAAUAAGGCUAGUCCGUUAUCAmAmCmUmUm GmAmAmAmAmAmGmUmGmGmCmAmCmCmGmAmGmUmCmGmGmGmUmGm CmU*mU*mU*mU (SEQ ID NO: 358), wobei N ein beliebiges von A, C, G und U bezeichnet; * eine Phosphorothioat(PS-)Verknüpfung bezeichnet; und mN, mA, mC, mG und mU jeweils N, A, C, G und U modifiziert mit 2'-O-Methyl (2'-O-Me) bezeichnen.

2. Zusammensetzung, umfassend die sgRNA nach Anspruch 1 und ein Lipid-Nanopartikel (LNP).

3. Zusammensetzung, umfassend die sgRNA nach Anspruch 1 oder die Zusammensetzung nach Anspruch 2, ferner umfassend eine Nuklease oder eine mRNA, die für die Nuklease kodiert.

4. Zusammensetzung nach Anspruch 3, wobei die Nuklease ein Cas-Protein ist.

5. Zusammensetzung nach Anspruch 4, wobei das Cas-Protein ein Cas9 ist.

6. Zusammensetzung nach Anspruch 5, wobei das Cas9 *Streptococcus pyogenes* Cas9 ist.

7. Pharmazeutische Formulierung, umfassend die sgRNA nach Anspruch 1 oder die Zusammensetzung nach einem der Ansprüche 2-6 und einen pharmazeutisch annehmbaren Träger.

8. SgRNA, Zusammensetzung oder pharmazeutische Formulierung nach einem der Ansprüche 1-7 zur Verwendung bei Behandlung einer Erkrankung oder Störung.

## Revendications

1. ARN guide unique (ARNsg) comprenant mN*mN*mN*NNNNNNNNNNNNNNNNNGUUUUAGAmGmCmUmAmGmAmA mAmUmAmGmCAAGUUAAAAUAAGGCUAGUCCGUUAUCAmAmCmUmUm GmAmAmAmAmAmGmUmGmGmCmAmCmCmGmAmGmUmCmGmGmGmUmGm CmU*mU*mU*mU (SEQ ID N° : 358), dans lequel N désigne l'un quelconque de A, C, G et U ; * désigne une liaison phosphorothioate (PS) ; et mN, mA, mC, mG et mU désignent respectivement N, A, C, G et U modifiés par 2'-O-méthyle (2'-O-Me).

2. Composition comprenant l'ARNsg de la revendication 1 et une nanoparticule lipidique (LNP).

3. Composition comprenant l'ARNsg de la revendication 1 ou la composition de la revendication 2, comprenant en outre une nucléase ou un ARNm codant pour la nucléase.

4. Composition de la revendication 3, dans laquelle la nucléase est une protéine Cas.

5. Composition de la revendication 4, dans laquelle la protéine Cas est une Cas9.

6. Composition de la revendication 5, dans laquelle la Cas9 est la Cas9 de *Streptococcus pyogenes.*

7. Formulation pharmaceutique comprenant l'ARNsg de la revendication 1 ou la composition de l'une quelconque des revendications 2 à 6 et un support pharmaceutiquement acceptable.

8. ARNsg, composition ou formulation pharmaceutique de l'une quelconque des revendications 1 à 7, destiné(e) à être utilisé(e) dans le traitement d'une maladie ou d'un trouble.
